# EUROPEAN PATENT SPECIFICATION

(11) **EP 4 103 143 B1**
(45) Date of publication and mention of the grant of the patent: **29.07.2026**
(21) Application number: 21834792.0
(22) Date of filing: 09.12.2021
(51) Int. Cl.: A61K 8/34, A61Q 5/02, A61Q 5/06, A61Q 5/12, A61Q 11/00, A61Q 13/00, A61Q 15/00, A61Q 17/00, A61Q 17/04, A61Q 19/00, A61K 47/10, A61Q 5/00

(54) **COMPOSITIONS COMPRISING NATURAL POLYMERS AND ONE OR MORE (BIO)-ALKANEDIOLS**
ZUSAMMENSETZUNGEN MIT NATÜRLICHEN POLYMEREN UND EINEM ODER MEHREREN (BIO)-ALKANDIOLEN
COMPOSITIONS COMPRENANT DES POLYMÈRES NATURELS ET UN OU PLUSIEURS (BIO)-ALCANEDIOLS

(30) Priority: 09.12.2020 WO PCT/EP2020/085166
(43) Date of publication of application: 21.12.2022
(73) Proprietor: Symrise AG, 37603 Holzminden (DE)
(72) Inventor: KOCH, Christin, 32760 Detmold (DE); LANGE, Sabine, 37603 Holzminden (DE); KRAELING, Ricarda, 37603 Holzminden (DE); MASCHER, Maik, 31860 Emmerthal (DE); BUGDAHN, Nikolas, 37603 Holzminden (DE)
(74) Representative: Global IP Europe Patentanwaltskanzlei
(86) International application number: PCT/EP2021/084939
(87) International publication number: WO 2022/122890

(56) References cited:
- WO-A1-2018/099570
- WO-A1-2018/221337
- WO-A2-2018/154145
- US-A1- 2005 222 276
- US-A1- 2009 221 716

## Description

### Technical field

The present invention refers to a cosmetic, pharmaceutical, preferably dermatological, composition or homecare product comprising or consisting of a natural polymer and an effective amount of a mixture of two or more different specific alkanediols and the use of said compositions as a cosmetic, for personal care, as a pharmaceutical, for animal care or homecare. Additionally, the present invention relates to the use of a mixture of two or more different specific alkanediols in an effective amount for reducing the microbial load of a natural polymer and/or as an antimicrobial replacer. Finally, the present invention relates to a method for reducing the microbial load of a cosmetic or pharmaceutical composition or homecare product comprising a natural polymer.

### Background Art

Cosmetic, personal care, dermatological pharmaceutical or homecare products are complex mixtures intended to be applied to the external parts of the human body or for laundering, washing and cleaning purposes. In the long list of ingredients are included polymers which are either natural or synthetic. A broad spectrum of natural and synthetic polymers is extensively used in a wide range of cosmetic and personal care products to serve a variety of functions, such as thickening, emulsifying (keeping ingredients mixed together), creating protective films or barriers, and making products feel either "drier" or more moist, smoother, or more pleasant overall.

Natural polymers or biopolymers have attracted considerable interest owing to their biocompatibility, nontoxicity, renewability, and mild processing conditions. These polymers are degradable in vivo, either enzymatically or nonenzymatically, and can be metabolized and excreted via normal physiological pathways.

Additionally, naturally polymers or biopolymers are increasing in popularity due to the microplastic discussion (see for example Carbomer, which is a persistent polyacrylic acid polymer) and the growing demand of consumers for natural and biodegradable ingredients.

The drawback of these natural polymers or biopolymers is their naturally higher contamination with bacteria compared to synthetic polymers. Usually, a bacterial load of maximum 1000 colony forming units per 1 g of raw material is accepted for cosmetics but 100 colony forming units per 1 g of raw material is most preferred. The natural contamination with bacteria makes it difficult, to store the polymer solution or the polymer containing formulation. In particular, in the preparation of cosmetic or pharmaceutical dispersions, the enlargement of the surface and the water content facilitates bacteria growth. Hence, the use of natural polymers challenges the above limits.

In order to reduce the microbial load of said formulations antimicrobial agents or sterilisation are applied. The addition of antimicrobial agents is increasingly undesired, in particular in regard to natural cosmetics and a sterilisation step causes additional cost of production. Sterilisation of the formulation is less desired due to a possible degradation of ingredients of the formulation on heat treatment and additional process costs involved.

US 2005/222276 A1 discloses an antimicrobial composition comprising an antimicrobially effective amount of a mixture of two, three or more straight-chain 1,2-alkandeiols, the chain lengths of which are (i) different and (ii) each in the range of 5 to 10 carbon atoms as antimicrobially active compounds. The explicitly disclosed 1,2-alkanediol mixtures include 1,2-hexanediol and one, two or three other straight-chain 1,2-alkanediols, in particular the combinations of 1,2-hexanediol plus 1,2-octanediol and 1,2-hexanediol plus 1,2-decanediol.

Thus, there is still an ongoing need to develop novel compositions that are microbial safe. The cosmetic or pharmaceutical industry is permanently searching for ingredients which can improve this negative aspect.

When searching for agents having an antimicrobial action it must be taken into account that the substances used in the cosmetic or pharmaceutical sector must be toxicologically acceptable, well tolerated by the skin, stable, especially in the customary cosmetic and/or pharmaceutical formulations or homecare products, substantially and preferably odourless and able to be prepared inexpensively, i.e. using standard methods and/or starting from standard precursors.

However, there is no clear dependency between the chemical structure of a substance, on the one hand, and its biological activity towards specific microorganisms and its stability, on the other hand, which makes the search for suitable substances more difficult. Furthermore, there is no predictable relationship between antimicrobial action, toxicological acceptability, tolerability and the stability of a substance.

It is therefore an object of the present invention to provide a cosmetic or pharmaceutical, in particular dermatological, composition or a homecare product comprising or consisting of a natural polymer which has a reduced microbial load and which is stable, i.e. which can be stored.

It is another object of the present invention to provide a substance or a mixture, which is capable of reducing microbial load of a natural polymer, in particular in a cosmetic or pharmaceutical composition or a homecare product and/or by which the amount of an antimicrobial in a cosmetic or pharmaceutical composition can be reduced, i.e. a substance which is suited as an antimicrobial replacer.

Furthermore, it is an object of the present invention, to provide a method for reducing microbial load in a cosmetic or pharmaceutical composition or a homecare product comprising a natural polymer.

Surprisingly, it has been found that the addition of an effective amount of a mixture of two or more different specific alkanediols as defined herein to a cosmetic or pharmaceutical composition or a homecare product comprising a natural polymer synergistically reduces the microbial load in a natural polymer comprising cosmetic or pharmaceutical composition or homecare product, which results in a microbial stable formulation which can be stored.

In particular, it has been found that specific alkanediol mixtures as defined herein are outstanding suitable to reduce the microbial load in a natural polymer comprising cosmetic or pharmaceutical composition or homecare product, resulting in a microbial stable formulation which can be stored.

### Summary of the invention

In order to accomplish the above problem, the present invention provides a cosmetic or pharmaceutical composition or a homecare product, comprising or consisting of
(a) a mixture comprising at least one first linear alkanediol having a carbon chain of 5 to 14 carbon atoms and one or more second linear alkanediol having a carbon chain of 5 to 14 carbon atoms which is different from the first linear alkanediol;
(b) at least one natural polymer; and
(c) optionally at least one active substance for a cosmetic or pharmaceutical composition or a homecare product and/or additive,
wherein the mixture comprising at least one first linear alkanediol and one or more second linear alkanediol is selected from the group consisting of:
a mixture comprising 1,2-hexanediol and 2,3-hexanediol;
a mixture comprising 1,2-heptanediol and 2,3-heptanediol;
a mixture comprising 1,2-octanediol and 2,3-octanediol; and
a mixture comprising 1,2-heptanediol and 1,2-nonanediol; and
wherein the at least one natural polymer is selected from the group consisting of guar gum, xanthan gum, carboxymethylcellulose, and mixtures of two or more of the aforesaid natural polymers.

In a further aspect, the present invention provides for the use of the cosmetic or pharmaceutical composition as cosmetic for personal care, in particular for skin, hair, scalp and nail care, as pharmaceutical, for animal care or homecare.

In a further aspect, the present invention provides for the use of a mixture comprising at least one first linear alkanediol and one or more second linear alkanediol(s) for reducing the microbial load of a natural polymer and/or as an antimicrobial replacer.

In a final aspect, the present invention provides a method for reducing the microbial load in a natural polymer comprising cosmetic or pharmaceutical composition or homecare product.

### Description of Figures

Figure 1 shows the representative results of microbial load of Nativacare (3 %) in water directly after preparation of the mixture (A), after 2 days storage at 40 °C (B) and after 7 days storage at 40 °C (C).
Figure 2 shows the representative results of microbial load of Polycos-N75 (0.5%) in water (A) or in water with addition of 0.5 % of 1,2-pentanediol (B), 1,2-hexanediol (C) or 1,2-heptanediol (D) respectively after 7 days of storage at 40 °C.
Figure 3 are photographs showing from left to right samples of the liquid 1,2-pentanediol, 1,2-hexanediol, 1,2-heptanediol, 2,3-heptanediol, 2,3-octanediol and 2,3-undecanediol in comparison to the solid 1,2-octanediol, 1,2-undecanediol and 1,2-nonanediol; and the liquid 2,3-nonanediol.
Figure 4 is a representative photograph showing samples of mixtures of 1,2-alkanediols with the respective 2,3-alkanediol. All mixtures are liquid. They are shown in the following order: 1,2-heptanediol (95 %) + 2,3-heptanediol (5 %), 1,2-octanediol (95 %) + 2,3-octanediol (5 %), 1,2-nonanediol (95 %) + 2,3-honanediol (5 %).

### Detailed description of the invention

The present invention is specified in the appended claims. The invention itself, and its preferred variants, other objects and advantages, are however also apparent from the following detailed description in conjunction with the accompanying examples and figures.

The present invention relates to a cosmetic or pharmaceutical composition or homecare product, comprising or consisting of:
(a) a mixture comprising at least one first linear alkanediol having a carbon chain of 5 to 14 carbon atoms and one or more second linear alkanediol having a carbon chain of 5 to 14 carbon atoms which is different from the first linear alkanediol;
(b) at least one natural polymer; and
(c) optionally at least one active substance for a cosmetic or pharmaceutical composition or homecare product and/or additive,
wherein the mixture comprising at least one first linear alkanediol and one or more second linear alkanediol is selected from the group consisting of:
a mixture comprising 1,2-hexanediol and 2,3-hexanediol;
a mixture comprising 1,2-heptanediol and 2,3-heptanediol;
a mixture comprising 1,2-octanediol and 2,3-octanediol; and
a mixture comprising 1,2-heptanediol and 1,2-nonanediol; and
wherein the at least one natural polymer is selected from the group consisting of guar gum, xanthan gum, carboxymethylcellulose, and mixtures of two or more of the aforesaid natural polymers.

The term "comprising" means that the named components are essential, but other components may be added and is still embraced by the present invention.

The term "consisting of" as used according to the present invention means that the total amount of components (a) to (c) adds up to 100 % by weight, based on the total weight of the cosmetic or pharmaceutical composition, and signifies that the subject matter is closed-ended and can only include the limitations that are expressly recited.

Whenever reference is made to "comprising" it is intended to cover both meanings as alternatives, that is the meaning can be either "comprising" or "consisting of", unless the context dictates otherwise.

The term "at least one ..." means that the cosmetic or pharmaceutical composition according to the present invention can comprise either one or a mixture of two, three, four, five, six or even more different of the respective components following said term.

The term "optionally" means that the subsequently described component may but need not to be present in the composition, and that the description includes variants, where the compound is included or variants, where the compound is absent.

Alkanediols are glycols, i.e. any of a class of organic compounds belonging to the alcohol family; in the molecule of a glycol, two hydroxyl (-OH) groups are attached to different carbon atoms of a carbon chain.

Straight chain 1,2-alkanediols have been used for more than 15 years as multifunctional actives. Short chain 1,2-alkanediols are amphiphilic compounds and thus, like 1,2-pentanediol and 1,2-hexanediol, are soluble both in water and cosmetic oils. In contrast, 1,2-octanediol is a solid and tends to precipitate or recrystallize in oily solutions as well as in water at ≥ 0.5 %. On the other hand, 1,2-decanediol is a solid and soluble only in cosmetic oils. Apart from moisturizing, some 1,2-alkanediols are used as viscosity modifiers.

The component (a) in the cosmetic or pharmaceutical composition or homecare product according to the present invention includes at least one first linear alkanediol having a carbon chain of 5 to 14 carbon atoms and at least one second linear alkanediol having a carbon chain of 5 to 14 carbon atoms. Therefore, the invention entails a mixture comprising a first and a second alkanediol as described herein.

In the mixtures of first and second linear alkanediols, where these are specifically different, the specific terms "first" and "second" will be used to distinguish the two alkanediol components.

As used in this document, the phrase "at least one first linear alkanediol" means that the composition comprises one first linear alkanediol having a carbon chain of 5 to 14 carbon atoms or can comprise more than one first linear alkanediol having a carbon chain of 5 to 14 carbon atoms, i.e. two, three, four or more different linear alkanediols or first linear alkanediols having a carbon chain of 5 to 14 carbon atoms.

The first linear alkanediol preferably consists of a chain of 5 to 14 carbon atoms joined to each other by single covalent bonds with two OH functional groups attached to two different carbon atoms in the chain.

The at least one first linear alkanediol having a carbon chain of 5 to 14 carbon atoms in the cosmetic or pharmaceutical composition or the homecare product according to the present invention is at least one first linear alkanediol selected from the group consisting of hexanediol, heptanediol, octanediol, and nonanediol, .

The at least one first linear alkanediol according to the present invention is selected from the group consisting of hexanediol, heptanediol, octanediol, and nonanediol.

The cosmetic or pharmaceutical composition or the homecare product according to the present invention comprises a mixture comprising at least one first linear alkanediol and one or more second linear alkanediol which is/are different from the first linear alkanediol.

This means that the alkanediol mixture is a mixture comprising at least one first linear alkanediol and one or more, i.e. two, three, four or more second linear alkanediols

For example, the mixture includes one first linear alkanediol and one, two, three or more second linear alkanediols; or the mixture can include two first linear alkanediols and one, two, three or more second linear alkanediols, etc. with the proviso, that in each mixture, the first linear alkanediols and the second linear alkanediols are different from each other.

The phrase "different from each other means" that the first linear alkanediols and the second linear alkanediols in the mixture are either different with regard to the length of their carbon chain, i.e. number of the carbon atoms, and/or with regard to their constitutional isomerism or with regard to their stereoisomerism.

Likewise, the number of the carbon atoms of the first linear alkanediols and the second linear alkanediols in the mixture can also be same. For example, the first linear alkanediol and the second linear alkanediol have a carbon chain of 7 carbon atoms, but the first linear alkanediol and the second linear alkanediol are different with regard to their constitutional isomerism or with regard to their stereoisomerism.

The second linear alkanediol preferably consists of a chain of 5 to 14 carbon atoms joined to each other by single covalent bonds with two OH functional groups attached to two different carbon atoms in the chain.

The at least one second linear alkanediol having a carbon chain of 5 to 14 carbon atoms in the cosmetic or pharmaceutical composition or the homecare product according to the present invention is selected from the group consisting of hexanediol, heptanediol, octanediol, and nonanediol.

The one or more second linear alkanediol according to the present invention is selected from the group consisting of hexanediol, heptanediol, octanediol, and nonanediol. If both the first and the second alkanediol is for example heptanediol, then the second linear alkanediol heptanediol is a different constitutional isomer or stereoisomer from the first linear alkanediol.

The term "alkanediol" within the context of the present invention also includes its constitutional isomers or position isomers. Constitutional isomers are compounds that have the same molecular formula and different connectivity. Position isomers, a particular form of constitutional isomerism, are structural isomers that can be viewed as differing only on the position of a functional group on a parent structure, which in this case is the position of the two alcohol functions.

Depending on the number of the carbon atoms of the carbon chain of the alkanediol, there are various position isomers of the alkanediol: (x,x+1) constitutional isomers; (x,x+2) constitutional isomers; (x,x+3) constitutional isomers; etc. and alpha,omega constitutional isomers when the alcohol functions are at the terminal ends of the carbon chain, wherein x stands for the number of the carbon atom in the alkanediol chain, to which the OH groups of the alkanediol are chemically bonded. For example: if x is 1, the two OH groups of the alkanediol are chemically bonded to the C1 and C2 carbon atoms in the alkanediol chain; if x is 2, the two OH groups of the alkanediol are chemically bonded to the C2 and C3 carbon atoms in the alkanediol chain, etc.

In the (x,x+1) constitutional isomers, the two OH functional groups are vicinal attached to two different adjacent carbon atoms in the chain. In the (x,x+2) constitutional isomers, the two OH functional groups are attached to two different carbon atoms in the chain where the two carbon atoms are separated by one C atom. In the (x,x+3) constitutional isomers, the two OH functional groups are attached to two different carbon atoms in the chain where the two carbon atoms are separated by two C atoms. In the alpha,omega constitutional isomers, the two functional groups are attached to the first C atom and to the terminal C atom.

In the cosmetic or pharmaceutical composition or homecare product according to the present invention, the first linear alkanediol and/or the second linear alkanediol having a carbon chain of 5 to 14 carbon atoms is a vicinal (x,x+1) diol, selected from the group consisting of a 1,2-diol, 2,3-diol, and mixtures thereof, preferably an alpha,beta 1,2 constitutional isomer.

In the cosmetic or pharmaceutical composition according to the present invention, the first linear alkanediol and/or the second linear alkanediol, is a 1,2-alkanediol, a 2,3-alkanediol, or mixtures thereof.

The 1,2-alkanediols of the first linear alkanediol having a carbon chain of 5 to 14 carbon atoms and/or the second linear alkanediol having a carbon chain of 5 to 14 carbon atoms, can preferably be those as represented by the following formulae:

| | |
|---|---|
| 1,2-hexanediol | 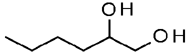 |
| 1,2-heptanediol | 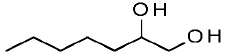 |
| 1,2-octanediol | 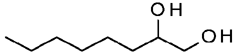 |
| 1,2-nonanediol | 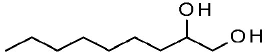 |

The 2,3-alkanediols of the first linear alkanediol having a carbon chain of 5 to 14 carbon atoms and/or the second linear alkanediol having a carbon chain of 5 to 14 carbon atoms, can preferably be those as represented by the following formulae:

| | |
|---|---|
| 2,3-hexanediol | 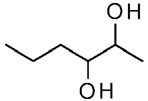 |
| 2,3-heptanediol | 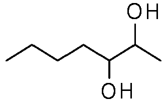 |
| 2,3-octanediol | 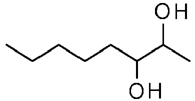 |

The cosmetic or pharmaceutical composition according to the present invention comprises a mixture comprising at least one first linear alkanediol having a carbon chain of 5 to 14 carbon atoms and one or more second linear alkanediol having a carbon chain of 5 to 14 carbon atoms, wherein the number of the carbon atoms of the first and the second alkanediol is either same or different.

If both, the first and the second alkanediol have the same number of carbon atoms, such an alkanediol combination is herein also referred to as "homo alkanediol mixture" or "homo combination". For example, the first linear alkanediol and the second linear alkanediol have a carbon chain of 7 carbon atoms, but the first linear alkanediol and the second linear alkanediol are different with regard to their constitutional isomerism or in regard to their stereoisomerism.

If the first and the second alkanediol have a different number of carbon atoms, such an alkanediol combination is herein also referred to as "hetero alkanediol mixture" or "hetero combination". For example, the first linear alkanediol has a carbon chain of 7 carbon atoms and the second linear alkanediol has a carbon chain of 8 carbon atoms. However, beyond that, the first linear alkanediol and the second linear alkanediol can be different in regard to their constitutional isomerism or with regard to their stereoisomerism.

In the cosmetic or pharmaceutical composition according to the present invention, the first linear alkanediol is selected from the group consisting of: 1,2-hexanediol, 1,2-heptanediol, 1,2-octanediol, and 1,2-nonanediol.

In the cosmetic or pharmaceutical composition or the homecare product according to the present invention, the second linear alkanediol is selected from the group consisting of:
2,3-hexanediol, 2,3-heptanediol, 2,3-octanediol, and 1,2-nonanediol.

The cosmetic or pharmaceutical composition or the homecare product according to the present invention which comprises a mixture or a combination comprising at least one first linear alkanediol and one or more second linear alkanediol includes any one of the following mixtures/combinations:
▪ a mixture comprising 1,2-hexanediol and 2,3-hexanediol;
▪ a mixture comprising 1,2-heptanediol and 2,3-heptanediol;or
▪ a mixture comprising 1,2-octanediol and 2,3-octanediol.

In said homo alkanediol mixture the first linear alkanediol and the second linear alkanediol have the same number of carbon atoms.

The above specified homo alkanediol mixtures according to the present invention, are characterized by a synergistically intensified action, i.e. the alkanediol mixture reduces the microbial load caused by gram(+) bacteria, gram(-) bacteria, yeast and mold, in a cosmetic or pharmaceutical preparation or homecare product comprising a natural polymer in a synergistic way compared to their respective single 1,2-alkanediol or 2,3-alkanediol substances as it is demonstrated in the following examples.

The homo mixture including 1,2-heptanediol and 2,3-heptanediol is particularly beneficial since it has a particular pronounced synergistic effect on the reduction of the microbial load in a cosmetic or pharmaceutical preparation or homecare product comprising a natural polymer.

With the reduction of the microbial load of the natural polymer, the storage stability and, thus, shelf life of the cosmetic or pharmaceutical preparation or homecare product can be considerably improved.

Additionally, with the admixture of the corresponding liquid 2,3-alkanediol to a solid 1,2-alkanediol, the solid 1,2-alkanediole results in a liquid alkanediol mixture. The 2,3-alkanediol serves as solvent for the solid 1,2-alkanediols. Such liquid mixtures have the benefit that firstly the availability of the solid 1,2-alkanediol in the mixture is improved and secondly the incorporation of the solid 1,2-alkanediol in semi-finished products or final products is facilitated. With the admixture of the corresponding liquid 2,3-alkanediol the 1,2-alkanediol remains in the water phase where usually microbial contamination takes place. This is in particular beneficial for the development of the antimicrobial effect of the 1,2-alkanediol in the water phase.

In addition, the above alkanediol mixture comprising a 1,2-alkanediol and the corresponding 2,3-alkanediol renders possible the cold preparation of cosmetic or pharmaceutical formulations or homecare products, i.e. no heating during preparation is necessary. Furthermore, the above alkanediol mixture comprising a 1,2-alkanediol and the corresponding 2,3-alkanediol improves the wetting properties of the natural polymer. Without the addition of such alkanediol mixtures the natural polymer formulation would separate. Moreover, the above alkanediol mixtures comprising a 1,2-alkanediol and the corresponding 2,3-alkanediol prevent the natural polymer from polymer aggregation.

Thus, with the afore-mentioned properties of the specified alkanediol mixture including a 1,2-alkanediol and the respective 2,3-alkanediol the processability in formulations can be improved.

Hence, a good compromise can be achieved when active antimicrobial mixture is combined in such a way as to maintain the antimicrobial effect, while improving processability in formulations. The above specified 1,2-alkanediol and corresponding 2,3-alkanediol combination solve this balancing problem over the 1,2-alkanediol substances or 2,3-alkanediol substances alone.

The cosmetic or pharmaceutical composition or the homecare product according to the present invention which comprises a mixture or a combination comprising at least one first linear alkanediol and one or more second linear alkanediol thus includes the following mixture/combination:
▪ a mixture comprising 1,2-heptanediol and 1,2-nonanediol.

In the hetero alkanediol mixture described before the first linear alkanediol and the second linear alkanediol have a different number of carbon atoms.

The above specified alkanediol mixture according to the present invention, is characterized by a synergistically intensified action, i.e. the alkanediol mixtures reduce the microbial load caused by gram(+) bacteria, gram(-) bacteria, yeast and mold, in a cosmetic or pharmaceutical preparation or homecare product comprising a natural polymer in a synergistic way compared to the corresponding individual 1,2-alkanediol or 2,3-alkanediol substances.

The specified hetero alkanediol mixture according to the present invention, shows a synergistically intensified action, i.e. this mixture reduces the germ load in a cosmetic or pharmaceutical preparation or homecare product comprising a natural polymer in a synergistic way compared to their corresponding single 1,2-alkanediol or 2,3-alkanediol substances.

For the afore-specified mixture it is possible to reduce the concentration of colony forming units (CFU).

With the reduction of the germ load of the natural polymer, the shelf-life of the cosmetic or pharmaceutical preparation or homecare product can considerably be prolonged.

The afore specified hetero alkane diol mixture displays a synergistic activity in reducing the microbial load of a natural polymer comprising cosmetic or pharmaceutical preparation or homecare product. The intensified action is against gram(+) bacteria and gram(-) bacteria, such as E. coli or salmonella, yeast and mold, and are clearly superior to the corresponding individual 1,2-alkanediols or 2,3-alkanediols.

Especially, the alkanediol mixture including 1,2-heptanediol and 1,2-nonanediol shows a synergistic intensification of the activity, i.e. a distinct reduction in the germ count in the natural polymer containing solutions.

Consequently, with the reduction of the microbial load of the natural polymer, the storage stability and shelf life of the cosmetic or pharmaceutical preparation or homecare product can be considerably improved.

The first linear alkanediol or the second linear alkanediol are characterized by a maximum water solubility of less than or equal to 10 % by weight. In a preferred variant, the maximum water solubility of the linear alkanediol or the first linear alkanediol or the second linear alkanediol is less than or equal to 10 % by weight and more than or equal to 1.2 % by weight. More preferred, the maximum water solubility of the linear alkanediol or the first linear alkanediol or the second linear alkanediol is less than or equal to 5 % by weight and more than or equal to 1.4 % by weight.

Preferably, the alkanediol with said water solubility is selected from the group consisting of 1,2-heptanediol, 2,3-heptanediol, and mixtures thereof.

The water solubility of a substance is the saturation mass concentration of the substance in water at a given temperature. The determination of the solubility in water relates to essentially pure substances which are stable in water and not volatile. The determination of the water solubility of the alkanediols is further described in the following Example 2.

The term "alkanediol" within the context of the present invention also includes its stereoisomers. Stereoisomers are molecules that have the same molecular formula and differ only in how their atoms are arranged in three-dimensional space. In accordance with said definition, the first linear alkanediol having a carbon chain of 5 to 14 carbon atoms or the second linear linear alkanediol having a carbon chain of 5 to 14 carbon atoms as described above in detail encompass the following stereoisomers:
1,2-alkanediol stereoisomers:
   (2S)-hexane-1,2-diol,
   (2S)-heptane-1,2-diol,
   (2S)-octane-1,2-diol,
   (2S)-nonane-1,2-diol,
   (2R)-hexane-1,2-diol,
   (2R)-heptane-1,2-diol,
   (2R)-octane-1,2-diol,
   (2R)-nonane-1,2-diol,
2,3-alkanediol stereoisomers:
   (2S, 3S)-hexane-2, 3-diol,
   (2S,3S)-heptane-2,3-diol,
   (2S,3S)-octane-2,3-diol,
   (2R,3R)-hexane-2,3-diol,
   (2R,3R)-heptane-2,3-diol,
   (2R,3R)-octane-2,3-diol,
   (2S,3R)-hexane-2,3-diol,
   (2S,3R)-heptane-2,3-diol,
   (2S,3R)-octane-2,3-diol,
   (2R,3S)-hexane-2,3-diol,
   (2R,3S)-heptane-2,3-diol,
   (2R,3S)-octane-2,3-diol.

In the context of the present text, the terms "1,2-diol" or "2,3-diol" includes both the corresponding S-configured enantiomers and also the R-enantiomeras well as arbitrary mixutres of these S- and R-configured enantiomers, i.e. mixtures of racemates of the respective diols.

The first linear alkanediol and the second linear alkanediol as defined in detail above, may present in the alkanediol mixture according to the present invention in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

In the cosmetic or pharmaceutical preparation according to the present invention, the first linear alkanediol and the second linear alkanediol may comprised in the mixture preferably in a ratio in a range of 98 : 2 to 99.9 : 0.1

In the homecare products according to present invention, the first linear alkanediol and the second alkanediol may comprised in the alkanediol mixture preferably in a ratio in a range of 95 : 5 to 99.9 : 0.1.

Preferably, the first linear alkanediol and the second alkanediol may comprised in the mixture of the cosmetic or pharmaceutical composition or homecare product according to the present invention in a ratio of ≥ 95 : ≤ 5, more preferred in a ratio of ≥ 96 : ≤ 4; still more preferred in a ratio of ≥ 97 : ≤ 3, and most preferred in a ratio of ≥ 98 : ≤ 2.

Most of all the mixture of the cosmetic or pharmaceutical composition or homecare product according to the present invention comprises the first linear alkanediol and the second linear alkanediol in a ratio of ≥ 95 : ≤ 5, including the ratios ≥ 95.5 : ≤ 4.5; ≥ 96 : ≤ 4; ≥ 96.5 : ≤ 3.5; ≥ 97 : ≤ 3; ≥ 97.5 : 2,5 and ≥ 98.0 : ≤ 2.0.

Even more preferred the alkanediol mixture of the cosmetic or pharmaceutical composition or homecare product according to the present invention comprises the first linear alkandeiol and the second alkanediol in a ratio of ≥ 98 : ≤ 2, including the ratios of ≥ 98.1 : ≤ 1.9; ≥ 98.2 : ≤ 1.8; ≥ 98.3 : ≤ 1.7; ≥ 98.4 : ≤ 1.6; ≥ 98.5 : ≤ 1.5; ≥ 98.6 : ≤ 1.4; ≥ 98.7 : ≤ 1.3; ≥ 98.8 : ≤ 1.2; ≥ 98.9 : ≤ 1.1; ≥ 99 : ≤ 1.0; ≥ 99.1 : ≤ 0.9; ≥ 99.2 : ≤ 0.8; ≥ 99.3 : ≤ 0.7; ≥ 99.4 : ≤ 0.6; ≥ 99.5 : ≤ 0.5; ≥ 99.6 : ≤ 0.4; ≥ 99.7 : ≤ 0.3; ≥ 99.8 : ≤ 0.2 and ≥ 99.9 : ≤ 0.1.

Alternatively, said ratio ranges for the first and second alkanediol are switched, such that the second alkanediol is the main component and the first alkanediol is the secondary component.

In an advantageous variant according to the present invention, the mixture comprises as first linear alkanediol an 1,2-alkanediol, such as 1,2-heptanediol, and as second linear alkanediol the corresponding 2,3-alkanediol, such as 2,3-heptanediol, in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said mixture the ratios of first linear alkanediol : second alkanediol or ranges of ratios as described above are also applicable.

According to the present invention, in the mixtures comprising a combination of a 1,2-alkanediol as first linear alkandediol and the corresponding 2,3-alkanediol as second linear alkanediol, such as 1,2-heptanediol and 2,3-heptanediol, the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2 or still more preferred in ratio in a range of 90 : 10 to 95 : 5.

Likewise, for said mixtures the ratios of first linear alkanediol : second alkanediol or ranges of ratios as described above are also applicable.

By these mixing ratios the afore mentioned mixture shows a synergistically intensified activity in direct comparison with the corresponding individual substances as it is described and demonstrated in the experimental part and it is possible to remarkably reduce the concentration of colony forming units.

The present invention also encompasses a mixture including as first linear alkanediol 1,2-heptanediol and as second linear alkanediol 1,2-nonanediolwherein the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2 or still more preferred in a ratio in a range of 90 : 10 to 95 : 5.

Likewise, for said mixture, the ratios of first linear alkanediol : second alkanediol or ranges of ratios as described above are also applicable.

By these mixing ratios the afore mentioned mixture show a synergistically intensified activity in direct comparison with the corresponding individual substances as it is described and demonstrated in the experimental part and it is possible to remarkably reduce the concentration of colony forming units.

The alkanediols are obtained either by synthesis from petrochemical or other fossil fuel sources by known methods such as olefin bishydroxylation, hydrolysis from epoxide or various chemical transformations or from bioderived feedstock by fermentation or from bio-based natural and renewable feedstock such as biomass by catalytic synthesis as it is described in US 2019/0241491 A1 and US 2020/0189995 A1. The alkanediols used according to the present invention comprise either petrochemically derived and biobased natural and renewable feedstock derived alkanediols. Preferably, the alkanediols are from bio-based sources and are thus bio-alkanediols.

The compound (b) of the cosmetic or pharmaceutical composition according to the present invention relates to a natural polymer which is used in a wide range of cosmetic, personal care products or pharmaceuticals to serve a variety of functions, such as thickening, emulsifying (keeping ingredients mixed together), creating protective films or barriers, and making products feel either "drier" or more moist, smoother, or more pleasant overall:

Natural polymers respective biopolymers within the context of the present invention are defined as a diverse group of non-petrochemically derived polymers, i.e. from natural origin or natural feedstock or produced by fermentation.

Natural polymers or biopolymers have attracted considerable interest owing to their biocompatibility, nontoxicity, renewability, and mild processing conditions. These polymers are degradable in vivo, either enzymatically or nonenzymatically, and can be metabolized and excreted via normal physiological pathways.

The natural polymer component in the cosmetic or pharmaceutical composition according to the present invention is selected from the group consisting of Guar gum, xanthan gum, carboxymethylcellulose and mixtures of two or more of the aforesaid natural polymers.

The afore specified natural polymers can be used either as a single component or in mixture with two or more different natural polymers as specified above.

In a particular advantageously variant, the cosmetic or pharmaceutical composition according to the present invention comprises one of the following combinations of components (a) and (b):
▪ 1,2-hexanediol in combination with 2,3-hexanediol plus one or more of the following natural polymers:
   guar gum, and/or xanthan gum, and/or carboxymethylcellulose; or
▪ 1,2-heptanediol in combination with 2,3-heptanediol plus one or more of the following natural polymers:
   guar gum, and/or xanthan gum, and/or carboxymethylcellulose; or
▪ 1,2-octanediol in combination with 2,3-octanediol plus one or more of the following natural polymers:
   guar gum, and/or xanthan gum, and/or carboxymethylcellulose; or
▪ 1,2-heptanediol and 1,2-nonanediol plus one or more of the following natural polymers:
   guar gum, and/or xanthan gum, and/or carboxymethylcellulose.

Compared with the use of a single 1,2-alkanediol, the above specified mixtures comprising a first linear alkanediol and a second linear alkanediol according to the present invention even have a synergistically intensified action, i.e. the mixtures significant reduce the microbial load in a cosmetic or pharmaceutical preparation or homecare product comprising a natural polymer in a synergistic way as their respective single alkanediol substances. This effect or action is especially pronounced for the homo mixture of 1,2-heptanediol and 2,3-heptanediol

Also an alkanediol mixture including different first linear alkanediol and second linear such as hetero mixtures including a mixture including 1,2-heptanediol and 1,2-nonanediol, as specified above shows a synergistically intensified action in reducing microbial load in a cosmetic or pharmaceutical preparation or homecare product comprising a natural polymer.

The microbial stabilization allows the cosmetic or pharmaceutical compositions or homecare product to be stored without microbial degradation and deterioration thereby extending their shelf time.

In addition, the homo alkanediol mixture comprising a 1,2-alkanediol and the corresponding 2,3-alkanediol as described above renders possible the cold preparation of cosmetic or pharmaceutical formulations or homecare products, i.e. no heating during preparation is necessary. Furthermore, the above alkanediol mixture comprising a 1,2-alkanediol and the corresponding 2,3-alkanediol improve the wetting properties of the natural polymer. Without the addition of such alkanediol mixtures the natural polymer formulation would separate. Moreover, the above alkanediol mixture comprising a 1,2-alkanediol and the corresponding 2,3-alkanediol prevent the natural polymer from polymer aggregation.

Thus, with the afore-mentioned properties of the alkanediol mixture including a 1,2-alkanediol and the respective 2,3-alkanediol as specified above the processability in formulations can be improved.

Hence, a good compromise can be achieved when active antimicrobial mixture is combined in such a way as to maintain the antimicrobial effect, while improving processability in formulations.

In view of the comprehensive research on the activity of individual alkanediols it was surprising that especially mixtures of homo alkanediols, as described above, display a synergistic activity and that said mixtures are superior to the individually corresponding 1,2-alkanediols or 2,3-alkanediols and having the same concentration. In all proposed solutions according to the present invention a reduction of the CFU value (CFU = number of colony-forming units) can be achieved.

The above-defined specific mixtures of alkanediols plus a natural polymer can be combined with one or more further components (c) as described later on.

In order to further reduce the microbial load, the cosmetic or pharmaceutical composition or homecare product as defined herein, is advantageously combined with at least one antimicrobial agent.

An antimicrobial agent is a substance that either kills or slows the growth of microorganisms. Among the antimicrobial agents are antibacterial agents, antiviral agents, antifungal agents, and antiparasitic drugs. The combination with an antimicrobial agent provides reliable protection against microbial degradation and deterioration of the cosmetic or pharmaceutical composition, in particular during storage.

Antimicrobial agent in the context of the present invention is selected from the group consisting of Caprylhydroxamic Acid, o-cymen-5-ol, Isopropylparaben, Capryloyl Glycine, Phenylpropanol, Tropolone, PCA Ethyl Cocoyl Arginate, 2-Methyl 5-Cyclohexylpentanol, Phenoxyethanol, Disodium EDTA, Methylparaben and its salts, Sodium Benzoate, Benzyl Alcohol, Potassium Sorbate, Benzyl Salicylate, Propylparaben, Methylchloroisothiazolinone, Methylisothiazolinone, Ethylhexylglycerin, Butylparaben, Ethylparaben, Sodium Propylparaben, DMDM Hydantoin, Hydroxyethoxyphenyl Butanone, Hydroxyethoxyphenyl Butanol, Itaconic Acid and salts thereof, Octopirox, Propanediol Caprylate, Climbazole, 4-Hydroxyacetophenone, Dehydroacetic Acid, lodopropynyl Butylcarbamate, Salicylic Acid, Chlorphenesin, Isobutylparaben, Sodium Ethylparaben, Diazolidinyl Urea, Farnesol, Bisabolol, Glyceryl Caprylate, Sodium Phytate or Phytic acid, Sodium Levulinate or Levulinic Acid and esters thereof, Chlorhexidine, Glyceryl Laurate, Anisic Acid and salts thereof, Chlorhexidine Digluconate, TEA-Salicylate, Phenethyl Alcohol, Glyceryl Caprate, Sorbitan Caprylate, Tetrasodium Glutamate Diacetate, Trisodium Ethylenediamine Disuccinate, Sodium Lactate, Undecylenoyl glycine, Thymol, 1,3-Propanediol, 4-hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, 3,4,4'-trichlorocarbanilide (TTC), antibacterial fragrances, thymol, thyme oil, eugenol, oil of cloves, menthol, mint oil, glycerol monocaprate, glycerol monocaprylate, glycerol monolaurate (GML), diglycerol monocaprate (DMC), salicylic acid N-alkylamides, such as, for example, n-octylsalicylamide or n-decylsalicylamide, Dehydroacetic Acid, 4-Methylbenzyl Alcohol, Frambinon, Xylityl caprylate, Benzoic acid 3-hyroxypropylester, Anisic acid 3-hydroxypropylester, 3-Hydroxypropyl 2-hydroxybenzoate and mixtures of two or more the aforesaid antimicrobial agents.

In a preferred variant according to the present invention, the antimicrobial agent is selected from the group consisting of Phenoxyethanol, Disodium EDTA, Methylparaben and its salts, Sodium Benzoate, Benzyl Alcohol, Potassium Sorbate, Benzyl Salicylate, Propylparaben, Methylchloroisothiazolinone, Methylisothiazolinone, Ethylhexylglycerin, Butylparaben, Ethylparaben, Sodium Propylparaben, DMDM Hydantoin, Hydroxyethoxyphenyl Butanone, Hydroxyethoxyphenyl Butanol, Itaconic Acid and salts thereof, Octopirox, Propanediol Caprylate, Climbazole, 4-Hydroxyacetophenone, Dehydroacetic Acid, lodopropynyl Butylcarbamate, Salicylic Acid, Chlorphenesin, Isobutylparaben, Sodium Ethylparaben, Diazolidinyl Urea, Farnesol, Bisabolol, Glyceryl Caprylate, Sodium Phytate or Phytic acid, Sodium Levulinate or Levulinic Acid and esters thereof, Chlorhexidine, Glyceryl Laurate, Anisic Acid and salts thereof, Chlorhexidine Digluconate, TEA-Salicylate, Phenethyl Alcohol, Glyceryl Caprate, Sorbitan Caprylate, Tetrasodium Glutamate Diacetate, Trisodium Ethylenediamine Disuccinate, Sodium Lactate, Frambinon, Xylityl caprylate, Benzoic acid 3-hyroxypropylester, Anisic acid 3-hydroxypropylester, 3-Hydroxypropyl 2-hydroxybenzoate and and mixtures of two or more the aforesaid antimicrobial agents.

In a most preferred variant according to the present invention, the antimicrobial agent is selected from the group consisting of Phenoxyethanol, Disodium EDTA, Methylparaben and its salts, Sodium Benzoate, Benzyl Alcohol, Potassium Sorbate, Benzyl Salicylate, Propylparaben, Methylchloroisothiazolinone, Methylisothiazolinone, Ethylhexylglycerin, Butylparaben, Ethylparaben, Sodium Propylparaben, DMDM Hydantoin, Hydroxyethoxyphenyl Butanone, Hydroxyethoxyphenyl Butanol, Itaconic Acid and salts thereof, Octopirox, Propanediol Caprylate, Climbazole, 4-Hydroxyacetophenone, Dehydroacetic Acid, Frambinon, Xylityl caprylate, Benzoic acid 3-hyroxypropylester, Anisic acid 3-hydroxypropylester, 3-Hydroxypropyl 2-hydroxybenzoate and and mixtures of two or more of the aforesaid antimicrobial agents.

The cosmetic or pharmaceutical, in particular dermatological, composition as defined herein, are preferably based on a carrier, which comprises at least one oil phase. Accordingly, oils, oil-in-water and water-in-oil emulsions, etc. are suitable. However, preparations solely based on water or water/alcohol or water/glycol are likewise possible or even preferred.

The oil phase or oil component in the cosmetic or pharmaceutical composition according to the present invention which may be suitable are for example plant oils, hydrocarbons, fatty alcohols, fatty acid esters, liquid UV filters, or mixtures of two or more of the aforesaid oil components.

The oil phase or oil component in the cosmetic or pharmaceutical composition is preferably a plant oil and even more preferably a liquid plant oil. It can also advantageously be a mixture comprising two or more plant oils components, especially liquid plant oil mixtures.

Plant oils or vegetable oils are oils extracted from seeds, or less often, from other parts of fruits. Like animal fats, plant oils are mixtures of triglycerides. Soybean oil, rapeseed oil and cocoa butter are examples of plant oils from seeds. Olive oil, palm oil and rice bran oil are examples of oils from other parts of fruits. In common usage, plant oil or vegetable oil may refer exclusively to vegetable fats which are liquid at room temperature or at 35 to 37 °C skin temperature. Vegetable oils are usually edible.

The term "plant oils" also includes unsaturated plant oils. Unsaturated oils or vegetable oils can be transformed through partial or complete "hydrogenation" into oils of higher melting point. The hydrogenation process involves "sparging" the oil at high temperature and pressure with hydrogen in the presence of a catalyst, typically a powdered nickel compound. As each carbon-carbon double-bond is chemically reduced to a single bond, two hydrogen atoms each form single bonds with the two carbon atoms. The elimination of double bonds by adding hydrogen atoms is called saturation; as the degree of saturation increases, the oil progresses toward being fully hydrogenated. An oil may be hydrogenated to increase resistance to rancidity (oxidation) or to change its physical characteristics. As the degree of saturation increases, the oil's viscosity and melting point increase.

In a preferred variant, the plant oil is selected from the group consisting of Persea Gratissima (Avocado Oil), Abies Alba Seed Oil, Acacia Victoriae Seed Oil, Actinidia Chinensis (Kiwi) Seed Oil, Amaranthus Hypochondriacus Seed Oil, Arachis Hypogaea (Peanut) Oil, Astrocaryum Murumuru Seed Butter, Astrocaryum Tucuma Seed Butter, Astrocaryum Tucuma Seed Oil, Astrocaryum Vulgare Fruit Oil, Astrocaryum Vulgare Kernel Oil, Avena Sativa (Oat) Kernel Oil, Brassica Alba Seed Oil, Brassica Campestris (Rapeseed) Seed Oil, Butyrospermum Parkii (Shea) Butter, Butyrospermum Parkii (Shea) Oil, Calendula Officinalis Seed Oil, Calophyllum Inophyllum Seed Oil, Calophyllum Tacamahaca Seed Oil, Camellia Oleifera Seed Oil, Camellia Reticulata Seed Oil, Camellia Sinensis Seed Oil, Cannabis Sativa Seed Oil, Cannabis Sativa Seed/Stem Oil, Canola Oil, Carthamus Tinctorius (Safflower) Seed Oil, Chlorella Vulgaris Oil, Citrullus Lanatus (Watermelon) Seed Oil, Citrus Aurantifolia (Lime) Seed Oil, Citrus Aurantium Dulcis (Orange) Seed Oil, Citrus Grandis (Grapefruit) Seed Oil, Cocos Nucifera (Coconut) Oil, Cocos Nucifera (Coconut) Seed Butter, Coffea Arabica (Coffee) Seed Oil, Chlorella Oil (biotech), Corylus Americana (Hazelnut) Seed Oil, Corylus Avellana (Hazelnut) Seed Oil, Cucumis Melo (Melon) Seed Oil, Cucumis Sativus (Cucumber) Seed Oil, Cucurbita Pepo (Pumpkin) Seed Oil, Elaeis Guineensis (Palm) Oil, Elaeis (Palm) Fruit Oil, Glycine Soja (Soybean) Oil, Gossypium Herbaceum (Cotton) Seed Oil, Gossypium Hirsutum (Cotton) Seed Oil, Helianthus Annuus (Sunflower) Seed Oil, Macadamia Integrifolia Seed Oil, Macadamia Ternifolia Seed Oil, Mangifera Indica (Mango) Seed Butter, Mangifera Indica (Mango) Seed Oil, Melissa Officinalis Seed Oil, Microalgae Oil, Moringa Oleifera Seed Oil, Moringa Peregrina Seed Oil, Oenothera Biennis (Evening Primrose) Oil, Olea Europaea (Olive) Fruit Oil, Olus Oil, Orbignya Oleifera Seed Oil, Orbignya Speciosa Kernel Oil, Oryza Sativa (Rice) Bran/Germ Oil, Oryza Sativa (Rice) Bran Oil, Oryza Sativa (Rice) Germ Oil, Oryza Sativa (Rice) Lipids, Oryza Sativa (Rice) Seed Oil, Papaver Somniferum Seed Oil, Passiflora Edulis Seed Oil, Persea Gratissima (Avocado) Butter, Persea Gratissima (Avocado) Oil, Prunus Amygdalus Dulcis (Sweet Almond) Oil, Prunus Armeniaca (Apricot) Kernel Oil, Prunus Persica (Peach) Kernel Oil, Punica Granatum Seed Oil, Pyrus Malus (Apple) Seed Oil, Ricinoleic/Caproic/Caprylic/Capric Triglyceride(s), Ricinus Communis (Castor) Seed Oil, Rosa Canina Fruit Oil, Rosa Moschata Seed Oil, Rubus Idaeus (Raspberry) Seed Oil, Sesamum Indicum (Sesame) Seed Butter, Soybean Glycerides, Theobroma Cacao (Cocoa) Seed Butter, Theobroma Grandiflorum Seed Butter, Triticum Vulgare (Wheat) Bran Lipids, Triticum Vulgare (Wheat) Germ Oil, Vitis Vinifera (Grape) Seed Oil, Zea Mays (Corn) Germ Oil, and Zea Mays (Corn) Oil.

In a particular preferred variant, the cosmetic or pharmaceutical composition according to the present invention comprises as oil component a plant oil selected from the group consisting of Caprylic Capric Triglycerides, Helianthus Annuus (Sunflower) Seed Oil, Simmondsia Chinensis ((Jojoba) Seed Oil, Olea Europaea (Olive) Fruit Oil, Argania spinosa kernel oil (Argan oil), Prunus Amygdalus Dulcis (Sweet Almond) Oil, Persea Gratissima (Avocado Oil), Butyrospermum Parkii (Shea) Butter, Cocos Nucifera (Coconut) Oil, Theobroma Cacao (Cocoa) Seed Butter, and mixtures of two or more of the aforesaid plant oils. The plant oil can be used either as a single component or in mixture with one or more further different plant oil(s) as specified above.

Hydrocarbons (mineral oils) are in general organic compounds consisting entirely of hydrogen and carbon. As defined by IUPAC nomenclature or organic chemistry, the classifications for hydrocarbons are:
1. Saturated hydrocarbons are the simplest of the hydrocarbon species. They are composed entirely of single bonds and are saturated with hydrogen. The formula for acyclic saturated hydrocarbons (i.e., alkanes) is C*ₙ*H_{2*n*+2}. The most general form of saturated hydrocarbons is C*ₙ*H_{2*n*+2(1-*r*)}, where r is the number of rings. Those with exactly one ring are the cycloalkanes. Saturated hydrocarbons are the basis of petroleum fuels and are found as either linear or branched species.
2. Unsaturated hydrocarbons have one or more double or triple bonds between carbon atoms. Those with double bond are called alkenes. Those with one double bond have the formula C*ₙ*H_{2*n*} (assuming non-cyclic structures). Those containing triple bonds are called alkynes. Those with one triple bond have the formula C*ₙ*H_{2*n*-2}.
3. Aromatic hydrocarbons, also known as arenes, are hydrocarbons that have at least one aromatic ring.

Hydrocarbons can be inter alia liquids (e.g. hexane and benzene), waxes or low melting solids (e.g. paraffin wax and naphthalene). The term 'aliphatic' refers to non-aromatic hydrocarbons. Saturated aliphatic hydrocarbons are sometimes referred to as "paraffins". Mineral oils and waxes are mixtures of predominantly saturated hydrocarbons consisting of straight-chain, branched and ring structures with carbon chain lengths greater than C14. Mineral oils and waxes are chemical substances prepared from naturally occurring crude petroleum oil. They mainly consist of mineral oil saturated hydrocarbons (MOSH) and mineral oil aromatic hydrocarbons (MOAH). Hydrocarbons have been used for many decades in skin and lip care cosmetic products due to their excellent skin tolerance as well as their high protecting and cleansing performance and broad viscosity options. In contrast to vegetable oils, mineral oils are non-allergenic since they are highly stable and not susceptible to oxidation or rancidity.

In a preferred variant, the hydrocarbon is selected from the group consisting of undecane, tridecane, mineral oil, petrolatum, squalane, isohexadecane, C7 - C8 isoparaffin, C8 - C9 isoparaffin, C9 - C11 isoparaffin, C9 - C12 isoparaffin, C9 - C13 isoparaffin, C9 - C14 isoparaffin, C9 - C16 isoparaffin, C10 - C11 isoparaffin, C10 - C12 isoparaffin, C10 - C13 isoparaffin, C11 - C12 isoparaffin, C11 - C13 isoparaffin, C11 - C14 isoparaffin, C12 - C14 isoparaffin, C12 - C15 isoparaffin, C12 - C20 isoparaffin, C13 - C14 isoparaffin, C13 - C16 isoparaffin, C14 - C16 isoparaffin, C15 - C19 isoparaffin, and C18 - C70 isoparaffin. The hydrocarbon can be used either as a single component or in mixture with one or more further different hydrocarbon(s) as specified above.

A fatty alcohol (or long-chain alcohol) is usually a high-molecular-weight, straight-chain primary alcohol, but can also range from as few as 4 to 6 carbons to as many as 22 to 26, derived from natural fats and oils. The precise chain length varies with the source. Some commercially important fatty alcohols are lauryl, stearyl and oleyl alcohols. They are colorless oily liquids (for smaller carbon numbers) or waxy solids, although impure samples may appear yellow. Fatty alcohols usually have an even number of carbon atoms and a single alcohol group (-OH) attached to the terminal carbon. Some are unsaturated and some are branched. Most fatty alcohols in nature are found as waxes which are esters with fatty acids and fatty alcohols. The traditional sources of fatty alcohols have largely been various vegetable oils and these remain a large-scale feedstock. The alcohols are obtained from the triglycerides (fatty acid triesters), which form the bulk of the oil. The process involves the transesterification of the triglycerides to give methyl esters which are then hydrogenated to give the fatty alcohols. Fatty alcohols are also prepared from petrochemical sources. In the Ziegler process, ethylene is oligomerized using triethylaluminium followed by air oxidation. Alternatively, ethylene can be oligomerized to give mixtures of alkenes, which are subjected to hydroformylation, this process affording odd-numbered aldehyde, which is subsequently hydrogenated. Fatty alcohols are mainly used in the production of detergents and surfactants. They are components also of cosmetic solvents. They find use as co-emulsifiers, emollients and thickeners in cosmetics.

In a preferred variant, the fatty alcohol is selected from the group consisting of phenyl propanol, dimethyl phenylbutanol, hexyldecanol, octyldodecanol, octyldecanol, tridecylalcohol, isostearyl alcohol, phenylisohexanol, phenylpropanol, trimethylbenzenepropanol, isoamylalcohol, isostearyl alcohol, and isotridecyl alcohol. The fatty alcohol can be used either as a single component or in mixture with one or more further different fatty alcohol(s) as specified above.

A fatty acid ester is a type of ester that results from the combination of a fatty acid with an alcohol. When the alcohol component is glycerol, the fatty acid esters produced can be monoglycerides, diglycerides or triglycerides. Fatty acid esters have a conditioning effect of softening the skin to create a smoothing sensation. They are also added to cosmetics to dissolve high-polarity active ingredients and UV absorbers. Esters of straight-chain fatty acids and lower alcohols are effective for dissolving slightly soluble ingredients for oils with a light touch during application. Isostearic acids and other liquid oils with branched fatty acids and unsaturated fatty acids are commonly used as emollients. Higher fatty acid esters and esters of higher alcohols with relatively high melting points are added to skin creams to adjust the application touch.

In a preferred variant, the fatty acid ester is selected from the group consisting of C12 - C15 Alkyl Benzoate, Capric/Lauric/Myristic/Oleic Triglyceride, Caprylic/Capric Triglyceride, Caprylic/Capric/Lauric Triglyceride, Caprylic/Capric/Linoleic Triglyceride, Caprylic/Capric/Myristic/Stearic Triglyceride, Caprylic/Capric/Palmitic/Stearic Triglyceride, Caprylic/Capric/Stearic Triglyceride, Caprylic/Capric/Succinic Triglyceride, Caprylyl Caprylate, Cetearyl Ethylhexanoate, Cetearyl Isononanoate, Cetearyl Nonanoate, Coco-Caprylate, Decyl Cocoate, Decyl Oleate, Dicaprylyl Carbonate, Diethyl Succinate, Diethylhexyl 2,6-Naphthalate, Diethylhexyl Carbonate, Dibutyl Adipate, Diisopropyl Adipate, Dipropylheptyl Carbonate, Ethyl Laurate, Ethylhexyl Isononanoate, Ethylhexyl Palmitate, Ethylhexyl Stearate, Glyceryl Caprylate Caprate, Glyceryl Caprylate, Glyceryl Laurate, Glyceryl Triacetyl Hydroxystearate, Glyceryl Triacetyl Ricinoleate, Hexyl Laurate, Isoamyl acetate, Isoamyl Cocoate, Isopropyl Palmitate, Isosorbide Dicaprylate, Isopropyl Myristate, Myristyl Myristate, Oleic/Linoleic Triglyceride, Oleic/Palmitic Triglyceride, Oleic/Palmitic/Lauric/Myristic/Linoleic Triglyceride, Propanediol Caprylate, Ricinoleic/Caproic/Caprylic/ Capric Triglyceride, Oleostearine, Oleyl Erucate, Palmitic/Stearic Triglyceride, Propanediol Dicaprylate Caprate, Propylheptyl Caprylate, Stearyl Heptanoate/Stearyl Caprylate, Triheptanoin, Trihydroxystearin, Triisononanoin, Triisopalmitin, Triisostearin, Trilaurin, Trilinolein, Trilinolenin, Trimyristin, Triolein, Tripalmitin, Tripalmitolein, Tripelargonin, Triricinolein, and Tristearin. The fatty acid ester can be used either as a single component or in mixture with one or more further different fatty ester(s) as specified above.

An UV filter is a compound or a mixture of compounds that block or absorb ultraviolet (UV) light. Since excessive UV radiation can cause sunburn, photoaging, and skin cancer, care products such as sunscreen usually include a classification for the specific wavelengths they filter. UV classifications include UVA (320 - 400 nm), UVB (290 - 320 nm) and UVC (200 - 280 nm). UV-absorbing compounds are used not only in sunscreen, but also in other personal care products, such as lipstick, shampoo, hair spray, body wash, toilet soap, and insect repellent. Chemical filters protect against UV radiation by absorbing, reflecting, or scattering. Reflection and scattering are accomplished by inorganic physical UV filters, such as titanium dioxide (TiO₂) and zinc oxide (ZnO). Absorption, mainly of UVB, is done by organic UV filters, which are known as chemical UV filters.

In a preferred variant, the liquid UV filter is selected from the group consisting of Octocrylene, Ethylhexyl Salicylate, Homosalate, Ethylhexyl Methoxycinnamate, Isoamyl p-Methoxycinnamate, Camphor Benzalkonium Methosulfate, Polyarylamidomethyl Benzylidene Camphor, Isooctyl Methoxycionnamate, Ethylhexyl Triazone, Drometrizole Trisiloxane, Diethylhexyl Butamido Triazone, 3-Benzylidene Camphor, Octyl Salicylate, Ethylhexyl Dimethyl PABA, Bis-Ethylhexyloxyphenol Methoxyphenyl Triazine, Polysilicone-15, Diethylamino Hydroxybenzoyl Hexyl benzoate, N,N,N-Trimethyl-4-(2-oxoborn-3-ylidenemethyl) anilinium methyl sulfate, Benzoic acid-2-hydroxy-3,3,5-trimethylcyclohexyl ester/Homosalate, 2-Cyano-3,3-diphenyl acrylic acid, 2- Ethylhexyl ester/Octocrilene, 2-Ethylhexyl-4-methoxycinnamate/Octinoxate, Ethoxylated Ethyl-4-aminobenzoate, 2-Ethylhexyl salicylate/Octisalate, 2-Ethylhexyl-4-(dimethylamino)benzoate/Padimate O (USAN:BAN), 2,2'-Methylene-bis(6-(2H-benzotriazol-2-yl)-4-(1,1,3,3-tetramethylbutyl)phenol)/Bisoctrizole, and Dimethico-diethylbenzalmalonate. The aforementioned UV filter(s) can be used either as a single component or in mixture with one or more further different UV filter(s) as further described below.

In addition to the oil phase or oil component as defined above, the cosmetic or pharmaceutical composition according to the present invention preferably includes one or more oil bodies. Suitable oil bodies, which form constituents of the O/W emulsions, are, for example, Guerbet alcohols based on fatty alcohols having 6 to 18, preferably 8 to 10, carbon atoms, esters of linear C5 - C22 fatty acids with linear or branched C5 - C22 fatty alcohols or esters of branched C1 - C13 carboxylic acids with linear or branched C6 - C22 fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6 - C22 fatty acids with branched alcohols, in particular 2-ethylhexanol, esters of C18 - C38 alkylhydroxy carboxylic acids with linear or branched C6 - C22 fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C6 - C10 fatty acids, liquid mono-/di-/triglyceride mixtures based on C6 - C18 fatty acids, esters of C6 - C22 fatty alcohols and/or Guerbet alcohols with aromatic carboxylic acids, in particular benzoic acid, esters of C2 - C12 dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched CC - C22 fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

Within the context of the present invention, it is also possible and in some cases advantageous to combine the cosmetic or pharmaceutical composition or homecare product according to the present invention with other customary active substances, adjuvants or additives.

Optionally, other conventional active substances, adjuvants or additives for cosmetic or pharmaceutical compositions or homecare products, as further described below, may be added as component (c), i.e. in order to obtain a ready-for-use composition or formulation.

The cosmetic or pharmaceutical composition or homecare product according to the present invention can advantageously be combined with other active agents and/or adjuvants and/or additives or auxiliaries, such as are customarily used in such compositions, such as for example abrasives, anti-acne agents, agents against ageing of the skin, anti-cellulitis agents, anti-dandruff agents, anti-inflammatory agents, irritation-preventing agents, irritation-inhibiting agents, antioxidants, astringents, odor absorbers, perspiration-inhibiting agents, antiseptic agents, anti-statics, binders, buffers, carrier materials, chelating agents, cell stimulants, cleansing agents, depilatory agents, surface-active substances, deodorizing agents, antiperspirants, softeners, emulsifiers, enzymes, enzyme inhibitors, essential oils, fibers, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, dyes, friction-reducing agents, lubricants, moisturizing creams, ointments, opacifying agents, plasticizing agents, covering agents, polish, preservatives, gloss agents, synthetic polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, surfactants, UV-absorbing agents, UV filters, primary sun protection factors, secondary sun protection factors, detergents, fabric conditioning agents, suspending agents, skin-tanning agents, actives modulating skin or hair pigmentation, matrix-metalloproteinase inhibitors, skin moisturizing agents, glycosaminoglycan stimulators, TRPV1 antagonists, desquamating agents, anti-cellulite agents or fat enhancing agents, hair growth activators or inhibitors, thickeners, rheology additives, vitamins, oils, waxes, pearlizing waxes, fats, phospholipids, saturated fatty acids, mono- or polyunsaturated fatty acids, α-hydroxy acids, polyhydroxyfatty acids, liquefiers, dyestuffs, colour-protecting agents, pigments, anticorrosives, fragrances or perfume oils, aromas, flavouring substances, odoriferous substances, polyols, electrolytes, organic solvents, and mixtures of two or more of the aforementioned substances, as further described below.

Of the above cosmetically or pharmaceutically active agents and/or adjuvants and/or additives or auxiliaries against ageing of the skin, antioxidants, chelating agents, emulsifiers, surfactants, preservatives, synthetic polymers, skin-cooling agents, rheology additives, oils, fragrances or perfume oils, and polyols are particularly preferred in the preparation of cosmetic and pharmaceutical composition.

Since dermatological conditions or diseases are often associated with dry skin, scratched skin, skin lesions or even inflammation, the cosmetic or pharmaceutical composition according to the present invention advantageously contains preferably anti-inflammatories, antibacterial or antimycotic substances, substances having a reddening-alleviating or itch-alleviating action, lenitive substances, moisturisers and/or cooling agents, osmolytes, keratolytic substances, nurturing substances, anti-inflammatory, antibacterial or antimycotic substances, substances having a reddening-alleviating or itch-alleviating action, lenitive substances, anti-dandruff substances, or other active compounds such as solvents, fragrances antioxidants, preservatives, (metal) chelating agents, penetration enhancers, or mixtures of two or more of afore specified agents, as further described below.

The cosmetically or pharmaceutically active agents and/or adjuvants and/or additives can in some instances provide one or more than one benefit or operate via more than one mode of action.

**Anti-ageing** actives: The cosmetic or pharmaceutical composition according to the present invention preferably contains one or more anti-ageing actives. In the context of the invention, anti-ageing or biogenic agents are, for example antioxidants, matrix-metalloproteinase inhibitors (MMPI), skin moisturizing agents, glycosaminglycan stimulators, anti-inflammatory agents, TRPV1 antagonists and plant extracts.

**Antioxidants:** A preferred cosmetic or pharmaceutical composition according to the present invention comprises one or more antioxidants. Suitable antioxidants encompass amino acids (preferably glycine, histidine, tyrosine, tryptophan) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone. If vitamin E and/or derivatives thereof are used as the antioxidant(s), it is advantageous to choose their concentrations from the range from about 0.001 to about 10 % by weight, based on the total weight of the composition. If vitamin A or vitamin A derivatives or carotenes or derivatives thereof are used as the antioxidant(s), it is advantageous to choose their concentrations from the range from about 0.001 to about 10 % by weight based on the total weight of the composition.

**Matrix-Metalloproteinase inhibitors (MMPI):** A preferred cosmetic or pharmaceutical composition according to the present invention comprises one or more matrix-metalloproteinase inhibitors, especially those inhibiting matrix-metalloproteinases enzymatically cleaving collagen, selected from the group consisting of ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf, as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**Skin-moisturizing agents:** A preferred cosmetic or pharmaceutical composition according to the present invention comprises one or more skin-moisturizing agents. Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators:** A preferred cosmetic or pharmaceutical composition according to the present invention comprises one or more substances stimulating the synthesis of glycosaminoglycans which are selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**TRPV1 antagonists:** A preferred cosmetic or pharmaceutical composition according to the present invention comprises one or more TRPV1 antagonists. Suitable compounds which reduce the hypersensitivity of skin nerves based on their action as TRPV1 antagonists, encompass e.g. trans-4-tert-butyl cyclohexanol, or indirect modulators of TRPV1 by an activation of the µ-receptor, e.g. acetyl tetrapeptide-15, are preferred.

**Plant extracts:** A preferred cosmetic or pharmaceutical composition according to the present invention comprises one or more plant extracts. Plant extracts, special highly active plant extract fractions and also highly pure active substances isolated from plant extracts can also be used in the cosmetic or pharmaceutical composition according to the present invention. Extracts, fractions and active substances from camomile, *aloe vera, Commiphora* species, *Rubia* species, willows, willow-herb, ginger, marigold, arnica, Glycyrrhiza species, Echinacea species, Rubus species and pure substances such as *inter alia* bisabolol, apigenin, apigenin-7-glucoside, gingerols such as [6]-gingerol, paradols such as [6]-paradol, boswellic acid, phytosterols, glycyrrhizine, glabridin or licochalcone A are particularly preferred.

**Anti-inflammatory agents:** The cosmetic or pharmaceutical composition according to the present invention preferably also contains anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. More particularly:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and ß-glucans, in particular 1,3->1,4-ß-glucan from oats.

When bisabolol is used in the context of the present invention it can be of natural or synthetic origin and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide (CO₂), hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

**Physiological cooling agents:** The cosmetic or pharmaceutical composition according to the present invention can be particularly advantageously combined with one or more physiological cooling agent(s). The use of cooling agents can alleviate itching. Preferred individual cooling agents for use within the framework of the present invention are listed below. The person skilled in the art can add many other cooling agents to this list; the cooling agents listed can also be used in combination with one another: which are preferably selected here from the following list: menthol and menthol derivatives (for example L-menthol, D-menthol, racemic menthol, isomenthol, neoisomenthol, neomenthol) menthylethers (for example (I-menthoxy)-1,2-propanediol, (I-menthoxy)-2-methyl-1,2-propanediol, I-menthyl-methylether), menthone glyceryl acetal, menthone glyceryl ketal or mixtures of both, menthylesters (for example menthylformiate, menthylacetate, menthylisobutyrate, menthyhydroxyisobutyrat, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], menthanecarboxylic acid-N-(4-cyanophenyl)amide or menthanecarboxylic acid-N-(4-cyanomethylphenyl)amide, menthanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates and [(1R,2S,5R)-2-isopropyl-5-methyl-cyclohexyl] 2-(ethylamino)-2-oxo-acetate (X Cool). Cooling agents which are preferred due to their particular synergistic effect are I-menthol, d-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), substituted menthyl-3-carboxamides (such as menthyl-3-carboxylic acid N-ethyl amide), 2-isopropyl-N-2,3-trimethyl butanamide, substituted cyclohexane carboxamides, 3-menthoxypropane-1,2-diol, 2-hydroxyethyl menthyl carbonate, 2-hydroxypropyl menthyl carbonate and isopulegol. Particularly preferred cooling agents are I-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), 3-menthoxypropane-1,2-diol, 2-hydroxyethyl menthyl carbonate and 2-hydroxypropyl menthyl carbonate. Very particularly preferred cooling agents are I-menthol, menthone glycerol acetal (trade name: Frescolat^{®} MGA) and menthyl lactate (preferably I-menthyl lactate, in particular I-menthyl I-lactate (trade name: Frescolat^{®} ML).

**Moisturising and/or moisture-retaining substances:** Itching occurs with particular intensity when the skin is dry. The use of skin-moisturising and/or moisture-retaining substances can significantly alleviate itching. The cosmetic or pharmaceutical composition according to the present invention can therefore advantageously also contain one or more of the following moisturising and/or moisture-retaining substances: sodium lactate, urea, urea derivatives, alcohols, glycerol, diols such as propylene glycol, hexylene glycol, collagen, elastin or hyaluronic acid, diacyl adipates, petrolatum, urocanic acid, lecithin, panthenol, phytantriol, lycopene, (pseudo-)ceramides, glycosphingolipids, cholesterol, phytosterols, chitosan, chondroitin sulphate, lanolin, lanolin esters, amino acids, alpha-hydroxy acids (such as citric acid, lactic acid, malic acid) and their derivatives, mono-, di- and oligosaccharides such as glucose, galactose, fructose, mannose, fructose and lactose, polysugars such as R-glucans, in particular 1,3-1,4-β-glucan from oats, alpha-hydroxy fatty acids, triterpene acids such as betulinic acid or ursolic acid, and algae extracts.

**Lenitive substances:** The cosmetic or pharmaceutical composition according to the present invention can also contain advantageously one or more lenitive substances, wherein any lenitive substances can be used which are suitable or customary in cosmetic or pharmaceutical applications such as alpha-bisabolol, azulene, guaiazulene, 18-beta-glycyrrhetinic acid, allantoin, Aloe vera juice or gel, extracts of Hamamelis virginiana (witch hazel), Echinacea species, Centella asiatica, chamomile, Arnica monatana, Glycyrrhiza species, algae, seaweed and Calendula officinalis, and vegetable oils such as sweet almond oil, baobab oil, olive oil and panthenol, Laureth-9, Trideceth-9 and 4-t-butylcyclohexanol.

**Antibacterial or antimycotic active substances:** Antibacterial or antimycotic active substances can also particularly advantageously be used in the cosmetic or pharmaceutical composition according to the present invention, wherein any antibacterial or antimycotic active substances can be used which are suitable or customary in cosmetic or pharmaceutical, in particular dermatological applications. In addition to the large group of conventional antibiotics, other products which are advantageous here include those relevant to cosmetics such as in particular triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate or combinations of said substances, which are used *inter alia* against underarm odour, foot odour or dandruff.

**Desquamating agents:** The cosmetic or pharmaceutical composition according to the present invention preferably contains one or more desquamating agents. The expression "desquamating agent" is understood to mean any compound capable of acting:
- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosulphonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine^{®}, prickly pear extracts such as those marketed under the name Exfolactive^{®} by the company SILAB, or Phytosphingosine SLC^{®} (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, chestnut or prickly pear extracts, in particular marketed by SILAB; reducing compounds such as cysteine or cysteine precursors. Desquamating agents which can be used are also nicotinic acid and its esters and nicotinamide, also called vitamin B3 or vitamin PP, and ascorbic acid and its precursors.

**Anti-dandruff substances:** In addition, the cosmetic or pharmaceutical composition according to the present invention can also advantageously be used in combination with one or more anti-dandruff substances, including triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate, Propanediol Monocaprylate or combinations of said substances, which are used inter alia against dandruff.

Further suitable anti-dandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-{4-{2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

**(Metal) chelating agents:** A combination with one or more (metal) chelating agents can also be advantageous used in the cosmetic or pharmaceutical composition according to the present invention, wherein any metal chelating agents can be used which are suitable or customary in cosmetic or pharmaceutical applications. Preferred (metal) chelating agents include α-hydroxy fatty acids, phytic acid, lactoferrin, α-hydroxy acids, such as *inter alia* gluconic acid, glyceric acid, glycolic acid, isocitric acid, citric acid, lactic acid, malic acid, mandelic acid, tartaric acid, as well as humic acids, bile acids, bile extracts, bilirubin, biliverdin or EDTA, EGTA and their derivatives. The use of one or more chelating agent(s) improves the stability of the composition according to the present invention.

**Emulsifiers:** In addition, the cosmetic or pharmaceutical composition according to the present invention can also advantageously contain one or more emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:
Partial glycerides: Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Sorbitan esters: Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Polyglycerol esters: Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate, and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Anionic emulsifiers: Typical anionic emulsifiers are aliphatic C12 to C 22 fatty acids, such as palmitic acid, stearic acid or behenic acid for example, and C12 to C22 dicarboxylic acids, such as azelaic acid or sebacic acid for example.

Amphoteric emulsifiers: Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-coco-alkylaminopropionate, cocoacylaminoethyl aminopropionate and C_{12/18} acyl sarcosine.

**Surfactants:** The cosmetic or pharmaceutical composition according to the present invention preferably includes one or more anionic and/or amphoteric or zwitterionic surfactants. Typical examples encompass: Almondamidopropylamine Oxide, Almondamidopropyl Betaine, Aminopropyl Laurylglutamine, Ammonium C12-15 Alkyl Sulfate, Ammonium C12-16 Alkyl Sulfate, Ammonium Capryleth Sulfate, Ammonium Cocomonoglyceride Sulfate, Ammonium Coco-Sulfate, Ammonium Cocoyl Isethionate, Ammonium Cocoyl Sarcosinate, Ammonium C12-15 Pareth Sulfate, Ammonium C9-10 Perfluoroalkylsulfonate, Ammonium Dinonyl Sulfosuccinate, Ammonium Dodecylbenzenesulfonate, Ammonium Isostearate, Ammonium Laureth-6 Carboxylate, Ammonium Laureth-8 Carboxylate, Ammonium Laureth Sulfate, Ammonium Laureth-5 Sulfate, Ammonium Laureth-7 Sulfate, Ammonium Laureth-9 Sulfate, Ammonium Laureth-12 Sulfate, Ammonium Lauroyl Sarcosinate, Ammonium Lauryl Sulfate, Ammonium Lauryl Sulfosuccinate, Ammonium Myreth Sulfate, Ammonium Myristyl Sulfate, Ammonium Nonoxynol-4 Sulfate, Ammonium Nonoxynol-30 Sulfate, Ammonium Oleate, Ammonium Palm Kernel Sulfate, Ammonium Stearate, Ammonium Tallate, AMPD-Isostearoyl Hydrolyzed Collagen, AMPD-Rosin Hydrolyzed Collagen, AMP-Isostearoyl Hydrolyzed Collagen, AMP-Isostearoyl Hydrolyzed Keratin, AMP-Isostearoyl Hydrolyzed Soy Protein, AMP-Isostearoyl Hydrolyzed Wheat Protein, Apricotamidopropyl Betaine, Arachidic Acid, Arginine Hexyldecyl Phosphate, Avocadamidopropyl Betaine, Avocado Oil Glycereth-8 Esters, Babassu Acid, Babassuamidopropylamine Oxide, Babassuamidopropyl Betaine, Beeswax Acid, Behenamidopropyl Betaine, Behenamine Oxide, Beheneth-25, Beheneth-30, Behenic Acid, Behenyl Betaine, Bis- Butyldimethicone Polyglyceryl-3, Butoxynol-5 Carboxylic Acid, Butoxynol-19 Carboxylic Acid, Butyldimoniumhydroxypropyl Butylglucosides Chloride, Butyldimoniumhydroxypropyl Laurylglucosides Chloride, Butyl Glucoside, Butylglucoside Caprate, Butylglucosides Hydroxypropyltrimonium Chloride, Butyloctanoic Acid, C18-36 Acid, C20-40 Acid, C30-50 Acid, C16-22 Acid Amide MEA, Calcium Dodecylbenzenesulfonate, Calcium Lauroyl Taurate, C9-16 Alkane/Cycloalkane, C10-14 Alkyl Benzenesulfonic Acid, C12-14 Alkyl Diaminoethylglycine HCL, C9-15 Alkyl Phosphate, Candida Bombicola/Glucose/Methyl Rapeseedate Ferment, Canolamidopropyl Betaine, Capric Acid, Caproic Acid, Caproyl Ethyl Glucoside, Capryl/Capramidopropyl Betaine, Capryleth-4 Carboxylic Acid, Capryleth-6 Carboxylic Acid, Capryleth-9 Carboxylic Acid, Caprylic Acid, Capryloyl Collagen Amino Acids, Capryloyl Glycine, Capryloyl Hydrolyzed Collagen, Capryloyl Hydrolyzed Keratin, Capryloyl Keratin Amino Acids, Capryloyl Silk Amino Acids, Caprylyl/Capryl Glucoside, Caprylyl/Capryl Wheat Bran/Straw Glycosides, Caprylyl Glucoside, Caprylyl Glyceryl Ether, Caprylyl Pyrrolidone, Carnitine, Ceteareth-20, Ceteareth-23, Ceteareth-24, Ceteareth-25, Ceteareth-27, Ceteareth-28, Ceteareth-29, Ceteareth-30, Ceteareth-33, Ceteareth-34, Ceteareth-40, Ceteareth-50, Ceteareth-55, Ceteareth-60, Ceteareth-80, Ceteareth-100, Ceteareth-25 Carboxylic Acid, Ceteareth-2 Phosphate, Ceteareth-4 Phosphate, Ceteareth-5 Phosphate, Ceteareth-10 Phosphate, Ceteth-20, Ceteth-23, Ceteth-24, Ceteth-25, Ceteth-30, Ceteth-40, Ceteth-45, Ceteth-150, Ceteth-8 Phosphate, Ceteth-10 Phosphate, Ceteth-20 Phosphate, Cetoleth-22, Cetoleth-24, Cetoleth-25, Cetoleth-30, Cetyl Betaine, Chrysanthemum Sinense Flower Extract, C12-14 Hydroxyalkyl Hydroxyethyl Beta-Alanine, C12-14 Hydroxyalkyl Hydroxyethyl Sarcosine, Cocamidoethyl Betaine, Cocamidopropylamine Oxide, Cocamidopropyl Betainamide MEA Chloride, Cocamidopropyl Betaine, Cocamidopropyl Hydroxysultaine, Cocamine Oxide, Cocaminobutyric Acid, Cocaminopropionic Acid, Coceth-7 Carboxylic Acid, Coceth-4 Glucoside, Cocoamphodipropionic Acid, Cocobetainamido Amphopropionate, Coco-Betaine, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Coco-Glucoside, Cocoglucosides Hydroxypropyltrimonium Chloride, Coco- Hydroxysultaine, Coco-Morpholine Oxide, Coconut Acid, Coconut Oil Glycereth-8 Esters, Coco/Oleamidopropyl Betaine, Coco-Sultaine, Coco/Sunfloweramidopropyl Betaine, Cocoylcholine Methosulfate, Cocoyl Glutamic Acid, Cocoyl Hydrolyzed Collagen, Cocoyl Hydrolyzed Keratin, Cocoyl Hydrolyzed Oat Protein, Cocoyl Hydrolyzed Rice Protein, Cocoyl Hydrolyzed Silk, Cocoyl Hydrolyzed Soy Protein, Cocoyl Hydrolyzed Wheat Protein, Cocoyl Sarcosine, Corn Acid, Cottonseed Acid, Cottonseed Oil Glycereth-8 Esters, C10-16 Pareth-1, C10-16 Pareth-2, C11-13 Pareth-6, C11-13 Pareth-9, C11-13 Pareth-10, C11-15 Pareth-30, C11-15 Pareth-40, C12-13 Pareth-1, C12-13 Pareth- 23, C12-14 Pareth-5, C12-14 Pareth-9, C13-15 Pareth-21, C14-15 Pareth-8, C20-22 Pareth-30, C20- 40 Pareth-40, C20-40 Pareth-95, C22-24 Pareth-33, C30-50 Pareth-40, C9-11 Pareth-6 Carboxylic Acid, C9-11 Pareth-8 Carboxylic Acid, C11-15 Pareth-7 Carboxylic Acid, C12-13 Pareth-5 Carboxylic Acid, C12-13 Pareth-7 Carboxylic Acid, C12-13 Pareth-8 Carboxylic Acid, C12-13 Pareth-12 Carboxylic Acid, C12-15 Pareth-7 Carboxylic Acid, C12-15 Pareth-8 Carboxylic Acid, C12-15 Pareth- 12 Carboxylic Acid, C14-15 Pareth-8 Carboxylic Acid, C6-10 Pareth-4 Phosphate, C12-13 Pareth-2 Phosphate, C12-13 Pareth-10 Phosphate, C12-15 Pareth-6 Phosphate, C12-15 Pareth-8 Phosphate, C12-15 Pareth-10 Phosphate, C12-16 Pareth-6 Phosphate, C4-18 Perfluoroalkylethyl Thiohydroxypropyltrimonium Chloride, Cupuassuamidopropyl Betaine, DEA-C12-13 Alkyl Sulfate, DEA-C12-15 Alkyl Sulfate, DEA-Ceteareth-2 Phosphate, DEA-Cetyl Sulfate, DEA- Cocoamphodipropionate, DEA-C12-13 Pareth-3 Sulfate, DEA-Cyclocarboxypropyloleate, DEA- Dodecylbenzenesulfonate, DEA-Isostearate, DEA-Laureth Sulfate, DEA-Lauryl Sulfate, DEA- Linoleate, DEA-Methyl Myristate Sulfonate, DEA-Myreth Sulfate, DEA-Myristate, DEA-Myristyl Sulfate, DEA-Oleth-5 Phosphate, DEA-Oleth-20 Phosphate, DEA PG-Oleate, Deceth-7 Carboxylic Acid, Deceth-7 Glucoside, Deceth-9 Phosphate, Decylamine Oxide, Decyl Betaine, Decyl Glucoside, Decyltetradeceth-30, Decyltetradecylamine Oxide, Diammonium Lauramido-MEA Sulfosuccinate, Diammonium Lauryl Sulfosuccinate, Diammonium Oleamido PEG-2 Sulfosuccinate, Dibutoxymethane, Di-Cl 2-15 Pareth-2 Phosphate, Di-Cl 2-15 Pareth-4 Phosphate, Di-Cl 2-15 Pareth-6 Phosphate, Di- C12-15 Pareth-8 Phosphate, Di-Cl 2-15 Pareth-10 Phosphate, Didodecyl Butanetetracarboxylate, Diethylamine Laureth Sulfate, Diethylhexyl Sodium Sulfosuccinate, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Dimethicone PEG-7 Phosphate, Dimethicone PEG-10 Phosphate, Dimethicone PEG/PPG-7/4 Phosphate, Dimethicone PEG/PPG-12/4 Phosphate, Dimethicone/Polyglycerin-3 Crosspolymer, Dimethicone Propyl PG- Betaine, Dimyristyl Phosphate, Dioleoylamidoethyl Hydroxyethylmonium Methosulfate, DIPA-Hydrogenated Cocoate, DIPA-Lanolate, DIPA-Myristate, Dipotassium Capryloyl Glutamate, Dipotassium Lauryl Sulfosuccinate, Dipotassium Undecylenoyl Glutamate, Disodium Babassuamido MEA-Sulfosuccinate, Disodium Caproamphodiacetate, Disodium Caproamphodipropionate, Disodium Capryloamphodiacetate, Disodium Capryloamphodipropionate, Disodium Capryloyl Glutamate, Disodium Cetearyl Sulfosuccinate, Disodium Cetyl Phenyl Ether Disulfonate, Disodium Cetyl Sulfosuccinate, Disodium Cocamido MEA-Sulfosuccinate, Disodium Cocamido MIPA PEG-4 Sulfosuccinate, Disodium Cocamido MIPA-Sulfosuccinate, Disodium Cocamido PEG-3 Sulfosuccinate, Disodium Coceth-3 Sulfosuccinate, Disodium Cocoamphocarboxyethylhydroxypropylsulfonate, Disodium Cocoamphodiacetate, Disodium Cocoamphodipropionate, Disodium Coco-Glucoside Sulfosuccinate, Disodium Coco-Sulfosuccinate, Disodium Cocoyl Butyl Gluceth-10 Sulfosuccinate, Disodium Cocoyl Glutamate, Disodium C12-14 Pareth-1 Sulfosuccinate, Disodium C12-14 Pareth-2 Sulfosuccinate, Disodium C12-15 Pareth Sulfosuccinate, Disodium C12-14 Sec-Pareth-3 Sulfosuccinate, Disodium C12-14 Sec-Pareth-5 Sulfosuccinate, Disodium C12-14 Sec-Pareth-7 Sulfosuccinate, Disodium C12-14 Sec-Pareth-9 Sulfosuccinate, Disodium C12-14 Sec-Pareth-12 Sulfosuccinate, Disodium Deceth-5 Sulfosuccinate, Disodium Deceth-6 Sulfosuccinate, Disodium Decyl Phenyl Ether Disulfonate, Disodium Dihydroxyethyl Sulfosuccinylundecylenate, Disodium Ethylene Dicocamide PEG-15 Disulfate, Disodium Hydrogenated Cottonseed Glyceride Sulfosuccinate, Disodium Hydrogenated Tallow Glutamate, Disodium Hydroxydecyl Sorbitol Citrate, Disodium Isodecyl Sulfosuccinate, Disodium Isostearamido MEA-Sulfosuccinate, Disodium Isostearamido MIPA-Sulfosuccinate, Disodium Isostearoamphodiacetate, Disodium Isostearoamphodipropionate, Disodium Isostearyl Sulfosuccinate, Disodium Laneth-5 Sulfosuccinate, Disodium Lauramido MEA-Sulfosuccinate, Disodium Lauramido MIPA Glycol Sulfosuccinate, Disodium Lauramido PEG-2 Sulfosuccinate, Disodium Lauramido PEG-5 Sulfosuccinate, Disodium Laureth-5 Carboxyamphodiacetate, Disodium Laureth-7 Citrate, Disodium Laureth Sulfosuccinate, Disodium Laureth-6 Sulfosuccinate, Disodium Laureth-9 Sulfosuccinate, Disodium Laureth-12 Sulfosuccinate, Disodium Lauriminobishydroxypropylsulfonate, Disodium Lauriminodiacetate, Disodium Lauriminodipropionate, Disodium Lauriminodipropionate Tocopheryl Phosphates, Disodium Lauroamphodiacetate, Disodium Lauroamphodipropionate, Disodium N-Lauroyl Aspartate, Disodium Lauroyl Glutamate, Disodium Lauryl Phenyl Ether Disulfonate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleoamphodipropionate, Disodium Oleth-3 Sulfosuccinate, Disodium Oleyl Phosphate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PEG-12 Dimethicone Sulfosuccinate, Disodium PEG-8 Palm Glycerides Sulfosuccinate, Disodium PPG-2-Isodeceth-7 Carboxyamphodiacetate, Disodium Ricinoleamido MEA-Sulfosuccinate, Disodium Sitostereth-14 Sulfosuccinate, Disodium Soyamphodiacetate, Disodium Stearamido MEA-Sulfosuccinate, Disodium Steariminodipropionate, Disodium Stearoamphodiacetate, Disodium Stearoyl Glutamate, Disodium Stearyl Sulfosuccinamate, Disodium Stearyl Sulfosuccinate, Disodium 2-Sulfolaurate, Disodium 2-Sulfopalmitate, Disodium Tallamido MEA-Sulfosuccinate, Disodium Tallowamido MEA-Sulfosuccinate, Disodium Tallowamphodiacetate, Disodium Tallowiminodipropionate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Undecylenoyl Glutamate, Disodium Wheat Germamido MEA-Sulfosuccinate, Disodium Wheat Germamido PEG-2 Sulfosuccinate, Disodium Wheatgermamphodiacetate, Di-TEA-Cocamide Diacetate, Di-TEA-Oleamido PEG-2 Sulfosuccinate, Di-TEA-Palmitoyl Aspartate, Ditridecyl Sodium Sulfosuccinate, Dodecylbenzene Sulfonic Acid, Erucamidopropyl Hydroxysultaine, Ethylhexeth-3 Carboxylic Acid, Ethyl PEG-15 Cocamine Sulfate, Glyceryl Capryl Ether, Hexyldecanoic Acid, Hydrogenated Coconut Acid, Hydrogenated Laneth-25, Hydrogenated Menhaden Acid, Hydrogenated Palm Acid, Hydrogenated Palm Kernel Amine Oxide, Hydrogenated Tallow Acid, Hydrogenated Tallowamine Oxide, Hydrogenated Tallow Betaine, Hydrogenated Talloweth-25, Hydrogenated Tallowoyl Glutamic Acid, Hydrolyzed Candida Bombicola Extract, Hydroxyceteth-60, Hydroxyethyl Acetomonium PG-Dimethicone, Hydroxyethylbutylamine Laureth Sulfate, Hydroxyethyl Carboxymethyl Cocamidopropylamine, Hydroxyethyl Hydroxypropyl C12-15 Alkoxypropylamine Oxide, Hydroxylauryl/Hydroxymyristyl Betaine, Hydroxystearic Acid, Hydroxysuccinimidyl C10-40 Isoalkyl Acidate, Hydroxysuccinimidyl C21-22 Isoalkyl Acidate, Hydroxysultaines, IPDI/PEG-15 Soyamine Oxide Copolymer, IPDI/PEG-15 Soyethonium Ethosulfate Copolymer, IPDI/PEG-15 Soy Glycinate Copolymer, Isoceteth-30, Isolaureth-4 Phosphate, Isopolyglyceryl-3 Dimethicone, Isopolyglyceryl-3 Dimethiconol, Isopropanolamine Lanolate, Isopropylamine Dodecylbenzenesulfonate, Isostearamidopropylamine Oxide, Isostearamidopropyl Betaine, Isostearamidopropyl Morpholine Oxide, Isosteareth-8, Isosteareth-16, Isosteareth-22, Isosteareth-25, Isosteareth-50, Isostearic Acid, Isostearoyl Hydrolyzed Collagen, Jojoba Oil PEG-150 Esters, Jojoba Wax PEG-80 Esters, Jojoba Wax PEG-120 Esters, Laneth-20, Laneth-25, Laneth-40, Laneth-50, Laneth-60, Laneth-75, Lanolin Acid, Lauramidopropylamine Oxide, Lauramidopropyl Betaine, Lauramidopropyl Hydroxysultaine, Lauramine Oxide, Lauraminopropionic Acid, Laurdimoniumhydroxypropyl Decylglucosides Chloride, Laurdimoniumhydroxypropyl Laurylglucosides Chloride, Laureth-16, Laureth-20, Laureth-21, Laureth-23, Laureth-25, Laureth-30, Laureth-38, Laureth-40, Laureth-3 Carboxylic Acid, Laureth-4 Carboxylic Acid, Laureth-5 Carboxylic Acid, Laureth- 6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, Laureth-6 Citrate, Laureth-7 Citrate, Laureth-1 Phosphate, Laureth-2 Phosphate, Laureth-3 Phosphate, Laureth-4 Phosphate, Laureth-7 Phosphate, Laureth-8 Phosphate, Laureth-7 Tartrate, Laurie Acid, Laurimino Bispropanediol, Lauriminodipropionic Acid, Lauroamphodipropionic Acid, Lauroyl Beta-Alanine, Lauroyl Collagen Amino Acids, Lauroyl Ethyltrimonium Methosulfate, Lauroyl Hydrolyzed Collagen, Lauroyl Hydrolyzed Elastin, Lauroyl Methyl Glucamide, Lauroyl Sarcosine, Lauroyl Silk Amino Acids, Lauryl Betaine, Lauryl Dimethicone/Polyglycerin-3 Crosspolymer, Lauryldimoniumhydroxypropyl Cocoglucosides Chloride, Lauryl Glucoside, Laurylglucosides Hydroxypropyltrimonium Chloride, Lauryl Glycol Hydroxypropyl Ether, Lauryl Hydroxysultaine, Lauryl Malamide, Lauryl Methylglucamide, Lauryl/Myristyl Glycol Hydroxypropyl Ether, Lauryl/Myristyl Wheat Bran/Straw Glycosides, Lauryl Polyglyceryl-3 Polydimethylsiloxyethyl Dimethicone, Lauryl Pyrrolidone, Lauryl Sultaine, Linoleic Acid, Linolenic Acid, Linseed Acid, Lysine Cocoate, Macadamia Seed Oil Glycereth-8 Esters, Magnesium Coceth Sulfate, Magnesium Coco-Sulfate, Magnesium Isododecylbenzenesulfonate, Magnesium Laureth-11 Carboxylate, Magnesium Laureth Sulfate, Magnesium Laureth-5 Sulfate, Magnesium Laureth-8 Sulfate, Magnesium Laureth-16 Sulfate, Magnesium Laureth-3 Sulfosuccinate, Magnesium Lauryl Hydroxypropyl Sulfonate, Magnesium Lauryl Sulfate, Magnesium Methyl Cocoyl Taurate, Magnesium Myreth Sulfate, Magnesium Oleth Sulfate, Magnesium/TEA-Coco-Sulfate, Manicouagan Clay, MEA-Cocoate, MEA-Laureth-6 Carboxylate, MEA- Laureth Sulfate, MEA-Lauryl Sulfate, MEA PPG-6 Laureth-7 Carboxylate, MEA-PPG-8-Steareth-7 Carboxylate, MEA-Undecylenate, Meroxapol 108, Meroxapol 174, Meroxapol 178, Meroxapol 254, Meroxapol 255, Meroxapol 258, Meroxapol 314, Methoxy PEG-450 Amidoglutaroyl Succinimide, Methoxy PEG-450 Amido Hydroxysuccinimidyl Succinamate, Methoxy PEG-450 Maleimide, Methyl Morpholine Oxide, Milkamidopropyl Amine Oxide, Milkamidopropyl Betaine, Minkamidopropylamine Oxide, Minkamidopropyl Betaine, MIPA C12-15 Pareth Sulfate, MIPA-Dodecylbenzenesulfonate, MIPA-Laureth Sulfate, MIPA-Lauryl Sulfate, Mixed Isopropanolamines Lanolate, Mixed Isopropanolamines Lauryl Sulfate, Mixed Isopropanolamines Myristate, Morpholine Oleate, Morpholine Stearate, Myreth-3 Carboxylic Acid, Myreth-5 Carboxylic Acid, Myristalkonium Chloride, Myristamidopropylamine Oxide, Myristamidopropyl Betaine, Myristamidopropyl Dimethylamine Phosphate, Myristamidopropyl Hydroxysultaine, Myristamidopropyl PG-Dimonium Chloride Phosphate, Myristamine Oxide, Myristaminopropionic Acid, Myristic Acid, Myristoyl Ethyltrimonium Methosulfate, Myristoyl Glutamic Acid, Myristoyl Hydrolyzed Collagen, Myristoyl Sarcosine, Myristyl Betaine, Myristyl/Cetyl Amine Oxide, Myristyldimoniumhydroxypropyl Cocoglucosides Chloride, Myristyl Glucoside, Myristyl Phosphate, Nonoxynol-20, Nonoxynol-23, Nonoxynol-25, Nonoxynol-30, Nonoxynol-35, Nonoxynol-40, Nonoxynol-44, Nonoxynol-50, Nonoxynol-100, Nonoxynol-120, Nonoxynol-5 Carboxylic Acid, Nonoxynol-8 Carboxylic Acid, Nonoxynol-10 Carboxylic Acid, Nonoxynol-3 Phosphate, Nonoxynol-4 Phosphate, Nonoxynol-6 Phosphate, Nonoxynol-9 Phosphate, Nonoxynol-10 Phosphate, Nonyl Nonoxynol-30, Nonyl Nonoxynol-49, Nonyl Nonoxynol-100, Nonyl Nonoxynol-150, Nonyl Nonoxynol-7 Phosphate, Nonyl Nonoxynol-8 Phosphate, Nonyl Nonoxynol-9 Phosphate, Nonyl Nonoxynol-10 Phosphate, Nonyl Nonoxynol-11 Phosphate, Nonyl Nonoxynol-15 Phosphate, Nonyl Nonoxynol-24 Phosphate, Oatamidopropyl Betaine, Octoxynol-16, Octoxynol-25, Octoxynol-30, Octoxynol-33, Octoxynol-40, Octoxynol-70, Octoxynol-20 Carboxylic Acid, Octyldodeceth-20, Octyldodeceth-25, Octyldodeceth-30, Oleamidopropylamine Oxide, Oleamidopropyl Betaine, Oleamidopropyl Hydroxysultaine, Oleamine Oxide, Oleic Acid, Oleoyl Hydrolyzed Collagen, Oleoyl Sarcosine, Oleth-20, Oleth-23, Oleth-24, Oleth-25, Oleth-30, Oleth-35, Oleth-40, Oleth-44, Oleth-50, Oleth-3 Carboxylic Acid, Oleth-6 Carboxylic Acid, Oleth-10 Carboxylic Acid, Oleyl Betaine, Olivamidopropylamine Oxide, Olivamidopropyl Betaine, Olive Acid, Olivoyl Hydrolyzed Wheat Protein, Ophiopogon Extract Stearate, Ozonized Oleth-10, Ozonized PEG-10 Oleate, Ozonized PEG-14 Oleate, Ozonized Polysorbate 80, Palm Acid, Palmamidopropyl Betaine, Palmeth-2 Phosphate, Palmitamidopropylamine Oxide, Palmitamidopropyl Betaine, Palmitamine Oxide, Palmitic Acid, Palmitoyl Collagen Amino Acids, Palmitoyl Glycine, Palmitoyl Hydrolyzed Collagen, Palmitoyl Hydrolyzed Milk Protein, Palmitoyl Hydrolyzed Wheat Protein, Palmitoyl Keratin Amino Acids, Palmitoyl Oligopeptide, Palmitoyl Silk Amino Acids, Palm Kernel Acid, Palm Kernelamidopropyl Betaine, Peach Kernel Oil Glycereth-8 Esters, Peanut Acid, PEG-10 Castor Oil, PEG-40 Castor Oil, PEG-44 Castor Oil, PEG-50 Castor Oil, PEG-54 Castor Oil, PEG-55 Castor Oil, PEG-60 Castor Oil, PEG-80 Castor Oil, PEG-100 Castor Oil, PEG-200 Castor Oil, PEG-11 Cocamide, PEG-6 Cocamide Phosphate, PEG-4 Cocamine, PEG-8 Cocamine, PEG-12 Cocamine, PEG-150 Dibehenate, PEG-90 Diisostearate, PEG-75 Dilaurate, PEG-150 Dilaurate, PEG-75 Dioleate, PEG-150 Dioleate, PEG-75 Distearate, PEG-120 Distearate, PEG-150 Distearate, PEG-175 Distearate, PEG-190 Distearate, PEG-250 Distearate, PEG-30 Glyceryl Cocoate, PEG-40 Glyceryl Cocoate, PEG-78 Glyceryl Cocoate, PEG-80 Glyceryl Cocoate, PEG-30 Glyceryl Isostearate, PEG-40 Glyceryl Isostearate, PEG-50 Glyceryl Isostearate, PEG-60 Glyceryl Isostearate, PEG-90 Glyceryl Isostearate, PEG-23 Glyceryl Laurate, PEG-30 Glyceryl Laurate, PEG-25 Glyceryl Oleate, PEG-30 Glyceryl Oleate, PEG-30 Glyceryl Soyate, PEG-25 Glyceryl Stearate, PEG-30 Glyceryl Stearate, PEG-40 Glyceryl Stearate, PEG-120 Glyceryl Stearate, PEG-200 Glyceryl Stearate, PEG-28 Glyceryl Tallowate, PEG-80 Glyceryl Tallowate, PEG-82 Glyceryl Tallowate, PEG-130 Glyceryl Tallowate, PEG-200 Glyceryl Tallowate, PEG-45 Hydrogenated Castor Oil, PEG-50 Hydrogenated Castor Oil, PEG-54 Hydrogenated Castor Oil, PEG-55 Hydrogenated Castor Oil, PEG-60 Hydrogenated Castor Oil, PEG-80 Hydrogenated Castor Oil, PEG-100 Hydrogenated Castor Oil, PEG-200 Hydrogenated Castor Oil, PEG-30 Hydrogenated Lanolin, PEG-70 Hydrogenated Lanolin, PEG-50 Hydrogenated Palmamide, PEG-2 Isostearate, PEG-3 Isostearate, PEG-4 Isostearate, PEG-6 Isostearate, PEG-8 Isostearate, PEG-10 Isostearate, PEG-12 Isostearate, PEG-20 Isostearate, PEG-30 Isostearate, PEG-40 Isostearate, PEG- 26 Jojoba Acid, PEG-40 Jojoba Acid, PEG-15 Jojoba Alcohol, PEG-26 Jojoba Alcohol, PEG-40 Jojoba Alcohol, PEG-35 Lanolin, PEG-40 Lanolin, PEG-50 Lanolin, PEG-55 Lanolin, PEG-60 Lanolin, PEG- 70 Lanolin, PEG-75 Lanolin, PEG-85 Lanolin, PEG-100 Lanolin, PEG-150 Lanolin, PEG-75 Lanolin Oil, PEG-2 Lauramide, PEG-3 Lauramine Oxide, PEG-20 Laurate, PEG-32 Laurate, PEG-75 Laurate, PEG-150 Laurate, PEG-70 Mango Glycerides, PEG-20 Mannitan Laurate, PEG-8 Methyl Ether Dimethicone, PEG-120 Methyl Glucose Dioleate, PEG-80 Methyl Glucose Laurate, PEG-120 Methyl Glucose Trioleate, PEG-4 Montanate, PEG-30 Oleamine, PEG-20 Oleate, PEG-23 Oleate, PEG-32 Oleate, PEG-36 Oleate, PEG-75 Oleate, PEG-150 Oleate, PEG-20 Palmitate, PEG-150 Polyglyceryl-2 Tristearate, PEG/PPG-28/21 Acetate Dimethicone, PEG/PPG-24/18 Butyl Ether Dimethicone, PEG/PPG-3/17 Copolymer, PEG/PPG-5/35 Copolymer, PEG/PPG-8/55 Copolymer, PEG/PPG-10/30 Copolymer, PEG/PPG-10/65 Copolymer, PEG/PPG-12/35 Copolymer, PEG/PPG-16/17 Copolymer, PEG/PPG-20/9 Copolymer, PEG/PPG-20/20 Copolymer, PEG/PPG-20/60 Copolymer, PEG/PPG- 20/65 Copolymer, PEG/PPG-22/25 Copolymer, PEG/PPG-28/30 Copolymer, PEG/PPG-30-35 Copolymer, PEG/PPG-30/55 Copolymer, PEG/PPG-35/40 Copolymer, PEG/PPG-50/40 Copolymer, PEG/PPG-150/35 Copolymer, PEG/PPG-160/30 Copolymer, PEG/PPG-190/60 Copolymer, PEG/PPG-200/40 Copolymer, PEG/PPG-300/55 Copolymer, PEG/PPG-20/22 Methyl Ether Dimethicone, PEG-26-PPG-30 Phosphate, PEG/PPG-4/2 Propylheptyl Ether, PEG/PPG-6/2 Propylheptyl Ether, PEG-7/PPG-2 Propylheptyl Ether, PEG/PPG-8/2 Propylheptyl Ether, PEG/PPG- 10/2 Propylheptyl Ether, PEG/PPG-14/2 Propylheptyl Ether, PEG/PPG-40/2 Propylheptyl Ether, PEG/PPG-10/2 Ricinoleate, PEG/PPG-32/3 Ricinoleate, PEG-55 Propylene Glycol Oleate, PEG-25 Propylene Glycol Stearate, PEG-75 Propylene Glycol Stearate, PEG-120 Propylene Glycol Stearate, PEG-5 Rapeseed Sterol, PEG-10 Rapeseed Sterol, PEG-40 Ricinoleamide, PEG-75 Shea Butter Glycerides, PEG-75 Shorea Butter Glycerides, PEG-20 Sorbitan Cocoate, PEG-20 Sorbitan Isostearate, PEG-40 Sorbitan Lanolate, PEG-75 Sorbitan Lanolate, PEG-10 Sorbitan Laurate, PEG-40 Sorbitan Laurate, PEG-44 Sorbitan Laurate, PEG-75 Sorbitan Laurate, PEG-80 Sorbitan Laurate, PEG-20 Sorbitan Oleate, PEG-80 Sorbitan Palmitate, PEG-40 Sorbitan Stearate, PEG-60 Sorbitan Stearate, PEG-160 Sorbitan Triisostearate, PEG-40 Soy Sterol, PEG-2 Stearamide Carboxylic Acid, PEG-9 Stearamide Carboxylic Acid, PEG-20 Stearate, PEG-23 Stearate, PEG-25 Stearate, PEG-30 Stearate, PEG-32 Stearate, PEG-35 Stearate, PEG-36 Stearate, PEG-40 Stearate, PEG-45 Stearate, PEG-50 Stearate, PEG-55 Stearate, PEG-75 Stearate, PEG-90 Stearate, PEG-100 Stearate, PEG- 120 Stearate, PEG-150 Stearate, PEG-45 Stearate Phosphate, PEG-20 Tallate, PEG-50 Tallow Amide, PEG-2 Tallowamide DEA, PEG-20 Tallowate, PEG-66 Trihydroxystearin, PEG-200 Trihydroxystearin, PEG-60 Tsubakiate Glycerides, Pelargonic Acid, Pentadoxynol-200, Pheneth-6 Phosphate, Poloxamer 105, Poloxamer 108, Poloxamer 182, Poloxamer 183, Poloxamer 184, Poloxamer 188, Poloxamer 217, Poloxamer 234, Poloxamer 235, Poloxamer 237, Poloxamer 238, Poloxamer 288, Poloxamer 334, Poloxamer 335, Poloxamer 338, Poloxamine 908, Poloxamine 1508, Polydimethylsiloxy PEG/PPG-24/19 Butyl Ether Silsesquioxane, Polydimethylsiloxy PPG-13 Butyl Ether Silsesquioxane, Polyglyceryl-6 Caprate, Polyglyceryl-10 Dilaurate, Polyglyceryl-20 Heptacaprylate, Polyglyceryl-20 Hexacaprylate, Polyglyceryl-2 Lauryl Ether, Polyglyceryl-10 Lauryl Ether, Polyglyceryl-20 Octaisononanoate, Polyglyceryl-6 Pentacaprylate, Polyglyceryl-10 Pentacaprylate, Polyglyceryl-3 Polydimethylsiloxyethyl Dimethicone, Polyglyceryl-6 Tetracaprylate, Polyglyceryl-10 Tetralaurate, Polyglyceryl-6 Tricaprylate, Polyglyceryl-10 Trilaurate, Polyquaternium-77, Polyquaternium-78, Polyquaternium-79, Polyquaternium-80, Polyquaternium-81, Polyquaternium- 82, Pomaderris Kumerahou Flower/Leaf Extract, Poria Cocos Extract, Potassium Abietoyl Hydrolyzed Collagen, Potassium Babassuate, Potassium Behenate, Potassium C9-15 Alkyl Phosphate, Potassium C11-15 Alkyl Phosphate, Potassium C12-13 Alkyl Phosphate, Potassium C12-14 Alkyl Phosphate, Potassium Caprate, Potassium Capryloyl Glutamate, Potassium Capryloyl Hydrolyzed Rice Protein, Potassium Castorate, Potassium Cocoate, Potassium Cocoyl Glutamate, Potassium Cocoyl Glycinate, Potassium Cocoyl Hydrolyzed Casein, Potassium Cocoyl Hydrolyzed Collagen, Potassium Cocoyl Hydrolyzed Corn Protein, Potassium Cocoyl Hydrolyzed Keratin, Potassium Cocoyl Hydrolyzed Oat Protein, Potassium Cocoyl Hydrolyzed Potato Protein, Potassium Cocoyl Hydrolyzed Rice Bran Protein, Potassium Cocoyl Hydrolyzed Rice Protein, Potassium Cocoyl Hydrolyzed Silk, Potassium Cocoyl Hydrolyzed Soy Protein, Potassium Cocoyl Hydrolyzed Wheat Protein, Potassium Cocoyl Hydrolyzed Yeast Protein, Potassium Cocoyl PCA, Potassium Cocoyl Sarcosinate, Potassium Cocoyl Taurate, Potassium Cornate, Potassium Cyclocarboxypropyloleate, Potassium Dihydroxyethyl Cocamine Oxide Phosphate, Potassium Dimethicone PEG-7 Phosphate, Potassium Dodecylbenzenesulfonate, Potassium Hempseedate, Potassium Hydrogenated Cocoate, Potassium Hydrogenated Palmate, Potassium Hydrogenated Tallowate, Potassium Hydroxystearate, Potassium Isostearate, Potassium Lanolate, Potassium Laurate, Potassium Laureth-3 Carboxylate, Potassium Laureth-4 Carboxylate, Potassium Laureth-5 Carboxylate, Potassium Laureth-6 Carboxylate, Potassium Laureth-10 Carboxylate, Potassium Laureth Phosphate, Potassium Lauroyl Collagen Amino Acids, Potassium Lauroyl Glutamate, Potassium Lauroyl Hydrolyzed Collagen, Potassium Lauroyl Hydrolyzed Pea Protein, Potassium Lauroyl Hydrolyzed Soy Protein, Potassium Lauroyl PCA, Potassium Lauroyl Pea Amino Acids, Potassium Lauroyl Sarcosinate, Potassium Lauroyl Silk Amino Acids, Potassium Lauroyl Wheat Amino Acids, Potassium Lauryl Phosphate, Potassium Lauryl Sulfate, Potassium Linoleate, Potassium Metaphosphate, Potassium Methyl Cocoyl Taurate, Potassium Myristate, Potassium Myristoyl Glutamate, Potassium Myristoyl Hydrolyzed Collagen, Potassium Octoxynol-12 Phosphate, Potassium Oleate, Potassium Oleoyl Hydrolyzed Collagen, Potassium Olivate, Potassium Olivoyl Hydrolyzed Oat Protein, Potassium Olivoyl Hydrolyzed Wheat Protein, Potassium Olivoyl/Lauroyl Wheat Amino Acids, Potassium Olivoyl PCA, Potassium Palmate, Potassium Palmitate, Potassium Palmitoyl Hydrolyzed Corn Protein, Potassium Palmitoyl Hydrolyzed Oat Protein, Potassium Palmitoyl Hydrolyzed Rice Protein, Potassium Palmitoyl Hydrolyzed Sweet Almond Protein, Potassium Palmitoyl Hydrolyzed Wheat Protein, Potassium Palm Kernelate, Potassium Peanutate, Potassium Rapeseedate, Potassium Ricinoleate, Potassium Safflowerate, Potassium Soyate, Potassium Stearate, Potassium Stearoyl Hydrolyzed Collagen, Potassium Tallate, Potassium Tallowate, Potassium Taurate, Potassium Taurine Laurate, Potassium Trideceth-3 Carboxylate, Potassium Trideceth-4 Carboxylate, Potassium Trideceth-7 Carboxylate, Potassium Trideceth-15 Carboxylate, Potassium Trideceth-19 Carboxylate, Potassium Trideceth-6 Phosphate, Potassium Trideceth-7 Phosphate, Potassium Tsubakiate, Potassium Undecylenate, Potassium Undecylenoyl Hydrolyzed Collagen, Potassium Undecylenoyl Hydrolyzed Rice Protein, PPG-30- Buteth-30, PPG-36-Buteth-36, PPG-38-Buteth-37, PPG-30-Capryleth-4 Phosphate, PPG-10 Cetyl Ether Phosphate, PPG-2 C9-11 Pareth-8, PPG-1-Deceth-5, PPG-3-Deceth-2 Carboxylic Acid, PPG-30 Ethylhexeth-4 Phosphate, PPG-20-Glycereth-30, PPG-2 Hydroxyethyl Coco/Isostearamide, PPG-2- Isodeceth-8, PPG-2-Isodeceth-10, PPG-2-Isodeceth-18, PPG-2-Isodeceth-25, PPG-4-Isodeceth-10, Propyltrimonium Hydrolyzed Collagen, Quaternium-24, Quaternium-52, Quaternium-87, Rapeseed Acid, Rice Bran Acid, Rice Oil Glycereth-8 Esters, Ricinoleam idopropyl Betaine, Ricinoleic Acid, Ricinoleth-40, Safflower Acid, Sapindus Oahuensis Fruit Extract, Saponaria Officinalis Root Powder, Saponins, Sekken-K, Sekken-Na/K, Sekken Soji, Sekken Soji-K, Sesame Oil Glycereth-8 Esters, Sesamidopropylamine Oxide, Sesamidopropyl Betaine, Shea Butteramidopropyl Betaine, Shea Butter Glycereth-8 Esters, Sodium Arachidate, Sodium Arganampohoacetate, Sodium Astrocaryum Murumuruate, Sodium Avocadoate, Sodium Babassuamphoacetate, Sodium Babassuate, Sodium Babassu Sulfate, Sodium Behenate, Sodium Bisglycol Ricinosulfosuccinate, Sodium Bis-Hydroxyethylglycinate Coco-Glucosides Crosspolymer, Sodium Bis-Hydroxyethylglycinate Lauryl- Glucosides Crosspolymer, Sodium Borageamidopropyl PG-Dimonium Chloride Phosphate, Sodium Butoxynol-12 Sulfate, Sodium Butylglucosides Hydroxypropyl Phosphate, Sodium C13-17 Alkane Sulfonate, Sodium C14-18 Alkane Sulfonate, Sodium C12-15 Alkoxypropyl Iminodipropionate, Sodium C10-16 Alkyl Sulfate, Sodium C11-15 Alkyl Sulfate, Sodium C12-13 Alkyl Sulfate, Sodium C12-15 Alkyl Sulfate, Sodium C12-18 Alkyl Sulfate, Sodium C16-20 Alkyl Sulfate, Sodium C9-22 Alkyl Sec Sulfonate, Sodium C14-17 Alkyl Sec Sulfonate, Sodium Caprate, Sodium Caproamphoacetate, Sodium Caproamphohydroxypropylsulfonate, Sodium Caproamphopropionate, Sodium Caproyl Methyltaurate, Sodium Caprylate, Sodium Capryleth-2 Carboxylate, Sodium Capryleth-9 Carboxylate, Sodium Capryloamphoacetate, Sodium Capryloamphohydroxypropylsulfonate, Sodium Capryloamphopropionate, Sodium Capryloyl Glutamate, Sodium Capryloyl Hydrolyzed Wheat Protein, Sodium Caprylyl PG-Sulfonate, Sodium Caprylyl Sulfonate, Sodium Castorate, Sodium Ceteareth-13 Carboxylate, Sodium Cetearyl Sulfate, Sodium Ceteth-13 Carboxylate, Sodium Cetyl Sulfate, Sodium Cocamidopropyl PG-Dimonium Chloride Phosphate, Sodium Cocaminopropionate, Sodium Coceth Sulfate, Sodium Coceth-30 Sulfate, Sodium Cocoabutteramphoacetate, Sodium Cocoa Butterate, Sodium Cocoamphoacetate, Sodium Cocoamphohydroxypropylsulfonate, Sodium Cocoamphopropionate, Sodium Cocoate, Sodium Coco/Babassu/Andiroba Sulfate, Sodium Coco/Babassu Sulfate, Sodium Cocoglucosides Hydroxypropyl Phosphate, Sodium Cocoglucosides Hydroxypropylsulfonate, Sodium Coco-Glucoside Tartrate, Sodium Cocoglyceryl Ether Sulfonate, Sodium Coco/Hydrogenated Tallow Sulfate, Sodium Cocoiminodiacetate, Sodium Cocomonoglyceride Sulfate, Sodium Cocomonoglyceride Sulfonate, Sodium Coco PG-Dimonium Chloride Phosphate, Sodium Coco-Sulfate, Sodium Coco Sulfoacetate, Sodium Cocoyl Alaninate, Sodium Cocoyl Amino Acids, Sodium Cocoyl Collagen Amino Acids, Sodium Cocoyl Glutamate, Sodium Cocoyl Glutaminate, Sodium Cocoyl Glycinate, Sodium Cocoyl/Hydrogenated Tallow Glutamate, Sodium Cocoyl Hydrolyzed Collagen, Sodium Cocoyl Hydrolyzed Keratin, Sodium Cocoyl Hydrolyzed Rice Protein, Sodium Cocoyl Hydrolyzed Silk, Sodium Cocoyl Hydrolyzed Soy Protein, Sodium Cocoyl Hydrolyzed Sweet Almond Protein, Sodium Cocoyl Hydrolyzed Wheat Protein, Sodium Cocoyl Hydrolyzed Wheat Protein Glutamate, Sodium Cocoyl Isethionate, Sodium Cocoyl Methylaminopropionate, Sodium Cocoyl Oat Amino Acids, Sodium Cocoyl/Palmoyl/Sunfloweroyl Glutamate, Sodium Cocoyl Proline, Sodium Cocoyl Sarcosinate, Sodium Cocoyl Taurate, Sodium Cocoyl Threoninate, Sodium Cocoyl Wheat Amino Acids, Sodium C12-14 Olefin Sulfonate, Sodium C14-16 Olefin Sulfonate, Sodium C14- 18 Olefin Sulfonate, Sodium C16-18 Olefin Sulfonate, Sodium Cornamphopropionate, Sodium Cottonseedamphoacetate, Sodium C13-15 Pareth-8 Butyl Phosphate, Sodium C9-11 Pareth-6 Carboxylate, Sodium C11-15 Pareth-7 Carboxylate, Sodium C12-13 Pareth-5 Carboxylate, Sodium C12-13 Pareth-8 Carboxylate, Sodium C12-13 Pareth-12 Carboxylate, Sodium C12-15 Pareth-6 Carboxylate, Sodium C12-15 Pareth-7 Carboxylate, Sodium C12-15 Pareth-8 Carboxylate, Sodium C14-15 Pareth-8 Carboxylate, Sodium C12-14 Sec-Pareth-8 Carboxylate, Sodium C14-15 Pareth-PG Sulfonate, Sodium C12-13 Pareth-2 Phosphate, Sodium C13-15 Pareth-8 Phosphate, Sodium C9-15 Pareth-3 Sulfate, Sodium C10-15 Pareth Sulfate, Sodium C10-16 Pareth-2 Sulfate, Sodium C12-13 Pareth Sulfate, Sodium C12-15 Pareth Sulfate, Sodium C12-15 Pareth-3 Sulfate, Sodium C13-15 Pareth-3 Sulfate, Sodium C12-14 Sec-Pareth-3 Sulfate, Sodium C12-15 Pareth-3 Sulfonate, Sodium C12-15 Pareth-7 Sulfonate, Sodium C12-15 Pareth-15 Sulfonate, Sodium Deceth-2 Carboxylate, Sodium Deceth Sulfate, Sodium Decylbenzenesulfonate, Sodium Decylglucosides Hydroxypropyl Phosphate, Sodium Decylglucosides Hydroxypropylsulfonate, Sodium Dilaureth-7 Citrate, Sodium Dilaureth-10 Phosphate, Sodium Dilinoleamidopropyl PG-Dimonium Chloride Phosphate, Sodium Dilinoleate, Sodium Dioleth-8 Phosphate, Sodium Dodecylbenzenesulfonate, Sodium Ethyl 2- Sulfolaurate, Sodium Glyceryl Oleate Phosphate, Sodium Grapeseedamidopropyl PG-Dimonium Chloride Phosphate, Sodium Grapeseedamphoacetate, Sodium Grapeseedate, Sodium Hempseedamphoacetate, Sodium Hexeth-4 Carboxylate, Sodium Hydrogenated Cocoate, Sodium Hydrogenated Cocoyl Methyl Isethionate, Sodium Hydrogenated Palmate, Sodium Hydrogenated Tallowate, Sodium Hydrogenated Tallowoyl Glutamate, Sodium Hydroxylauryldimonium Ethyl Phosphate, Sodium Hydroxypropyl Palm Kernelate Sulfonate, Sodium Hydroxypropylphosphate Decylglucoside Crosspolymer, Sodium Hydroxypropylphosphate Laurylglucoside Crosspolymer, Sodium Hydroxypropylsulfonate Cocoglucoside Crosspolymer, Sodium Hydroxypropylsulfonate Decylglucoside Crosspolymer, Sodium Hydroxypropylsulfonate Laurylglucoside Crosspolymer, Sodium Hydroxystearate, Sodium Isostearate, Sodium Isosteareth-6 Carboxylate, Sodium Isosteareth- 11 Carboxylate, Sodium Isostearoamphoacetate, Sodium Isostearoamphopropionate, Sodium N-Isostearoyl Methyltaurate, Sodium Laneth Sulfate, Sodium Lanolate, Sodium Lardate, Sodium Lauramido Diacetate, Sodium Lauraminopropionate, Sodium Laurate, Sodium Laureth-3 Carboxylate, Sodium Laureth-4 Carboxylate, Sodium Laureth-5 Carboxylate, Sodium Laureth-6 Carboxylate, Sodium Laureth-8 Carboxylate, Sodium Laureth-11 Carboxylate, Sodium Laureth-12 Carboxylate, Sodium Laureth-13 Carboxylate, Sodium Laureth-14 Carboxylate, Sodium Laureth-16 Carboxylate, Sodium Laureth-17 Carboxylate, Sodium Laureth Sulfate, Sodium Laureth-5 Sulfate, Sodium Laureth-7 Sulfate, Sodium Laureth-8 Sulfate, Sodium Laureth-12 Sulfate, Sodium Laureth-40 Sulfate, Sodium Laureth-7 Tartrate, Sodium Lauriminodipropionate, Sodium Lauroamphoacetate, Sodium Lauroamphohydroxypropylsulfonate, Sodium Lauroampho PG-Acetate Phosphate, Sodium Lauroamphopropionate, Sodium Lauroyl Aspartate, Sodium Lauroyl Collagen Amino Acids, Sodium Lauroyl Glycine Propionate, Sodium Lauroyl Hydrolyzed Collagen, Sodium Lauroyl Hydrolyzed Silk, Sodium Lauroyl Hydroxypropyl Sulfonate, Sodium Lauroyl Isethionate, Sodium Lauroyl Methylaminopropionate, Sodium Lauroyl Methyl Isethionate, Sodium Lauroyl Millet Amino Acids, Sodium Lauroyl/Myristoyl Aspartate, Sodium Lauroyl Oat Amino Acids, Sodium Lauroyl Sarcosinate, Sodium Lauroyl Silk Amino Acids, Sodium Lauroyl Taurate, Sodium Lauroyl Wheat Amino Acids, Sodium Lauryl Diethylenediaminoglycinate, Sodium Lauryl Glucose Carboxylate, Sodium Laurylglucosides Hydroxypropyl Phosphate, Sodium Laurylglucosides Hydroxypropylsulfonate, Sodium Lauryl Glycol Carboxylate, Sodium Lauryl Hydroxyacetamide Sulfate, Sodium Lauryl Phosphate, Sodium Lauryl Sulfate, Sodium Lauryl Sulfoacetate, Sodium Linoleate, Sodium Macadamiaseedate, Sodium Mangoamphoacetate, Sodium Mangoseedate, Sodium/MEA Laureth-2 Sulfosuccinate, Sodium Methoxy PPG-2 Acetate, Sodium Methyl Cocoyl Taurate, Sodium Methyl Lauroyl Taurate, Sodium Methyl Myristoyl Taurate, Sodium Methyl Oleoyl Taurate, Sodium Methyl Palmitoyl Taurate, Sodium Methyl Stearoyl Taurate, Sodium Methyl 2-Sulfolaurate, Sodium Methyl 2- Sulfopalmitate, Sodium Methyltaurate Isopalmitamide, Sodium Methyltaurine Cocoyl Methyltaurate, Sodium Myreth Sulfate, Sodium Myristate, Sodium Myristoamphoacetate, Sodium Myristoyl Glutamate, Sodium Myristoyl Hydrolyzed Collagen, Sodium Myristoyl Isethionate, Sodium Myristoyl Sarcosinate, Sodium Myristyl Sulfate, Sodium Nonoxynol-6 Phosphate, Sodium Nonoxynol-9 Phosphate, Sodium Nonoxynol-1 Sulfate, Sodium Nonoxynol-3 Sulfate, Sodium Nonoxynol-4 Sulfate, Sodium Nonoxynol-6 Sulfate, Sodium Nonoxynol-8 Sulfate, Sodium Nonoxynol-10 Sulfate, Sodium Nonoxynol-25 Sulfate, Sodium Octoxynol-2 Ethane Sulfonate, Sodium Octoxynol-2 Sulfate, Sodium Octoxynol-6 Sulfate, Sodium Octoxynol-9 Sulfate, Sodium Oleate, Sodium Oleoamphoacetate, Sodium Oleoamphohydroxypropylsulfonate, Sodium Oleoamphopropionate, Sodium Oleoyl Hydrolyzed Collagen, Sodium Oleoyl Isethionate, Sodium Oleth Sulfate, Sodium Oleyl Methyl Isethionate, Sodium Oleyl Sulfate, Sodium Olivamphoacetate, Sodium Olivate, Sodium Olivoyl Glutamate, Sodium Palmamphoacetate, Sodium Palmate, Sodium Palm Glyceride Sulfonate, Sodium Palmitate, Sodium Palmitoyl Hydrolyzed Collagen, Sodium Palmitoyl Hydrolyzed Wheat Protein, Sodium Palmitoyl Sarcosinate, Sodium Palm Kernelate, Sodium Palm Kerneloyl Isethionate, Sodium Palmoyl Glutamate, Sodium Passiflora Edulis Seedate, Sodium Peanutamphoacetate, Sodium Peanutate, Sodium PEG-6 Cocamide Carboxylate, Sodium PEG-8 Cocamide Carboxylate, Sodium PEG-4 Cocamide Sulfate, Sodium PEG-3 Lauramide Carboxylate, Sodium PEG-4 Lauramide Carboxylate, Sodium PEG-8 Palm Glycerides Carboxylate, Sodium Pentaerythrityl Hydroxypropyl Iminodiacetate Dendrimer, Sodium Propoxy PPG-2 Acetate, Sodium Rapeseedate, Sodium Ricebranamphoacetate, Sodium Ricinoleate, Sodium Ricinoleoamphoacetate, Sodium Rose Hipsamphoacetate, Sodium Rosinate, Sodium Safflowerate, Sodium Saffloweroyl Hydrolyzed Soy Protein, Sodium Sesameseedate, Sodium Sesamphoacetate, Sodium Sheabutteramphoacetate, Sodium Soyate, Sodium Soy Hydrolyzed Collagen, Sodium Stearate, Sodium Stearoamphoacetate, Sodium Stearoamphohydroxypropylsulfonate, Sodium Stearoamphopropionate, Sodium Stearoyl Casein, Sodium Stearoyl Glutamate, Sodium Stearoyl Hyaluronate, Sodium Stearoyl Hydrolyzed Collagen, Sodium Stearoyl Hydrolyzed Corn Protein, Sodium Stearoyl Hydrolyzed Silk, Sodium Stearoyl Hydrolyzed Soy Protein, Sodium Stearoyl Hydrolyzed Wheat Protein, Sodium Stearoyl Lactalbumin, Sodium Stearoyl Methyl Isethionate, Sodium Stearoyl Oat Protein, Sodium Stearoyl Pea Protein, Sodium Stearoyl Soy Protein, Sodium Stearyl Dimethyl Glycine, Sodium Stearyl Sulfate, Sodium Sunflowerseedamphoacetate, Sodium Surfactin, Sodium Sweetalmondamphoacetate, Sodium Sweet Almondate, Sodium Tallamphopropionate, Sodium Tallate, Sodium Tallowamphoacetate, Sodium Tallowate, Sodium Tallow Sulfate, Sodium Tamanuseedate, Sodium Taurate, Sodium Taurine Cocoyl Methyltaurate, Sodium Taurine Laurate, Sodium/TEA-Lauroyl Collagen Amino Acids, Sodium/TEA-Lauroyl Hydrolyzed Collagen, Sodium/TEA-Lauroyl Hydrolyzed Keratin, Sodium/TEA- Lauroyl Keratin Amino Acids, Sodium/TEA-Undecylenoyl Collagen Amino Acids, Sodium/TEA- Undecylenoyl Hydrolyzed Collagen, Sodium/TEA-Undecylenoyl Hydrolyzed Corn Protein, Sodium/TEA-Undecylenoyl Hydrolyzed Soy Protein, Sodium/TEA-Undecylenoyl Hydrolyzed Wheat Protein, Sodium Theobroma Grandiflorum Seedate, Sodium Trideceth-3 Carboxylate, Sodium Trideceth-4 Carboxylate, Sodium Trideceth-6 Carboxylate, Sodium Trideceth-7 Carboxylate, Sodium Trideceth-8 Carboxylate, Sodium Trideceth-12 Carboxylate, Sodium Trideceth-15 Carboxylate, Sodium Trideceth-19 Carboxylate, Sodium Trideceth Sulfate, Sodium Tridecylbenzenesulfonate, Sodium Tridecyl Sulfate, Sodium Trimethylolpropane Hydroxypropyl Iminodiacetate Dendrimer, Sodium Undeceth-5 Carboxylate, Sodium Undecylenate, Sodium Undecylenoamphoacetate, Sodium Undecylenoamphopropionate, Sodium Undecylenoyl Glutamate, Sodium Wheat Germamphoacetate, Sorbeth-160 Tristearate, Soy Acid, Soyamidopropylamine Oxide, Soyamidopropyl Betaine, Soybean Oil Glycereth-8 Esters, Stearamidopropylamine Oxide, Stearamidopropyl Betaine, Stearamine Oxide, Steareth-15, Steareth-16, Steareth-20, Steareth-21, Steareth-25, Steareth-27, Steareth-30, Steareth- 40, Steareth-50, Steareth-80, Steareth-100, Steareth-2 Phosphate, Steareth-3 Phosphate, Stearic Acid, Stearoxypropyltrimonium Chloride, Stearoyl Glutamic Acid, Stearoyl Sarcosine, Stearyl Betaine, Stearyldimoniumhydroxypropyl Butylglucosides Chloride, Stearyldimoniumhydroxypropyl Decylglucosides Chloride, Stearyldimoniumhydroxypropyl Laurylglucosides Chloride, Sulfated Castor Oil, Sulfated Coconut Oil, Sulfated Glyceryl Oleate, Sulfated Olive Oil, Sulfated Peanut Oil, Sunfloweramide MEA, Sunflower Seed Acid, Sunflowerseedamidopropyl Hydroxyethyldimonium Chloride, Sunflower Seed Oil Glycereth-8 Esters, Tall Oil Acid, Tallow Acid, Tallowamidopropylamine Oxide, Tallowamidopropyl Betaine, Tallowamidopropyl Hydroxysultaine, Tallowamine Oxide, Tallow Betaine, Tallow Dihydroxyethyl Betaine, Tallowoyl Ethyl Glucoside, TEA-Abietoyl Hydrolyzed Collagen, TEA-C12-14 Alkyl Phosphate, TEA-C10-15 Alkyl Sulfate, TEA-C11-15 Alkyl Sulfate, TEA- C12-13 Alkyl Sulfate, TEA-C12-14 Alkyl Sulfate, TEA-C12-15 Alkyl Sulfate, TEA C14-17 Alkyl Sec Sulfonate, TEA-Canolate, TEA-Cocamide Diacetate, TEA-Cocoate, TEA-Coco-Sulfate, TEA-Cocoyl Alaninate, TEA-Cocoyl Glutamate, TEA-Cocoyl Glutaminate, TEA-Cocoyl Glycinate, TEA-Cocoyl Hydrolyzed Collagen, TEA-Cocoyl Hydrolyzed Soy Protein, TEA-Cocoyl Sarcosinate, TEA- Dimethicone PEG-7 Phosphate, TEA-Dodecylbenzenesulfonate, TEA-Hydrogenated Cocoate, TEA- Hydrogenated Tallowoyl Glutamate, TEA-Isostearate, TEA-Isostearoyl Hydrolyzed Collagen, TEA- Lauraminopropionate, TEA-Laurate, TEA-Laurate/Myristate, TEA-Laureth Sulfate, TEA-Lauroyl Collagen Amino Acids, TEA-Lauroyl Glutamate, TEA-Lauroyl Hydrolyzed Collagen, TEA-Lauroyl Keratin Amino Acids, TEA-Lauroyl Methylaminopropionate, TEA-Lauroyl/Myristoyl Aspartate, TEA-Lauroyl Sarcosinate, TEA-Lauryl Phosphate, TEA-Lauryl Sulfate, TEA-Myristaminopropionate, TEA- Myristate, TEA-Myristoyl Hydrolyzed Collagen, TEA-Oleate, TEA-Oleoyl Hydrolyzed Collagen, TEA- Oleoyl Sarcosinate, TEA-Oleyl Sulfate, TEA-Palmitate, TEA-Palm Kernel Sarcosinate, TEA-PEG-3 Cocamide Sulfate, TEA-Rosinate, TEA-Stearate, TEA-Tallate, TEA-T ridecylbenzenesulfonate, TEA- Undecylenate, TEA-Undecylenoyl Hydrolyzed Collagen, Tetramethyl Decynediol, Tetrasodium Dicarboxyethyl Stearyl Sulfosuccinamate, TIPA-Laureth Sulfate, TIPA-Lauryl Sulfate, TIPA-Myristate, TIPA-Stearate, Tocopheryl Phosphate, Trehalose Undecylenoate, TM-C12-15 Pareth-2 Phosphate, TM-C12-15 Pareth-6 Phosphate, TM-C12-15 Pareth-8 Phosphate, TM-C12-15 Pareth-10 Phosphate, Trideceth-20, Trideceth-50, Trideceth-3 Carboxylic Acid, Trideceth-4 Carboxylic Acid, Trideceth-7 Carboxylic Acid, Trideceth-8 Carboxylic Acid, Trideceth-15 Carboxylic Acid, Trideceth-19 Carboxylic Acid, Trideceth-10 Phosphate, Tridecylbenzenesulfonic Acid, Trilaureth-9 Citrate, Trimethylolpropane Hydroxypropyl Bis-Hydroxyethylamine Dendrimer, Trisodium Lauroampho PG-Acetate Chloride Phosphate, Undecanoic Acid, Undeceth-5 Carboxylic Acid, Undecylenamidopropylamine Oxide, Undecylenamidopropyl Betaine, Undecylenic Acid, Undecylenoyl Collagen Amino Acids, Undecylenoyl Glycine, Undecylenoyl Hydrolyzed Collagen, Undecylenoyl Wheat Amino Acids, Undecyl Glucoside, Wheat Germ Acid, Wheat Germamidopropylamine Oxide, Wheat Germamidopropyl Betaine, Yucca Schidigera Leaf/Root/Stem Extract, Yucca Schidigera Stem Extract, Zinc Coceth Sulfatea and Zinc Coco-Sulfate.

**Preservatives:** For preservative purposes, the cosmetic or pharmaceutical composition according to the present invention preferably includes one or more preservatives which are suitable or customary in cosmetic or pharmaceutical composition. Suitable and advantageously preservatives are, for example, phenoxyethanol, formaldehyde solution, parabens, pentanediol or sorbic acid.

**Synthetic polymers:** Apart from the above-mentioned natural polymers, the cosmetic or pharmaceutical composition as defined herein, is advantageously combined with at least one further synthetic polymer. Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lame-quat^{®} L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®}A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300. Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones. In a preferred variant, the cosmetic or pharmaceutical composition according to the present invention preferably includes an uncrosslinked or polyol-crosslinked polyacrylic acid as additionally polymer component.

**Perfume oils and/or fragrances:** The cosmetic or pharmaceutical composition according to the present invention preferably includes one or more perfume oils and/or fragrances. Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, beta-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

**Anti-cellulite agent:** The cosmetic or pharmaceutical composition according to the present invention preferably includes one or more anti-cellulite agents. Anti-cellulite agents and lipolytic agents are preferably selected from the group consisting of beta-adrenergic receptor agonists such as synephrine and its derivatives, and cyclohexyl carbamates. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

**Fat enhancing agents:** The cosmetic or pharmaceutical composition according to the present invention preferably includes one or more fat enhancing agents and/or adipogenic agents as well as agents enhancing or boosting the activity of fat enhancing agents. A fat enhancing agent is for example hydroxymethoxyphenyl propylmethylmethoxybenzofuran (trade name: Sym3D^{®}).

**Superfatting agents and/or consistency factors:** The cosmetic or pharmaceutical composition according to the present invention preferably includes one or more superfatting agents and/or consistency factors. Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers. The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

**Pearlizing waxes:** The cosmetic or pharmaceutical composition according to the present invention preferably includes one or more pearlizing waxes. Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups, and mixtures thereof.

**Silicones:** In order to impart a silky, spreadable, and luxurious texture and to make skin look and feel smoother, and additionally to improve processability (antifoaming) the cosmetic or pharmaceutical composition according to the present invention preferably includes one or more silicones or silicone deratives. Suitable silicones can be chosen from the group consisting of Acefylline Methylsilanol Mannuronate, Acetylmethionyl Methylsilanol Elastinate Acrylates/Behenyl, Acrylate/Dimethicone Methacrylate Copolymer, Acrylates/Behenyl Methacrylate/Dimethicone Methacrylate Copolymer, Acrylates/Bis-Hydroxypropyl Dimethicone Crosspolymer, Acrylates/Dimethicone Copolymer, Acrylates/Dimethicone Methacrylate/Ethylhexyl Acrylate Copolymer, Acrylates/Dimethiconol Acrylate Copolymer, Acrylates/Ethylhexyl Acrylate/Dimethicone Methacrylate Copolymer, Acrylates/Octylacrylamide/Diphenyl Amodimethicone Copolymer, Acrylates/Polytrimethylsiloxymethacrylate Copolymer, Acrylates/Propyl Trimethicone Methacrylate Copolymer, Acrylates/Stearyl Acrylate/Dimethicone Methacrylate Copolymer, Acrylates/Tridecyl Acrylate/Triethoxysilylpropyl Methacrylate/Dimethicone Methacrylate Copolymer, Acrylates/Trifluoropropylmethacrylate/Polytrimethyl Siloxymethacrylate Copolymer, Amino Bispropyl Dimethicone, Aminoethylaminopropyl Dimethicone, Aminopropyl Dimethicone, Aminopropyl Phenyl Trimethicone, Aminopropyl Triethoxysilane, Ammonium Dimethicone PEG-7 Sulfate, Amodimethicone, Amodimethicone Hydroxystearate, Amodimethicone/Silsesquioxane Copolymer, Ascorbyl Carboxydecyl Trisiloxane, Ascorbyl Methylsilanol Pectinate, Behenoxy Dimethicone, Behentrimonium Dimethicone PEG-8 Phthalate, Behenyl Dimethicone, Bisamino PEG/PPG-41/3 Aminoethyl PG-Propyl Dimethicone, Bis-Aminopropyl/Ethoxy Aminopropyl Dimethicone, Bis(Butylbenzoate) Diaminotriazine Aminopropyltrisiloxane, Bis-Butyldimethicone Polyglyceryl-3, Bis-Butyloxyamodimethicone/PEG-60 Copolymer, Bis(C13-15 Alkoxy) Hydroxybutamidoamodimethicone, Bis(C13-15 Alkoxy) PG- Amodimethicone, Bis-(C1-8 Alkyl Lauroyl Lysine Decylcarboxamide) Dimethicone, Bis-Cetyl Cetyl Dimethicone, Bis-Cetyl/PEG-8 Cetyl PEG-8 Dimethicone, Bis-Diphenylethyl Disiloxane, Bis-Ethyl Ethyl Methicone, Bis-Gluconamidoethylaminopropyl Dimethicone, Bis-Hydrogen Dimethicone, Bis- Hydroxyethoxypropyl Dimethicone Bis-Hydroxylauryl, Dimethicone/IPDI Copolymer, Bis- Hydroxy/Methoxy Amodimethicone, Bis-Hydroxypropyl Dimethicone Behenate, Bis-Hydroxypropyl Dimethicone/SMDI Copolymer, Bis-Isobutyl PEG-14/Amodimethicone Copolymer, Bis-Isobutyl PEG-15/Amodimethicone Copolymer, Bis-Isobutyl PEG/PPG-20/35/Amodimethicone Copolymer, Bis- Isobutyl PEG/PPG-10/7/Dimethicone Copolymer, Bis-Isobutyl PEG-24/PPG-7/Dimethicone Copolymer, Bis-PEG-1 Dimethicone, Bis-PEG-4 Dimethicone, Bis-PEG-8 Dimethicone, Bis-PEG-12 Dimethicone,Bis-PEG-20 Dimethicone, Bis-PEG-12 Dimethicone Beeswax, Bis-PEG-12 Dimethicone Candelillate, Bis-PEG-15 Dimethicone/IPDI Copolymer, Bis-PEG-15 Methyl Ether Dimethicone, Bis- PEG-18 Methyl Ether Dimethyl Silane, Bis-PEG/PPG-14/14 Dimethicone, Bis-PEG/PPG-15/5 Dimethicone, Bis-PEG/PPG-18/6 Dimethicone, Bis-PEG/PPG-20/20 Dimethicone, Bis-PEG/PPG- 16/16 PEG/PPG-16/16 Dimethicone, Bis-PEG/PPG-20/5 PEG/PPG-20/5 Dimethicone, Bisphenylhexamethicone, Bis-Phenylpropyl Dimethicone, Bispolyethylene Dimethicone, Bis- (Polyglyceryl-3 Oxyphenylpropyl) Dimethicone, Bis-(Polyglyceryl-7 Oxyphenylpropyl) Dimethicone, Bis-PPG-15 Dimethicone/IPDI Copolymer, Bis(PPG-7 Undeceneth-21) Dimethicone, Bis-Stearyl Dimethicone, Bis-Trimethoxysilylethyl Tetramethyldisiloxyethyl Dimethicone, Bis-Vinyldimethicone, Bis-Vinyl Dimethicone/Dimethicone Copolymer, Borage Seed Oil PEG-7 Dimethicone Esters, Butyl Acrylate/C6-14 Perfluoroalkylethyl Acrylate/Mercaptopropyl Dimethicone Copolymer, Butyl Acrylate/Hydroxypropyl Dimethicone Acrylate Copolymer, Butyl Dimethicone Acrylate/Cyclohexylmethacrylate/Ethylhexyl Acrylate Copolymer, Butyldimethicone Methacrylate/Methyl Methacrylate Crosspolymer, t-Butyl Dimethyl Silyl Grape Seed Extract, Butyl Polydimethylsiloxyl Ethylene/Propylene/Vinylnorbornene Copolymer, C6-8 Alkyl C3-6 Alkyl Glucoside Dimethicone, C20-24 Alkyl Dimethicone,C24-28 Alkyl Dimethicone, C26-28 Alkyl Dimethicone, C30-45 Alkyl Dimethicone, C30-60 Alkyl Dimethicone, C32 Alkyl Dimethicone, C30-45 Alkyl Dimethicone/Polycyclohexene Oxide Crosspolymer, C26-28 Alkyldimethylsilyl Polypropylsilsesquioxane, C30-45 Alkyldimethylsilyl Polypropylsilsesquioxane, C20-24 Alkyl Methicone, C24-28 Alkyl Methicone, C26-28 Alkyl Methicone, C30-45 Alkyl Methicone, C20-28 Alkyl Perfluorodecylethoxy Dimethicone, C26-54 Alkyl Tetradecyl Dimethicone, Capryl Dimethicone, Caprylyl Dimethicone Ethoxy Glucoside, Caprylyl Methicone, Caprylyl Trimethicone, Carboxydecyl Trisiloxane, Castor Oil Bis-Hydroxypropyl Dimethicone Esters Cerotyl Dimethicone, Cetearyl Dimethicone Crosspolymer, Cetearyl Dimethicone/Vinyl Dimethicone Crosspolymer, Cetearyl Methicone, Cetrimonium Carboxydecyl PEG-8 Dimethicone, Cetrimonium Dimethicone PEG-7 Phthalate, Cetyl Behenyl Dimethicone, Cetyl Dimethicone, Cetyl Dimethicone/Bis-Vinyldimethicone Crosspolymer, Cetyl Hexacosyl Dimethicone, Cetyloxy Dimethicone, Cetyl PEG-8 Dimethicone, Cetyl PEG/PPG-15/15 Butyl Ether Dimethicone, Cetyl PEG/PPG-7/3 Dimethicone, Cetyl PEG/PPG-10/1 Dimethicone, Cetyl Triethylmonium Dimethicone PEG-8 Phthalate, Cetyl Triethylmonium Dimethicone PEG-8 Succinate, Copper Acetyl Tyrosinate Methylsilanol, Copper PCA Methylsilanol, C4-14 Perfluoroalkylethoxy Dimethicone, Cycloethoxymethicone, Cycloheptasiloxane, Cyclohexasiloxane, Cyclomethicone, Cyclopentasiloxane, Cyclophenylmethicone, Cyclotetrasiloxane,mCyclovinylmethicone, Cystine Bis-PG-Propyl Silanetriol, DEA PG-Propyl PEG/PPG-18/21 Dimethicone, Diisostearoyl Trimethylolpropane Siloxy Silicate, Dilauroyl Trimethylolpropane Siloxy Silicate, Dilinoleamidopropyl Dimethylamine Dimethicone PEG-7 Phosphate, Dimethicone, Dimethicone Crosspolymer, Dimethicone Crosspolymer-3, Dimethicone/Divinyldimethicone/Silsesquioxane Crosspolymer, Dimethicone Ethoxy Glucoside, Dimethicone Hydroxypropyl Trimonium Chloride, Dimethicone/Mercaptopropyl Methicone Copolymer, Dimethicone PEG-15 Acetate Dimethicone PEG-8 Adipate, Dimethicone PEG-7 Avocadoate, Dimethicone PEG-8 Avocadoate, Dimethicone PEG-8 Beeswax, Dimethicone PEG-8 Benzoate, Dimethicone PEG-8 Borageate, Dimethicone PEG-7 Cocoate, Dimethicone/PEG-10 Crosspolymer, Dimethicone/PEG-10/15 Crosspolymer, Dimethicone/PEG-15 Crosspolymer, Dimethicone PEG-7 Isostearate, Dimethicone PEG-8 Isostearate, Dimethicone PEG-7 Lactate, Dimethicone PEG-8 Lanolate, Dimethicone PEG-8 Laurate, Dimethicone PEG-8 Meadowfoamate, Dimethicone PEG-7 Octyldodecyl Citrate, Dimethicone PEG-7 Olivate, Dimethicone PEG-8 Olivate, Dimethicone PEG-7 Phosphate, Dimethicone PEG-8 Phosphate, Dimethicone PEG-10 Phosphate, Dimethicone PEG-7 Phthalate, Dimethicone PEG-8 Phthalate, Dimethicone PEG-8 Polyacrylate, Dimethicone PEG/PPG- 20/23 Benzoate, Dimethicone PEG/PPG-7/4 Phosphate, Dimethicone PEG/PPG-12/4 Phosphate, Dimethicone PEG-7 Succinate, Dimethicone PEG-8 Succinate, Dimethicone PEG-7 Sulfate, Dimethicone PEG-7 Undecylenate, Dimethicone PG-Diethylmonium Chloride, Dimethicone/Phenyl Vinyl Dimethicone Crosspolymer, Dimethicone/Polyglycerin-3 Crosspolymer, Dimethicone/PPG-20 Crosspolymer, Dimethicone Propylethylenediamine Behenate, Dimethicone Propyl PG-Betaine, Dimethicone/Silsesquioxane Copolymer, Dimethicone Silylate, Dimethicone Dimethicone Crosspolymer, Dimethicone/Vinyltrimethylsiloxysilicate Crosspolymer, Dimethiconol, Dimethiconol Arginine, Dimethiconol Beeswax, Dimethiconol Behenate, Dimethiconol Borageate, Dimethiconol Candelillate, Dimethiconol Carnaubate, Dimethiconol Cysteine, Dimethiconol Dhupa Butterate, Dimethiconol Fluoroalcohol Dilinoleic Acid, Dimethiconol Hydroxystearate, Dimethiconol Illipe Butterate, Dimethiconol/IPDI Copolymer, Dimethiconol Isostearate, Dimethiconol Kokum Butterate, Dimethiconol Lactate, Dimethiconol Meadowfoamate, Dimethiconol Methionine, Dimethiconol/Methylsilanol/Silicate Crosspolymer, Dimethiconol Mohwa Butterate, Dimethiconol Panthenol, Dimethiconol Sal Butterate, Dimethiconol/Silica Crosspolymer, Dimethiconol/Silsesquioxane Copolymer, Dimethiconol Stearate, Dimethiconol/Stearyl, Methicone/Phenyl Trimethicone Copolymer, Dimethoxysilyl Ethylenediaminopropyl Dimethicone, Dimethylaminopropylamido PCA Dimethicone, Dimethyl Oxobenzo Dioxasilane, Dimethylsilanol Hyaluronate, Dioleyl Tocopheryl Methylsilanol, Diphenyl Amodimethicone, Diphenyl Dimethicone, Diphenyl Dimethicone Crosspolymer Diphenyl Dimethicone?/inyl Diphenyl Dimethicone/Silsesquioxane Crosspolymer, Diphenylethyl Benzyloxy Dilsiloxane, Diphenylisopropyl Dimethicone, Diphenylsiloxy Phenyl/Propyl Trimethicone, Diphenylsiloxy Phenyl Trimethicone Disiloxane, Disodium Amodimethicone Disuccinamide, Disodium PEG-12 Dimethicone Sulfosuccinate, Disodium PEG-8 Lauryl Dimethicone Sulfosuccinate, Divinyldimethicone/Dimethicone Copolymer, Divinyldimethicone/Dimethicone Crosspolymer, Drometrizole Trisiloxane, Ethylhexyl Acrylate/VP/Dimethicone Methacrylate Copolymer, Ethyl Methicone, Ethyl Trisiloxane, Fluoro C2-8 Alkyldimethicone, Gluconamidopropyl Aminopropyl Dimethicone, 4-(2-Beta-Glucopyranosiloxy) Propoxy-2-Hydroxybenzophenone, Glyceryl Undecyl Dimethicone, Glycidoxy Dimethicone, Hexadecyl Methicone, Hexyl Dimethicone, Hexyl Methicone, Hexyltrimethoxysilane, Hydrogen Dimethicone, Hydrogen Dimethicone/Octyl Silsesquioxane Copolymer, Hydrolyzed Collagen PG-Propyl Dimethiconol, Hydrolyzed Collagen PG-Propyl Methylsilanediol, Hydrolyzed Collagen PG-Propyl Silanetriol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Silk PG-Propyl Methylsilanediol Crosspolymer, Hydrolyzed Soy Protein/Dimethicone PEG-7 Acetate, Hydrolyzed Soy Protein PG-Propyl Methylsilanediol, Hydrolyzed Vegetable Protein PG-Propyl Silanetriol, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein PG-Propyl Methylsilanediol, Hydrolyzed Wheat Protein PG-Propyl Silanetriol, Hydroxyethyl Acetomonium PG-Dimethicone, Hydroxypropyldimethicone, Hydroxypropyl Dimethicone Behenate, Hydroxypropyl Dimethicone Isostearate, Hydroxypropyl Dimethicone Stearate, Isobutylmethacrylate/Bis-Hydroxypropyl Dimethicone Acrylate Copolymer, Isobutylmethacrylate/Trifluoroethylmethacrylate/Bis-Hydroxypropyl Dimethicone Acrylate Copolymer, Isopentyl Trimethoxycinnamate Trisiloxane, Isopolyglyceryl-3 Dimethicone, Isopolyglyceryl-3 Dimethiconol, Isopropyl Titanium Triisostearate/Triethoxysilylethyl, Polydimethylsiloxyethyl Dimethicone Crosspolymer, Isostearyl Carboxydecyl PEG-8 Dimethicone, Lactoyl Methylsilanol Elastinate, Lauryl Dimethicone, Lauryl Dimethicone PEG-15 Crosspolymer, Lauryl Dimethicone PEG- 10 Phosphate, Lauryl Dimethicone/Polyglycerin-3 Crosspolymer, Lauryl Methicone, Lauryl PEG-8 Dimethicone, Lauryl PEG-10 Methyl Ether Dimethicone, Lauryl PEG-9 Polydimethylsiloxyethyl Dimethicone, Lauryl PEG/PPG-18/18 Methicone, Lauryl Phenylisopropyl Methicone, Lauryl Phenylpropyl Methicone, Lauryl Polydimethylsiloxyethyl Dimethicone/Bis-Vinyldimethicone Crosspolymer, Lauryl Polyglyceryl-3 Polydimethylsiloxyethyl Dimethicone, Lauryl Trimethicone, Linoleamidopropyl PG-Dimonium Chloride Phosphate Dimethicone, Methacryloyl Propyltrimethoxysilane, Methicone, Methoxy Amodimethicone/Silsesquioxane Copolymer, Methoxycinnamidopropyl Polysilsesquioxane, Methoxycinnamoylpropyl Silsesquioxane Silicate, Methoxy PEG-13 Ethyl Polysilsesquioxane, Methoxy PEG/PPG-7/3 Aminopropyl Dimethicone, Methoxy PEG/PPG-25/4 Dimethicone, Methoxy PEG-10 Propyltrimethoxysilane, Methyleugenyl PEG- 8 Dimethicone, Methylpolysiloxane Emulsion, Methylsilanol Acetylmethionate, Methylsilanol Acetyltyrosine, Methylsilanol Ascorbate, Methylsilanol Carboxymethyl Theophylline, Methylsilanol Carboxymethyl Theophylline Alginate, Methylsilanol Elastinate, Methylsilanol Glycyrrhizinate, Methylsilanol Hydroxyproline, Methylsilanol Hydroxyproline Aspartate, Methylsilanol Mannuronate, Methylsilanol PCA, Methylsilanol PEG-7 Glyceryl Cocoate, Methylsilanol/Silicate Crosspolymer, Methylsilanol Spirulinate, Methylsilanol Tri-PEG-8 Glyceryl Cocoate, Methyl Trimethicone, Methyltrimethoxysilane, Myristylamidopropyl Dimethylamine Dimethicone PEG-7 Phosphate, Myristyl Methicone, Myristyl Trisiloxane, Nylon-611/Dimethicone Copolymer, PCA Dimethicone, PEG-7 Amodimethicone, PEG-8 Amodimethicone, PEG-8 Cetyl Dimethicone, PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone, PEG-17 Dimethicone, PEG-10 Dimethicone Crosspolymer, PEG-12 Dimethicone Crosspolymer, PEG-8 Dimethicone Dimer Dilinoleate, PEG-8 Dimethicone/Dimer Dilinoleic Acid Copolymer, PEG-10 Dimethicone/Vinyl Dimethicone Crosspolymer, PEG-8 Distearmonium Chloride PG-Dimethicone, PEG-10/Lauryl Dimethicone Crosspolymer, PEG- 15/Lauryl Dimethicone Crosspolymer, PEG-15/Lauryl Polydimethylsiloxyethyl Dimethicone Crosspolymer, PEG-8 Methicone, PEG-6 Methicone Acetate, PEG-6 Methyl Ether Dimethicone, PEG- 7 Methyl Ether Dimethicone, PEG-8 Methyl Ether Dimethicone, PEG-9 Methyl Ether Dimethicone, PEG-10 Methyl Ether Dimethicone, PEG-11 Methyl Ether Dimethicone, PEG-32 Methyl Ether Dimethicone, PEG-8 Methyl Ether Triethoxysilane, PEG-10 Nonafluorohexyl Dimethicone Copolymer, PEG-4 PEG-12 Dimethicone, PEG-8 PG-Coco-Glucoside Dimethicone, PEG-9 Polydimethylsiloxyethyl Dimethicone, PEG/PPG-20/22 Butyl Ether Dimethicone, PEG/PPG-22/22 Butyl Ether Dimethicone, PEG/PPG-23/23 Butyl Ether Dimethicone, PEG/PPG-24/18 Butyl Ether Dimethicone, PEG/PPG-27/9 Butyl Ether Dimethicone, PEG/PPG-3/10 Dimethicone, PEG/PPG-4/12 Dimethicone, PEG/PPG-6/4 Dimethicone, PEG/PPG-6/11 Dimethicone, PEG/PPG-8/14 Dimethicone, PEG/PPG-8/26 Dimethicone, PEG/PPG-10/2 Dimethicone, PEG/PPG-12/16 Dimethicone, PEG/PPG- 12/18 Dimethicone, PEG/PPG-14/4 Dimethicone, PEG/PPG-15/5 Dimethicone, PEG/PPG-15/15 Dimethicone, PEG/PPG-16/2 Dimethicone, PEG/PPG-16/8 Dimethicone, PEG/PPG-17/18 Dimethicone, PEG/PPG-18/6 Dimethicone, PEG/PPG-18/12 Dimethicone, PEG/PPG-18/18 Dimethicone, PEG/PPG-19/19 Dimethicone, PEG/PPG-20/6 Dimethicone, PEG/PPG-20/15 Dimethicone, PEG/PPG-20/20 Dimethicone, PEG/PPG-20/23 Dimethicone, PEG/PPG-20/29 Dimethicone, PEG/PPG-22/23 Dimethicone, PEG/PPG-22/24 Dimethicone, PEG/PPG-23/6 Dimethicone, PEG/PPG-25/25 Dimethicone, PEG/PPG-27/27 Dimethicone, PEG/PPG-30/10 Dimethicone, PEG/PPG-25/25 Dimethicone/Acrylates Copolymer, PEG/PPG-20/22 Methyl Ether Dimethicone, PEG/PPG-24/24 Methyl Ether Glycidoxy Dimethicone, PEG/PPG-10/3 Oleyl Ether Dimethicone, PEG/PPG-5/3 Trisiloxane, PEG-4 Trifluoropropyl Dimethicone Copolymer, PEG-8 Trifluoropropyl Dimethicone Copolymer, PEG-10 Trifluoropropyl Dimethicone Copolymer, PEG-8 Trisiloxane, Perfluorocaprylyl riethoxysilylethyl Methicone, Perfluorononyl Dimethicone, Perfluorononyl Dimethicone/Methicone/Amodimethicone Crosspolymer, Perfluorononylethyl Carboxydecyl Behenyl Dimethicone, Perfluorononylethyl Carboxydecyl Hexacosyl Dimethicone, Perfluorononylethyl Carboxydecyl Lauryl/Behenyl Dimethicone, Perfluorononylethyl Carboxydecyl Lauryl Dimethicone, Perfluorononylethyl Carboxydecyl PEG-8 Dimethicone, Perfluorononylethyl Carboxydecyl PEG-10 Dimethicone, Perfluorononylethyl Dimethicone/Methicone Copolymer, Perfluorononylethyl PEG-8 Dimethicone, Perfluorononylethyl Stearyl Dimethicone, Perfluorooctylethyl/Diphenyl Dimethicone Copolymer, Perfluorooctylethyl Triethoxysilane, Perfluorooctylethyl Trimethoxysilane, Perfluorooctylethyl Trisiloxane, Perfluorooctyl Triethoxysilane, PG-Amodimethicone, Phenethyl Dimethicone, Phenethyl Disiloxane, Phenyl Dimethicone, Phenylisopropyl Dimethicone, Phenyl Methicone, Phenyl Methiconol, Phenylpropyldimethylsiloxysilicate, Phenylpropyl Ethyl Methicone, Phenyl Propyl Trimethicone, Phenyl Propyl Trimethicone/Diphenylmethicone, Phenyl Trimethicone, Platinum Divinyldisiloxane, Polyacrylate-6, Polydiethylsiloxane, Polydimethylsiloxyethyl Dimethicone/Bis-Vinyldimethicone Crosspolymer, Polydimethylsiloxyethyl Dimethicone/Methicone Copolymer, Polydimethylsiloxy PEG/PPG-24/19 Butyl Ether Silsesquioxane, Polydimethylsiloxy PPG- 13 Butyl Ether Silsesquioxane, Polyglyceryl-3 Disiloxane Dimethicone, Polyglyceryl-3/Lauryl Polydimethylsiloxyethyl Dimethicone Crosspolymer, Polyglyceryl-3 Polydimethylsiloxyethyl Dimethicone, Poly(Glycol Adipate)/Bis-Hydroxyethoxypropyl Dimethicone Copolymer, Polymethylsilsesquioxane, Polymethylsilsesquioxane/Trimethylsiloxysilicate, Polyphenylsilsesquioxane, Polypropylsilsesquioxane, Polysilicone-1, Polysilicone-2, Polysilicone-3, Polysilicone-4, Polysilicone-5, Polysilicone-6, Polysilicone-7, Polysilicone-8, Polysilicone-9, Polysilicone-10, Polysilicone-11, Polysilicone-12, Polysilicone-13, Polysilicone-14, Polysilicone-15, Polysilicone-16, Polysilicone-17, Polysilicone-18, Polysilicone-19, Polysilicone-20, Polysilicone-21, Polysilicone-18 Cetyl Phosphate, Polysilicone-1 Crosspolymer, Polysilicone-18 Stearate, Polyurethane-10, Potassium Dimethicone PEG-7 Panthenyl Phosphate, Potassium Dimethicone PEG- 7 Phosphate, PPG-12 Butyl Ether Dimethicone, PPG-2 Dimethicone, PPG-12 Dimethicone, PPG-27 Dimethicone, PPG-4 Oleth-10 Dimethicone, Propoxytetramethyl Piperidinyl Dimethicone, Propyl Trimethicone, Quaternium-80, Retinoxytrimethylsilane, Silanediol Salicylate, Silanetriol, Silanetriol Arginate, Silanetriol Glutamate, Silanetriol Lysinate, Silanetriol Melaninate, Silanetriol Trehalose Ether, Silica, Silica Dimethicone Silylate, Silica Dimethyl Silylate, Silica Silylate, Silicon Carbide, Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, SiliconeQuaternium-16, Silicone Quaternium-16/Glycidoxy Dimethicone Crosspolymer, Silicone Quaternium-17, Silicone Quaternium- 18, Silicone Quaternium-19, Silicone Quaternium-20, Silicone Quaternium-21, Silicone Quaternium- 22, Silicone Quaternium-24, Silicone Quaternium-25, Siloxanetriol Alginate, Siloxanetriol Phytate, Simethicone, Sodium Carboxydecyl PEG-8 Dimethicone, Sodium Dimethicone PEG-7 Acetyl Methyltaurate, Sodium Hyaluronate Dimethylsilanol, Sodium Lactate Methylsilanol, Sodium Mannuronate Methylsilanol, Sodium PCA Methylsilanol, Sodium PG-Propyldimethicone Thiosulfate Copolymer, Sodium PG-Propyl Thiosulfate Dimethicone, Sodium Propoxyhydroxypropyl Thiosulfate Silica, Sorbityl Silanediol, Soy Triethoxysilylpropyldimonium Chloride, Stearalkonium Dimethicone PEG-8 Phthalate, Stearamidopropyl Dimethicone, Steardimonium Hydroxypropyl Panthenyl PEG-7 Dimethicone Phosphate Chloride, Steardimonium Hydroxypropyl PEG-7 Dimethicone Phosphate Chloride, Stearoxy Dimethicone, Stearoxymethicone/Dimethicone Copolymer, Stearoxytrimethylsilane, Stearyl Aminopropyl Methicone, Stearyl Dimethicone, Stearyl/Lauryl Methacrylate Crosspolymer, Stearyl Methicone, Stearyl Triethoxysilanek, Stearyl Trimethicone, Styrene/Acrylates/Dimethicone Acrylate Crosspolymer, Styrene/Acrylates/Dimethicone Copolymer, TEA-Dimethicone PEG-7 Phosphate, Tetrabutoxypropyl Trisiloxane, Tetramethyl Hexaphenyl Tetrasiloxane, Tetramethyl Tetraphenyl Trisiloxane, Tocopheryloxypropyl Trisiloxane, Trideceth-9 PG-Amodimethicone, Triethoxycaprylylsilane, Triethoxysilylethyl Dimethicone/Methicone Copolymer, Triethoxysilylethyl Polydimethylsiloxyethyl Dimethicone, Triethoxysilylethyl Polydimethylsiloxyethyl Hexyl Dimethicone, Triethoxysilylpropylcarbamoyl Ethoxypropyl Butyl Dimethicone, Trifluoromethyl C1-4 Alkyl Dimethicone, Trifluoropropyl Cyclopentasiloxane, Trifluoropropyl Cyclotetrasiloxane, Trifluoropropyl Dimethicone, Trifluoropropyl Dimethicone/PEG-10 Crosspolymer, Trifluoropropyl Dimethicone/Trifluoropropyl Divinyldimethicone Crosspolymer, Trifluoropropyl Dimethicone/Vinyl Trifluoropropyl, Dimethicone/Silsesquioxane Crosspolymer, Trifluoropropyl Dimethiconol, Trifluoropropyldimethyl/trimethylsiloxysilicate, Trifluoropropyl Methicone, Trimethoxycaprylylsilane, Trimethoxysilyl Dimethicone, Trimethyl Pentaphenyl Trisiloxane, Trimethylsiloxyamodimethicone, Trimethylsiloxyphenyl Dimethicone, Trimethylsiloxysilicate, Trimethylsiloxysilicate/Dimethicone Crosspolymer, Trimethylsiloxysilicate/Dimethiconol Crosspolymer, Trimethylsiloxysilylcarbamoyl Pullulan, Trimethylsilyl Hydrolyzed Conchiolin Protein PG-Propyl Methylsilanediol Crosspolymer, Trimethylsilyl Hydrolyzed Silk PG-Propyl Methylsilanediol Crosspolymer, Trimethylsilyl Hydrolyzed Wheat Protein PG-Propyl Methylsilanediol Crosspolymer, Trimethylsilyl Pullulan, Trimethylsilyl Trimethylsiloxy Glycolate, Trimethylsilyl Trimethylsiloxy Lactate, Trimethylsilyl Trimethylsiloxy Salicylate, Triphenyl Trimethicone, Trisiloxane, Tris-Tributoxysiloxymethylsilane, Undecylcrylene Dimethicone, Vinyl Dimethicone, Vinyl Dimethicone/Lauryl Dimethicone Crosspolymer, Vinyl Dimethicone/Methicone Silsesquioxane Crosspolymer, Vinyldimethyl/Trimethylsiloxysilicate Stearyl Dimethicone Crosspolymer, VP/Dimethiconylacrylate/Polycarbamyl/Polyglycol Ester, Zinc Carboxydecyl Trisiloxane, Zinc Dimethicone PEG-8 Succinate, and mixtures thereof.

More preferably the silicones to be contained in the cosmetic or pharmaceutical composition according to the invention are Dimethicone, Cyclomethicone, Cyclopentasiloxane, Cyclotetrasiloxane, Phenyl Trimethicone, and Cyclohexasiloxane.

**Waxes and/or stabilizers:** Besides natural oils used, one or more waxes may also be present in the cosmetic or pharmaceutical composition according to the present invention, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes. Metal salts of fatty acids such as, for example, magnesium, aluminium and/or zinc stearate or ricinoleate may be used as stabilizers.

Apart from the above-mentioned liquid UV filters, the cosmetic or pharmaceutical composition as defined herein, is advantageously combined with at least one further primary sun protection factor and/or with at least one further secondary sun protection factor, in order to increase the SPF, i.e. to obtain a high SPF and to cover a broad UVA and UVB range.

**Primary sun protection factors:** In addition to the above disclosed solid UV filters, the cosmetic or pharmaceutical composition according to the present invention preferably includes one or more further primary sun protection factors. Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The cosmetic or pharmaceutical composition according to the invention advantageously contains at least one UV-A filter and/or at least one UV-B filter and/or a broadband filter and/or at least one inorganic pigment. Compositions according to the invention preferably contain at least one UV-B filter or a broadband filter, more particularly preferably at least one UV-A filter and at least one UV-B filter. Preferred cosmetic compositions, preferably topical compositions according to the present invention comprise one, two, three or more sun protection factors selected from the group consisting of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

In addition, a combination with active ingredients which penetrate into the skin and protect the skin cells from inside against sunlight-induced damage and reduce the level of cutaneous matrix metalloproteases is advantageously. Preferred respective ingredients are so called arylhydrocarbon receptor antagonists. Preferred is 2-benzylidene-5,6-dimethoxy-3,3-dimethylindan-1-one.

The UV filters cited below which can be used within the context of the present invention are preferred but naturally are not limiting.

UV filters which are preferably used in the cosmetic or pharmaceutical composition according to the present invention are selected from the group consisting of
- 1-(4-tert-Butylphenyl)-3-(4-methoxyphenyl)-propane-1,3-dione (INCI: Butyl Methoxydibenzoylmethane; Avobenzone),
- Sodium salt of 2,2'-bis(1,4-phenylene)-1H-benzimidazole-4,6-disulfonic acid (INCI: Disodium Phenyl Dibenzimidazole Tetrasulfonate),
- 2-Hydroxy-4-methoxybenzophenone (INCI: Benzophenone-3),
- 2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol),
- Isopentyl-4-methoxycinnamate (INCI: Isoamyl p-Methoxycinnamate; Amiloxate),
- 2-Phenylbenzimidazole-5-sulfonic acid and its potassium, sodium and triethanolamine salts (INCI: Phenylbenzimidazole Sulfonic Acid),
- 3-(4-Methylbenzylidene)-d1 camphor (INCI: 4-Methylbenzylidene Camphor),
- 1,3,5-Triazine-2,4,6-tris[1,1'-biphenyl]-4-yl (INCI: Tris-biphenyl Triazine, incl. Nanomaterial),
- 2-Hydroxy-4-methoxybenzophenone- 5-sulfonic acid and its sodium salt (INCI: Benzophenone-4/Benzophenone-5),
- Zinc Oxide (INCI: Zinc Oxide),
- Benzoic acid, 2-[-4-(diethylamino)-2-hydroxybenzoyl]-hexylester (INCI: Diethylamino Hydroxybenzoyl Hexyl Benzoate),
- 2,4,6-Trianilino-(p-carbo-2'-ethylhexyl-1'-oxy)-1,3,5-triazine (INCI: Ethylhexyl Triazone),
- alpha-(2-Oxoborn-3-ylidene)-toluene-4-sulphonic acid and its salts (INCI: Benzylidene Camphor Sulfonic Acid),
- Polymer of N-{(2 and 4)-[(2-oxoborn-3-ylidene)-methyl]-benzyl}-acrylamide (INCI: Polyacrylamido Methyl Benzylidene Camphor),
- 3,3'-(1,4-Phenylenedimethylene)-bis-(7, 7-dimethyl-2-oxobicyclo-[2.2.1]-hept-1-yl-methanesulfonic acid) and its salts (INCl: Terephthalylidene Dicamphor Sulfonic Acid),
- Ethoxylated Ethyl-4-aminobenzoate (INCI: PEG-25 PABA),
- Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone),
- Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCl: Drometrizole Trisiloxane),
- 3-Benzylidene camphor (INCI: 3-Benzylidene Camphor),
- Titanium dioxide (INCI: Titanium Dioxide), and
mixtures of two or more of the aforesaid solid UV filters.

UV filters which are also preferably used are selected from the group consisting of:
- p-aminobenzoic acid,
- p-aminobenzoic acid ethyl ester (25 mol) ethoxylated (INCI name: PEG-25 PABA),
- p-dimethylaminobenzoic acid-2-ethylhexyl ester,
- p-aminobenzoic acid ethyl ester (2 mol) N-propoxylated,
- p-aminobenzoic acid glycerol ester,
- salicylic acid homomenthyl ester (homosalates) (Neo Heliopan^{®}HMS),
- salicylic acid-2-ethylhexyl ester (Neo Heliopan^{®}OS),
- triethanolamine salicylate,
- 4-isopropyl benzyl salicylate,
- anthranilic acid menthyl ester (Neo Heliopan^{®}MA),
- diisopropyl cinnamic acid ethyl ester,
- p-methoxycinnamic acid-2-ethylhexyl ester (Neo Heliopan^{®}AV),
- diisopropyl cinnamic acid methyl ester,
- p-methoxycinnamic acid isoamyl ester (Neo Heliopan^{®}E 1000),
- p-methoxycinnamic acid diethanolamine salt,
- p-methoxycinnamic acid isopropyl ester,
- 2-phenylbenzimidazole sulfonic acid and salts (Neo Heliopan^{®}Hydro),
- 3-(4'-trimethylammonium) benzylidene bornan-2-one methyl sulfate,
- beta-imidazole-4(5)-acrylic acid (urocanic acid),
- 3-(4'-sulfo)benzylidene bornan-2-one and salts,
- 3-(4'-methyl benzylidene)-D,L-camphor (Neo Heliopan^{®}MBC),
- 3-benzylidene-D,L-camphor,
- N-[(2 and 4)-[2-(oxoborn-3-ylidene) methyl]benzyl] acrylamide polymer,
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB),
- benzylidene malonate polysiloxane (Parsol^{®}SLX),
- glyceryl ethylhexanoate dimethoxycinnamate,
- dipropylene glycol salicylate,
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150),
and mixutres thereof

In a preferred variant the cosmetic or pharmaceutical composition according to the present invention comprises a combination with one or more broadband filters which are selected from the group consisting of:
- 2-ethylhexyl-2-cyano-3,3-diphenyl acrylate,
- ethyl-2-cyano-3,3'-diphenyl acrylate,
- dihydroxy-4-methoxybenzophenone,
- 2,4-dihydroxybenzophenone,
- tetrahydroxybenzophenone,
- 2,2'-dihydroxy-4,4'-dimethoxybenzophenone,
- 2-hydroxy-4-n-octoxybenzophenone,
- 2-hydroxy-4-methoxy-4'-methylbenzophenone,
- sodium hydroxymethoxybenzophenone sulfonate,
- disodium-2,2'-dihydroxy-4,4'-dimethoxy-5,5'-disulfobenzophenone,
- phenol, 2-(2H-benzotriazol-2-yl)-4-methyl-6-(2-methyl-3(1,3,3,3-tetramethyl-1-(trimethylsilyl)oxy)disiloxyanyl)propyl),
- 2,2'-methylene-bis-(6-(2H-benzotriazol-2-yl)-4-1,1,3,3-tetramethylbutyl)phenol) (Methylene-Bis-Benzotriazolyl Tetramethylbutylphenol),
- 2,4-bis-[4-(2-ethylhexyloxy)-2-hydroxyphenyl]-1,3,5-triazine,
- 2,4-bis-[{(4-(2-ethylhexyloxy)-2-hydroxy}phenyl]-6-(4-methoxyphenyl)-1,3,5-triazine (INCI: Aniso Triazin),
- 2,4-bis-[{(4-(3-sulfonato)-2-hydroxypropyloxy)-2-hydroxy}phenyl]-6-(4-methoxyphenyl)-1,3,5-triazine sodium salt,
- 2,4-bis-[{(3-(2-propyloxy)-2-hydroxypropyloxy)-2-hydroxy}phenyl]-6-(4-methoxyphenyl)-1,3,5-triazine,
- 2,4-bis-[{4-(2-ethylhexyloxy)-2-hydroxy}phenyl]-6-[4-(2-methoxyethylcarbonyl) phenylamino]-1,3,5-triazine,
- 2,4-bis-[{4-(3-(2-propyloxy)-2-hydroxypropyloxy)-2-hydroxy}phenyl]-6-[4-(2-ethylcarboxyl) phenylamino]-1,3,5-triazine,
- 2,-bis-[{4-(2-ethylhexyloxy)-2-hydroxy}phenyl]-6-(1-methylpyrrol-2-yl)-1,3,5-triazine,
- 2,4-bis-[{4-tris-(trimethylsiloxysilylpropyloxy)-2-hydroxy}phenyl]-6-(4-methoxyphenyl)-1,3,5-triazine,
- 2,4-bis-[{4-(2"-methylpropenyloxy)-2-hydroxy}phenyl]-6-(4-methoxyphenyl)-1,3,5-triazine,
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine,
- dioctylbutylamidotriazone (INCI: Dioctylbutamidotriazone),
- 2,4-bis-[5-1(dimethylpropyl)benzoxazol-2-yl-(4-phenyl)-imino]-6-(2-ethylhexyl)-imino-1,3,5-triazine,
- 4,4',4"-(1,3,5-triazine-2,4,6-triyltriimino)tris-benzoic acid-tris(2-ethyl-hexylester) (also: 2,4,6-tris[anilino(p-carbo-2'-ethyl-1'-hexyloxy)]-1,3,5-triazine (INCI: Octyl Triazone),
- 2,4,6-tribiphenyl-4-yl-1,3,5-triazine,
- 2-Ethylhexyl 4-methoxycinnamate (Ethylhexyl Methoxycinnamate),
- Benzoic acid, 2-hydroxy-, 3,3,5-trimethylcyclohexyl ester (Homosalate),
- 2-Ethylhexyl salicylate (Ethylhexyl Salicylate),
- 2-Ethylhexyl 4-(dimethylamino)benzoate (Ethylhexyl Dimethyl PABA),
- N,N,N-Trimethyl-4-(2-oxoborn-3-ylidenemethyl) anilinium methyl sulfate (Camphor Benzalkonium Methosulfate),
- Dimethicodiethylbenzalmalonate (Polysilicone-15),
and mixtures thereof.

In a preferred variant the cosmetic or pharmaceutical composition according to the present invention comprises a combination with one or more UV-A filters which are selected from the group consisting of:
- 4-isopropyl dibenzoyl methane,
- terephthalylidene dibornane sulfonic acid and salts,
- 4-t-butyl-4'-methoxydibenzoyl methane (avobenzone),
- phenylene bis-benzimidazyl tetrasulfonic acid disodium salt,
- 2,2'-(1,4-phenylene)-bis-(1H-benzimidazole-4,6-disulfonic acid), monosodium salt,
- 2-(4-diethylamino-2-hydroxybenzoyl) benzoic acid hexyl ester,
- indanylidene compounds,
and mixtures thereof.

In a more preferred variant, the cosmetic or pharmaceutical composition according to the present invention comprises a combination with one or more UV filters which are selected from the group consisting of:
- p-aminobenzoic acid,
- 3-(4'-trimethylammonium) benzylidene bornan-2-one methyl sulfate.
- salicylic acid homomenthyl ester (Neo Heliopan^{®}HMS),
- 2-phenylbenzimidazole sulfonic acid (Neo Heliopan^{®}Hydro),
- terephthalylidene dibornane sulfonic acid and salts (Mexoryl^{®}SX),
- 4-tert-butyl-4'-methoxydibenzoyl methane (Neo Heliopan^{®}357),
- 3-(4'-sulfo)benzylidene bornan-2-one and salts,
- 2-ethylhexyl-2-cyano-3,3-diphenyl acrylate (Neo Heliopan^{®}303),
- N-[(2 and 4)-[2-(oxoborn-3-ylidene) methyl]benzyl] acrylamide polymer,
- p-methoxycinnamic acid-2-ethylhexyl ester (Neo Heliopan^{®}AV),
- p-aminobenzoic acid ethyl ester (25 mol) ethoxylated (INCI name: PEG-25 PABA),
- p-methoxycinnamic acid isoamyl ester (Neo Heliopan^{®}E1000),
- 2,4,6-trianilino-(p-carbo-2'-ethylhexyl-1'-oxy)-1,3,5-triazine (Uvinul^{®}T150),
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL),
- 4,4'-[(6-[4-(1,1-dimethyl)aminocarbonyl) phenylamino]-1,3,5-triazine-2,4-diyl)di-imino]-bis-(benzoic acid-2-ethylhexyl ester) (Uvasorb HEB),
- 3-(4'-methyl benzylidene)-D,L-camphor (Neo Heliopan^{®}MBC),
- 3-benzylidene camphor,
- salicylic acid-2-ethylhexyl ester (Neo Heliopan^{®}OS),
- 4-dimethylaminobenzoic acid-2-ethylhexyl ester (Padimate O),
- hydroxy-4-methoxybenzophenone-5-sulfonic acid and Na salt,
- 2,2'-methylene bis-(6-(2H-benzotriazol-2-yl)-4-1,1,3,3-tetramethylbutyl) phenol) (Tinosorb^{®}M),
- phenylene bis-benzimidazyl tetrasulfonic acid disodium salt (Neo Heliopan^{®}AP),
- 2,4-bis-[{(4-(2-ethylhexyloxy)-2-hydroxy}phenyl]-6-(4-methoxyphenyl)-1,3,5-triazine (Tinosorb^{®}S),
- benzylidene malonate polysiloxane (Parsol^{®}SLX),
- menthyl anthranilate (Neo Heliopan^{®}MA),
- 2-(4-diethylamino-2-hydroxybenzoyl) benzoic acid hexyl ester (Uvinul^{®} A Plus),
- indanylidene compounds,
and mixtures thereof.

In a further preferred variant the cosmetic or pharmaceutical composition according to the invention contains a total amount of sunscreen agents, i.e. in particular UV filters and/or inorganic pigments (UV filtering pigments) so that the composition according to the invention has a sun protection factor SPF of 5 to 50, preferably 5 to 60. Such compositions according to the invention are particularly suitable for protecting the skin and hair.

**Secondary sun protection factors:** Besides the groups of primary sun protection factors mentioned above, secondary sun protection factors of the antioxidant type may also be advantageously used in the cosmetic or pharmaceutical composition according to the present invention. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxy fatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts. The total quantity of inorganic pigments, in particular hydrophobic inorganic micro-pigments in the finished cosmetic preparation according to the present invention is advantageously from 0.1 to 30 % by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

Actives modulating skin and/or hair pigmentation: The cosmetic or pharmaceutical composition according to the present invention may include active ingredients for skin and/or hair lightening. Preferred active ingredients for skin and/or hair lightening are selected from the group consisting of kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates, sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract.

Preferred skin lighteners are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophyl-line and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-lle-Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(ll) bicarbonate complexes ("pseudocat-alases"), tetrasubstituted cyclohexene derivatives, isoprenoids, melanin derivatives such as Melasyn-100 and MelanZe, diacyl glycerols, aliphatic or cyclic diols, psoralens, prostaglandins and analogues thereof, activators of adenylate cyclase and compounds which activate the transfer of melanosomes to keratinocytes such as serine proteases or agonists of the PAR-2 receptor, extracts of plants and plant parts of the chrysanthemum species, sanguisorba species, walnut extracts, urucum extracts, rhubarb extracts, microalgae extracts, in particular Isochrysis galbana, trehalose, erythrulose and dihydroxyacetone. Flavonoids which bring about skin and hair tinting or browning (e.g. quercetin, rhamnetin, kaempferol, fisetin, genistein, daidzein, chrysin and apigenin, epicatechin, diosmin and diosmetin, morin, quercitrin, naringenin, hesperidin, phloridzin and phloretin) can also be used.

**Hair growth activators or inhibitors:** Cosmetic or pharmaceutical compositions according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormones, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, the cosmetic or pharmaceutical composition according to the present invention may include one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

**Enzyme inhibitors:** The cosmetic or pharmaceutical composition according to the present invention may comprise one or more enzyme inhibitors. Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

**Odour absorbers and/or antiperspirant active agents:** The cosmetic or pharmaceutical composition according to the present invention may include one or more odour absorbers and/or antiperspirant active agents (antiperspirants). Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide **NP,** evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2-propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

**Film formers:** The cosmetic or pharmaceutical composition according to the present invention may include one or more film formers. Standard film formers are preferably chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

**Carriers and hydrotropes:** The cosmetic or pharmaceutical composition according to the present invention may comprise a carrier or a mixture of different carriers. Preferred cosmetics carrier materials are solid or liquid at 25 °C and 1013 mbar (including highly viscous substances) as for example glycerol, 1,2-propylene glycol, 1,2-butylene glycol, 1,3-propylene glycol, 1,3-butylene glycol, ethanol, water, and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

The cosmetic or pharmaceutical composition as defined herein optionally include powders. The optional powders provide formulas that are smoother and softer on the skin. Representative powders include talc, mica, magnesium carbonate, calcium carbonate, magnesium silicate, aluminium magnesium silicate, silica, titanium dioxide, zinc oxide, red iron oxide, yellow iron oxide, black iron oxide, polyethylene powder, methacrylate powder, polystyrene powder, silk powder, Preferred solid powder materials, which may be a component of the cosmetic or pharmaceutical composition according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin, inulin, and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine; and
- dialcoholamines, such as diethanolamine or 2-aminopropane-1,3-diol.

**Dyes:** The cosmetic or pharmaceutical composition according to the present invention may comprise one or more dyes. Suitable dyes are any of the substances suitable and approved for cosmetic purposes. Examples include cochineal red A (CI. 16255), patent blue V (CI. 42051), indigotin (CI. 73015), chlorophyllin (CI. 75810), quinoline yellow (CI. 47005), titanium dioxide (CI. 77891), indanthrene blue RS (CI. 69800) and madder lake (CI. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

In addition to the above-described substances, further ingredients commonly used in the cosmetic or pharmaceutical industry, which are suitable or customary in the compositions of the present invention, can be used.

The mixture comprising at least one first linear alkanediol and one or more second linear alkanediol according to the present invention is present in the cosmetic or pharmaceutical composition or homecare product in an amount of 0.3 to 3.0 % by weight, based on the total weight of the composition. In a most preferred variant, the mixture comprising at least one first linear alkanediol and one or more second linear alkanediol is advantageously used in the cosmetic or pharmaceutical composition or homecare product in an amount of at 0.5 to 1.0 % by weight, based on the total weight of the composition or homecare product.

For the alkane mixture, the above amounts relate to the total content of the first linear alkanediols and the second linear alkanediols in the mixture, i.e. the amount is the sum of the content of the first linear alkanediols and the second linear alkanediols in the mixture.

In a particular preferred variant according to the present invention, the cosmetic or pharmaceutical composition or the homecare product comprises the 2,3-alkandediol of the mixture comprising at least one first linear alkanediol and one or more second linear alkanediol in an amount of 0.5 to 1.0 % by weight, based on the total weight of the composition or homecare product.

Even more preferred, the cosmetic or pharmaceutical composition or the homecare product comprises the 2,3-alkanediol of the mixture comprising at least one first linear alkanediol and one or more second linear alkanediol in an amount of 0.001 to 0.5 % weight, preferably in an amount of 0.005 to 0.1 % by weight and most preferably in an amount of 0.01 to 0.075 % by weight, based on the total weight of the composition or homecare product.

The natural polymer is present in the cosmetic or pharmaceutical composition or homecare product according to the present invention, in an amount of 0.05 to 6.0 % by weight, based on the total weight of the composition or the homecare product.

Compared with the use of the single 1,2-alkanediol or 2,3-alkanediol, the alkanediol mixtures according to the present invention have a synergistically intensified action, i.e. they reduce the microbial load in the cosmetic or pharmaceutical preparation or homecare product comprising the natural polymer in a synergistic way. This effect or action is especially pronounced for homo alkanediol mixtures as defined herein including including 1,2-heptanediol and 2,3-heptanediol

Also alkanediol mixtures with a different first linear alkanediol and second linear alkanediol such as hetero alkanediol mixtures as defined herein including 1,2-heptanediol and 1,2-nonanediol show a synergistically intensified action in reducing microbial load in the cosmetic or pharmaceutical preparation or homecare product comprising the natural polymer as it is demonstrated by the following experimental data, compared with the use of the single 1,2-alkanediols.

Without the use of an alkanediol mixture as specified herein, the microbial load in the cosmetic or pharmaceutical composition or homecare product comprising the natural polymer as defined herein, would increase, so that storage of such formulations would be problematic or even impossible.

Due to this advantageously effect, natural polymer solutions or cosmetic or pharmaceutical compositions or homecare products comprising a natural polymer as defined herein can be microbially stabilized by said alkandediol mixtures by reducing their microbial load. This effect can even be maintained during storage. The microbial stabilization allows the cosmetic or pharmaceutical compositions or the homecare product to be stored without microbial degradation and deterioration.

The cosmetic or pharmaceutical, in particular dermatological, composition according to the present invention is intended for topical applications. The term "topical" is understood to mean external applications on a mammal's skin, which are in particular for the treatment, protection, care and cleansing of the skin, scalp, eyelashes, eyebrows, nails, mucous membranes and hair. The mammal is preferably a human.

For topical application, the cosmetic or pharmaceutical composition is either a rinse off or leave on preparation.

The cosmetic or pharmaceutical, in particular dermatological, composition according to the present invention can be present in different forms of, e.g. in the form of a dispersion, in the form of a liquid surfactant formulation, or in the form of an aqueous, aqueous/alcoholic, in particular aqueous/ethanolic, or an aqueous/glycolic based solution.

In a particular preferred variant, the cosmetic or pharmaceutical composition according to the present invention is a cold formulation preparation, which can be prepared without a heating step.

In a preferred variant, the cosmetic or pharmaceutical, preferably dermatological, composition according to the present invention is a dispersion. The term "dispersion" in the context of the present invention means, that the cosmetic or pharmaceutical composition is a disperse two-phase system consisting of colloidal particles (disperse phase) and a medium in which they are suspended (disperse medium). Both phases are not miscible with each other, only with an emulsifier. Such dispersions, for example emulsions, comprise the oil component preferably in an amount of ≥ 1 % by weight, more preferably in an amount of ≥ 3 % by weight.

If the cosmetic or pharmaceutical composition according to the present invention is a dispersion, preferably an emulsion, the oil component is present in the cosmetic or pharmaceutical composition in an amount of 0.01 % to 50.0 by weight, based on the total weight of the composition. In a preferred variant, the cosmetic or pharmaceutical composition comprises the oil component in an amount of 1.0 to 40.0 % by weight, based on the total weight of the composition. In a particular preferred variant, the oil component is advantageously used in the cosmetic or pharmaceutical composition in an amount of at 3.0 to 30 % by weight, based on the total weight of the composition.

Preferably, the cosmetic or pharmaceutical composition according to the present invention takes various forms such as an emulsion, in particular a O/W emulsion, a W/O emulsion, a multiple emulsion, a hydrodispersion gel, a balm, a multiple emulsion of the water-in-oil type (W/O/WO) or of the oil-in-water type (O/W/O), PIT emulsion, Pickering emulsion, a micro-emulsion, a liquid, a lotion, a suspension, a milk, an ointment, a paste, a gel, a cream based, an oil based or a liposome-based formulation.

In a further alternative, the cosmetic or pharmaceutical composition according to the present invention is a liquid surfactant formulation.

Such liquid surfactant formulations include for example shampoo, shower gel, micellar water, liquid soap, cleansing preparations.

If the cosmetic or pharmaceutical composition according to the present invention is a liquid surfactant formulation, the surfactant component is present in the cosmetic or pharmaceutical composition in an amount of 1 to 40 % by weight, preferably in an amount of 3 to 30 % by weight, more preferably in an amount of 5 to 25 % by weight, based on the total weight of the composition.

Alternatively, and particularly preferred, the cosmetic or pharmaceutical composition as disclosed herein is an aqueous or aqueous/alcoholic or aqueous/glycolic based solution. The aqueous/alcoholic or aqueous/glycolic based solution comprises an aliphatic alcohol or a glycol in an amount of 0.1 to ≤ 50 % by weight, based on the total weight of the solution. The aliphatic alcohol is preferably selected from the group consisting of ethanol, isopropanol, n-propanol. The glycol is preferably selected from the group consisting of glycerin, propylen glycol, butylen glycol or dipropylen glycol. Preferably, the overall water content in the final composition of such compositions can be ≥ 60 %, more preferably ≥ 70 %, even more preferably ≥ 80 %, and in special cases preferably ≥ 90%. New applications such as those including water wipes have high water content. In a particular variant, the inventive compositions can be used for such wet wipe applications. They may then most preferably contain ≥ 95% water, or even ≥ 98% water.

Such aqueous or aqueous/alcoholic or aqueous/glycolic based solutions include for example deo/antiperspirant roll ons, after shaves, cleansing preparations, anti-acne preparations, or wet wipe solutions.

In one preferred variant, the cosmetic or pharmaceutical composition according to the present invention is in the form of an emulsion as defined herein, advantageously in the form of an oil-in-water (O/W) emulsion comprising an oily phase dispersed in an aqueous phase in the presence of an O/W emulsifier.

In a most preferred variant, the cosmetic or pharmaceutical composition according to the present invention is in the form of an aqueous or aqueous/alcoholic, preferably aqueous/ethanolic, or aqueous/glycolic based solution. Typically, this could be glycerin in water compositions.

The above formulations or compositions are prepared according to usual and known methods.

In a further aspect, the composition according to the present invention is a preparation for cosmetic, i.e. non-therapeutic, use for personal care, skin protection, skin care, scalp protection, scalp care, hair care, nail care, in particular for the prevention and/or treatment of skin aging, wrinkle formation, loss of skin volume, loss of skin elasticity, pigment spots, pigment abnormalities, skin dryness, flaking, greasiness, hypopigmentation and/or hyperpigmentation of the skin; or for animal care.

Examples of personal care are preferably anti-ageing preparations, skin care emulsions, body oils, body lotions, cleansing lotions, face or body balms, after shave balms, after sun balms, deo emulsions, cationic emulsions, body gels, treatment creams, skin protection ointments, moisturizing gels, face and/or body moisturizers, light protective preparations (sunscreens), micellar water, hair spray, colour protection hair care products, skin lightening product, anti-dark spot preparations, etc.

Alternatively, the composition according to the present invention is a preparation for use as a medicament.

The pharmaceutical, in particular dermatological, composition according to the present invention is a preparation for the use in the prevention and treatment of a condition of the skin or mucosa, preferably for the use in the prevention and/or treatment of skin conditions, intolerant or sensitive skin, skin irritation, skin reddening, rosacea, wheals, or pruritus (itching).

In order to be used, the cosmetic or pharmaceutical, in particular dermatological composition, according to the present invention is applied to the skin, hair, scalp and/or nails in an adequate amount in such manner as is customary with cosmetics and dermatological products.

The homecare products according to the present invention are predominantly detergent formulations, usually liquids, powders, sprays, granules or tablets, used to remove dirt, including dust, stains, bad smells and clutter on surfaces or other types of housecleaning or laundry detergent that is added for cleaning laundry or liquid soap. Typical homecare products include all purpose cleaners, dishwashing detergents, hard floor and surface cleaners, glass cleaners, carpet cleaners, oven cleaners, laundry detergents, fabric softeners, laundry scent, scent lotions, car shampoo, rim block gel, car shampoo or furniture polish, all afore-mentioned goods also in encapsulated form or air fresheners.

Surprisingly, the use of mixtures of alkanediols according to the present invention and defined herein in combination with a natural polymer containing cosmetic or pharmaceutical composition or homecare product according to the present invention stabilizes the composition by reducing the microbial load. The cosmetic or pharmaceutical composition or homecare product is microbial stable, and, thus, improved the shelf life of a cosmetic or pharmaceutical composition or homecare product.

Thus, the present invention relates in a further aspect to the use of a mixture comprising at least one first linear alkanediol and one or more second linear alkanediol for reducing the microbial load of a natural polymer, preferably the natural polymer in a cosmetic or pharmaceutical composition or homecare product, wherein the mixture comprising at least one first linear alkanedol and one or more second linear alkanediol is selected from the group consisting of
▪ a mixture comprising 1,2-hexanediol and 2,3-hexanediol;
▪ a mixture comprising 1,2-heptanediol and 2,3-heptanediol;
▪ a mixture comprising 1,2-octanediol and 2,3-octanediol; and
▪ a mixture comprising 1,2-heptanediol and 1,2-nonanediol; and
wherein the at least one natural polymer is selected from the group consisting of guar gum, xanthan gum, carboxymethylcellulose, and mixtures of two or more of the aforesaid natural polymers. In a preferred variant, the alkanediol is a liquid alkanediol.

In addition, due to the above-described advantageous properties of the mixture comprising at least one first linear alkanediol and one or more second linear alkanediol as defined herein, by reducing the microbial load, the amount of an antimicrobial agent in a natural polymer containing cosmetic or pharmaceutical composition or homecare product can be reduced.

Thus, the present invention relates in a further aspect also to the use of a mixture comprising at least one first linear alkanediol and one or more second linear alkanediol as defined herein as an antimicrobial agent replacer, preferably in a natural polymer containing cosmetic or pharmaceutical composition or homecare product, wherein the mixture comprising at least one first linear alkanedol and one or more second linear alkanediol is selected from the group consisting of
▪ a mixture comprising 1,2-hexanediol and 2,3-hexanediol;
▪ a mixture comprising 1,2-heptanediol and 2,3-heptanediol;
▪ a mixture comprising 1,2-octanediol and 2,3-octanediol; and
▪ a mixture comprising 1,2-heptanediol and 1,2-nonanediol; and
wherein the at least one natural polymer is selected from the group consisting of guar gum, xanthan gum, carboxymethylcellulose, and mixtures of two or more of the aforesaid natural polymers.

Finally, the present invention also relates to a method for reducing microbial load of a cosmetic or pharmaceutical composition or homecare product comprising a natural polymer as defined herein. In the method according to the invention a cosmetic or pharmaceutical composition or homecare product, which comprises a natural polymer, is provided. An effective amount of a mixture comprising at least one first linear alkanediol and one or more second linear alkanediol as defined herein is incorporated. In a preferred variant of the method, the incorporated alkanediol is a liquid alkanediol.

The present invention shall now be described in detail with reference to the following examples, which are merely illustrative of the present invention, such that the content of the present invention is not limited by or to the following examples.

### Examples

### Example 1:

The following tests were performed to show the influence of different alkanediols on the microbial load of natural polymers.

A selection of natural polymers as specified in Table 1 was systematically analyzed in combination with different 1,2-alkanediols and 2,3-alkanediols (0.5 %). The polymers were chosen to be representative from different polymer classes:

**Table 1**

| **Ingredient group** | **Product group** | **Product** | **Concentration (%) *** |
|---|---|---|---|
| Gum | Xanthan Gum | Rheocare XGN (BASF) | 0.5 |
| Galactomannan | Cyamopsis Tetragonolobus Gum (Guar Gum) | Polycos- N 75 (Safic Alcan) | 0.5 |
| Carrageenan | Carrageenan | Genuvisco CG-131 (CP-Kelco / Rahn) | 0.5 |
| Starch | Zea Mays (Corn) Starch | Nativacare 5600 (Ingredion / NRC) | 3 |
| Carboxymethylcellulose | Cellulose | Covathick 2009 (Sensient/ Goldmann) | 0.5 |

| | | | |
|---|---|---|---|
| *) concentration in accordance with supplier's recommendation | | | |

A mixture of the natural polymer (concentration as recommended by supplier) with water and 1,2-alkanediol (0.5 %) or 2,3-alkanediol (0.5 %) or a mixture of 1,2-alkanediol and 2,3-alkanediol (0.5 % final concentration) was prepared, as representative of a cold formulation preparation processes, and directly sampled for determination of microbial load. Additionally, the mixtures were stored at 40 °C and again sampled for microbial load after 2 days and 7 days.

The microbial load of the mixtures was determined by plating 1000 µl (or respective dilutions of the material) on an agar plate based on universal bacterial growth medium (Caseinpepton-Sojamehlpepton-Bouillon, Merck 1.05459 + Agar No.1, Oxoid LP0011). The plates were then incubated at 37 °C for 5 days and then photo documented. In parallel, bacterial colonies were counted (CFU - colony forming units per ml).

**Table 2: Bacterial load of mixtures of natural polymers with 1,2-alkanediols determined as colony forming units (CFU per ml) (Comparison)**

| | **Water** | | | **1,2-pentanediol** | | | **1,2-hexanediol** | | | **1,2-heptanediol** | | |
|---|---|---|---|---|---|---|---|---|---|---|---|---|
| **Time point** | **fresh** | **2 days** | **7 days** | **Fresh** | **2 days** | **7 days** | **fresh** | **2 days** | **7 days** | **Fresh** | **2 days** | **7 days** |
| Xanthan Gum | 8 | OV | OV | >249 | OV | OV | 7 | 1 | OV | OV | 2 | 0 |
| Polycos-N 75 | 70 | OV | OV | 13 | OV | 13 | 9 | 19 | 2 | 5 | 0 | 0 |
| Genuvisco | >87 | 1 | 0 | 35 | 2800 | 0 | 3 | OV | 0 | 0 | 0 | 0 |
| Nativacare | 1 | >631 | >1000 | 0 | 27 | >1000 | 1 | 0 | 0 | 0 | 0 | 0 |
| Covathick | 2 | >741 | 530 | 84 | >13 | 4 | 3 | 0 | >13 | 4 | 0 | 0 |

| | | | | | | | | | | | | |
|---|---|---|---|---|---|---|---|---|---|---|---|---|
| OV: overgrowth, no determination of exact CFU possible | | | | | | | | | | | | |

As shown in Table 2, 1,2-heptanediol has superior activity compared to 1,2-pentanediol and 1,2-hexanediol to stabilize natural polymer solutions for storage by removing their microbial load.

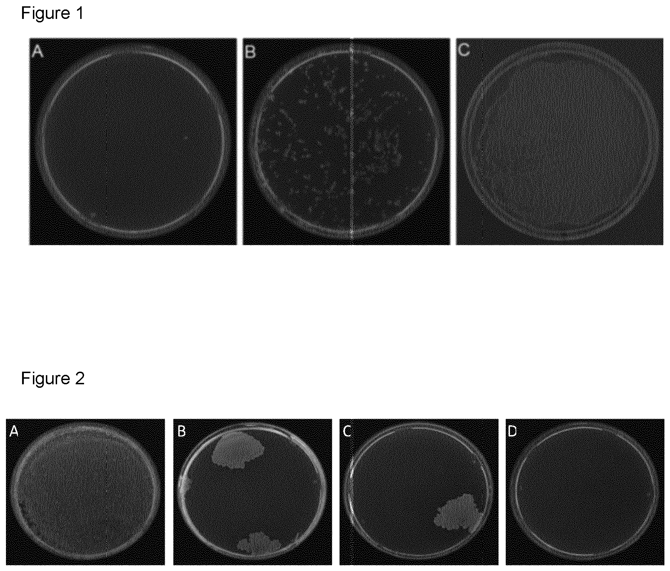

Images of the plates representing the results of Table 2 are shown in Figures 1 and 2:

Figure 1 shows the representative results of microbial load of Nativacare (3 %) in water directly after preparation of the mixture (A), after 2 days storage at 40 °C (B) and after 7 days storage at 40 °C (C).

Figure 2 shows the representative results of microbial load of Polycos-N75 (0.5%) in water (A) or in water with addition of 0.5 % of 1,2-pentanediol (B), 1,2-hexanediol (C) or 1,2-heptanediol (D) respectively after 7 days of storage at 40 °C.

**Table 3: Bacterial load of mixtures of natural polymers with 2,3-alkanediols determined as colony forming units (CFU per mL) (Comparison)**

| | **Wasser** | | | **2,3-heptanediol** | | | **2,3-octanediol** | | | **2,3-undecanediol** | | |
|---|---|---|---|---|---|---|---|---|---|---|---|---|
| **Time point** | **fresh** | **2 days** | **7 days** | **Fresh** | **2 days** | **7 days** | **fresh** | **2 days** | **7 days** | **fresh** | **2 days** | **7 days** |
| Xanthan Gum | OV | OV | OV | OV | OV | OV | OV | 0 | 0 | 0 | 0 | 0 |
| Polycos-N 75 | 29 | OV | OV | 2 | OV | OV | 1 | 0 | 0 | 0 | 0 | 0 |
| Genuvisco | OV | 0 | OV | 5 | 1 | 1 | 5 | 0 | 0 | 0 | 0 | 0 |
| Covathick | 17 | 57 | OV | 5 | 0 | 0 | 4 | 0 | 0 | 0 | 0 | 0 |

| | | | | | | | | | | | | |
|---|---|---|---|---|---|---|---|---|---|---|---|---|
| OV: overgrowth, no determination of exact CFU possible | | | | | | | | | | | | |

As it is obvious from Table 3, the 2,3-alkanediols have a superior effect on the microbial load of the natural polymer solutions. In particular 2,3-octanediol and 2,3-undecanediol show superior antimicrobial activity directly after preparation of the mixtures as well as after 7 days storage.

**Table 4: Bacterial load of mixtures of natural polymers with 1,2-heptanediol, 2,3-heptanediol and mixtures of 1,2-heptanediol and 2,3-heptanediol determined as colony forming units (CFU per mL)**

| | **Water** | | | **1,2-heptanediol (0.5 %)** | | | **2,3-heptanediol (0.5 %)** | | | **1,2-hepanediol (0.25 %) plus 2,3-heptanediol (0.25 %)** | | | **1,2-hepanediol (0.4 %) plus 2,3-heptanediol (0.1 %)** | | |
|---|---|---|---|---|---|---|---|---|---|---|---|---|---|---|---|
| **Time point** | **fresh** | **2 days** | **7 days** | **fresh** | **2 days** | **7 days** | **fresh** | **2 days** | **7 days** | **fresh** | **2 days** | **7 days** | **fresh** | **2 days** | **7 days** |
| Xanthan Gum (0.5 %) | 8 | OV | OV | OV | 2 | 0 | OV | OV | OV | 1 | 1 | 1 | 18 | 0 | 0 |
| Covathic k (0.5 %) | 229 | >741 | 530 | 4 | 0 | 0 | 5 | 0 | 0 | 7 | 0 | 0 | 2 | 0 | 0 |
| PolyCos N-75 (0.5 %) | 70 | OV | OV | 5 | 0 | 0 | 2 | OV | OV | 0 | 0 | 0 | 14 | 0 | 0 |

As it is obvious from Table 4, the homo alkanediol mixture including 1,2-heptanediol (0.25 %) and 2,3-heptanediol (0.25 %) by way of example has an enhanced effect on the microbial load of the natural polymer solutions. In particular, the mixture shows superior antimicrobial activity after 2 days storage of the mixtures as well as after 7 days storage. Compared with the corresponding individual 1,2-heptanediol or 2,3-heptanediol substances, the mixture has a synergistically intensified action.

Likewise, the same effect is shown for a homo alkanediol mixture including 1,2-heptanediol (0.4 %) and 2,3-heptanediol (0.1 %) but having a different alkanediol concentration, compared to the alkanediol mixture above. The mixture reduces the microbial load of the natural polymer solutions although the 2,3-heptanediol is used in a lower concentration. Compared with the corresponding individual 1,2-heptanediol or 2,3-heptanediol substances, the mixture has a synergistically intensified action.

**Table 5: Bacterial load of mixtures of natural polymers with 1,2-heptanediol and 1,2-heptanediol plus 1,2-nonanediol determined as colony forming units (CFU per mL)**

| | **Water** | | | **1,2-heptanediol (0.5 %)** | | | **1,2-heptanediol (0.25 %) plus 1,2-nonanediol (0.25 %)** | | |
|---|---|---|---|---|---|---|---|---|---|
| **Time point** | **fresh** | **2 days** | **7 days** | **fresh** | **2 days** | **7 days** | **fresh** | **2 days** | **7 days** |
| Xanthan Gum (0.5 %) | 8 | OV | OV | OV | 2 | 0 | 0 | 0 | 0 |
| Covathick (0.5 %) | 229 | >741 | 530 | 4 | 0 | 0 | 0 | 0 | 0 |
| PolyCos N-75 (0.5 %) | 70 | OV | OV | 5 | 0 | 0 | 0 | 0 | 0 |

The hetero alkanediol mixture was prepared by solving the 1,2-nonanediol powder in the liquid 1,2-heptanediol, thereafter the respective natural polymer as described above was added to the alkanediol mixture.

As can be seen from Table 5, the hetero alkanediol mixture including 1,2-heptanediol (0.25 %) and 1,2-nonanediol (0.25 %) by way of example has an enhanced effect on the microbial load of the natural polymer solutions. In particular, the mixture shows a very strong antimicrobial activity after 2 days storage of the mixture as well as after 7 days storage. Compared with the corresponding individual 1,2-heptanediol substance, the mixture has a synergistically intensified action.

### Example 2: Solubility of alkanediols in water

For the determination of the maximum water solubility of an alkanediol, water and different concentrations of the respective alkanediol were blended by stirring. The assessment of solubility was carried out at ambient temperature after 24 h storage at 5 °C. The determination of whether the alkanediol has dissolved in water is based entirely on visual observation. An alkanediol compound has dissolved if the mixture is clear and shows no signs of cloudiness or precipitation.

**Table 6:**

| **Tradename/ Chemical name** | **INCI** | **Maximum solubility in water (% by weight)** |
|---|---|---|
| Hydrolite^{®} 5 green | Pentylene Glycol | 50 |
| Hydrolite^{®} 6 | Hexanediol | 50 |
| 1,2-heptanediol | Heptanediol | 2.5 |
| Hydrolite^{®} 8 | Caprylyl Glycol | 0.5 |
| SymDiol^{®} 68 | Hexanediol, Caprylyl Glycol | 1.0 |
| 1,2-nonanediol | Nonanediol | 0.1 |
| SymClariol^{®} | Decylene Glycol | - |
| 1,2-undecanediol | Undecanediol | - |
| 2,3-heptanediol | - | 3.6 |
| 2,3-octanediol | - | 1.5 |
| 2,3-nonanediol | - | 0.3 |
| 2,3-undecanediol | - | - |

### Example 3:

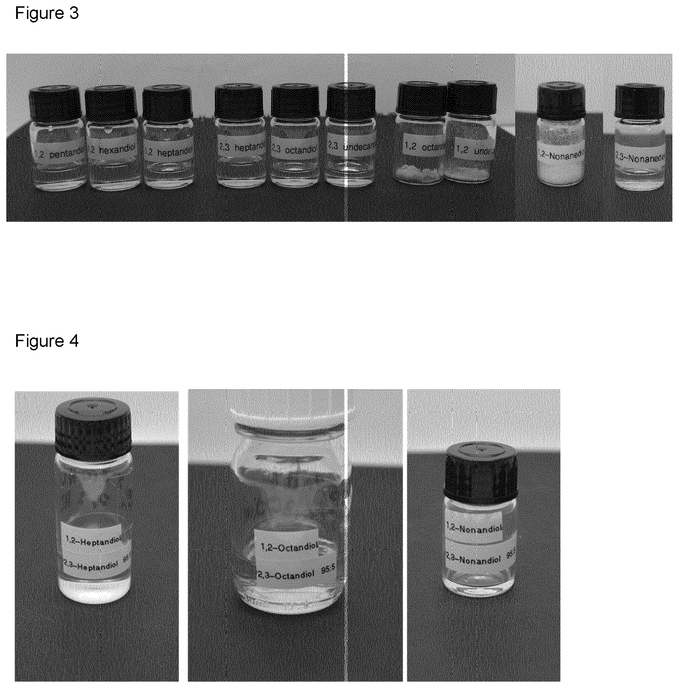

Figure 3 is a photograph showing from left to right samples of the liquid 1,2-pentanediol, 1,2-hexanediol, 1,2-heptanediol, 2,3-heptanediol, 2,3-octanediol and 2,3-undecanediol in comparison to the solid 1,2-octanediol, 1,2-undecanediol, 1,2-nonanediol and the liquid 2,3-nonanediol. The first six alkanediols are liquid at RTP, whereas 1,2-octanediol, 1,2-nonanediol and 1,2-undecanediol are solid substances at RTP; 2,3-nonanediol is also liquid at RTP.

Figure 4 is a representative photograph of samples of mixtures of 1,2-alkanediols with the respective 2,3-alkanediol. All mixtures are liquid. They are shown in the following order: 1,2-heptanediol (95 %) + 2,3-heptanediol (5 %), 1,2-octanediol (95 %) + 2,3-octanediol (5 %), and 1,2-nonanediol (95 %) + 2,3-nonanediol (5 %).

### Example 4: Formulation examples

In the following formulations examples the following five perfume oils PFO1, PFO2, PFO3, PFO4 or PFO5 were each used as fragrance.

**Table 7: Composition of perfume oil 1; PO1 (amounts in ‰ by weight)**

| **Ingredients** | **Amount** |
|---|---|
| ALDEHYDE C14 SO-CALLED | 2 |
| ALLYL AMYL GLYCOLATE 10% DPG | 5 |
| ANISIC ALDEHYDE PURE | 5 |
| APPLE OLIFFAC TYPE | 10 |
| BENZYLACETATE | 50 |
| BERGAMOT IDENTOIL^{®} COLOURLESS | 15 |
| CANTHOXAL | 5 |
| CETALOX 10% IPM | 3 |
| CITRONELLOL 950 | 40 |
| DAMASCENONE TOTAL 1% DPG | 5 |
| DAMASCONE ALPHA 10% DPG | 5 |
| DAMASCONE DELTA 10% DPG | 2 |
| DIMETHYL BENZYL CARBINYL BUTYRATE | 2 |
| DIPROPYLENE GLYCOL | 178 |
| EBANOL | 2 |
| ETHYL DECADIENOATE TRANS CIS-2,4 10% IPM | 2 |
| FLOROSA | 5 |
| FRAMBINON^{®} 10% DPG | 7 |
| GALAXOLIDE 50% IN IPM | 100 |
| GALBEX TYPE BASE | 1 |
| GERANYL ACETATE PURE | 2 |
| HEDIONE | 30 |
| HELIOTROPIN | 10 |
| HEXENYL ACETATE CIS-3 10% DPG | 1 |
| HEXENYL SALICYLATE CIS-3 | 5 |
| HEXYL CINNAMIC ALDEHYDE ALPHA | 70 |
| HEXYL SALICYLATE | 50 |
| HYDROXY CITRONELLAL | 10 |
| ISO E SUPER | 15 |
| ISORALDEINE 70 | 20 |
| LEAFOVERT^{®} | 1 |
| LILIAL | 60 |
| LINALOOL | 60 |
| LINALYL ACETATE | 20 |
| LYRAL | 7 |
| MANZANATE | 2 |
| PHENOXANOL | 7 |
| PHENYLETHYL ALCOHOL | 120 |
| SANDAL MYSORE CORE | 2 |
| SANDRANOL^{®} | 7 |
| STYRALYL ACETATE | 3 |
| TAGETES RCO 10% TEC | 2 |
| TERPINEOL PURE | 20 |
| TETRAHYDROGERANIOL 10% DPG | 5 |
| TONALIDE | 7 |
| VERTOCITRAL 10% DPG | 5 |
| VERTOFIX | 15 |
| **Total:** | **1000** |

**Table 8: Composition of perfume oil 2; PO2 (amounts in % by weight)**

| **Ingredients** | **Amount** |
|---|---|
| Acetophenone, 10% in DPG | 10 |
| n-Undecanal | 5 |
| Aldehyde C14, so-called (peach aldehyde) | 15 |
| Allylamyl glycolate, 10% in DPG | 20 |
| Amyl salicylate | 25 |
| Benzyl acetate | 60 |
| Citronellol | 80 |
| d-Limonene | 50 |
| Decenol trans-9 | 15 |
| Dihydromyrcenol | 50 |
| Dimethylbenzylcarbinyl acetate | 30 |
| Diphenyloxide | 5 |
| Eucalyptol | 10 |
| Geraniol | 40 |
| Nerol | 20 |
| Geranium oil | 15 |
| Hexenol cis-3, 10% in DPG | 5 |
| Hexenyl salicylate cis-3 | 20 |
| Indole, 10% in DPG | 10 |
| Alpha-ionone | 15 |
| Beta-ionone | 5 |
| Lilial^{®} (2-methyl-3-(4-tert-butyl-phenyl)propanal) | 60 |
| Linalool | 40 |
| Methylphenyl acetate | 10 |
| Phenylethyl alcohol | 275 |
| Styrolyl acetate | 20 |
| Terpineol | 30 |
| Tetrahydrolinalool | 50 |
| Cinnamyl alcohol | 10 |
| **Total:** | **1000** |

**Table 9: Composition of perfume oil 3; PO3 (amounts in % by weight)**

| **Ingredients** | **Amount** |
|---|---|
| Benzyl acetate | 60 |
| Citronellyl acetate | 60 |
| Cyclamenaldehyde (2-methyl-3-(4-isopropylphenyl)propanal | 20 |
| Dipropylene glycol (DPG) | 60 |
| Ethyllinalool | 40 |
| Florol (2-isobutyl-4-methyltetrahydro-2H-pyran-4-ol) | 30 |
| Globanone^{®} [(E/Z)-8-cyclohexadecen-1-one] | 180 |
| Hedione^{®} (methyldihydrojasmonate) | 140 |
| Hexenyl salicylate, cis-3 | 10 |
| Vertocitral (2,4-dimethyl-3-cyclohexenecarboxaldehyde) | 5 |
| Hydratropaldehyde, 10% in DPG | 5 |
| Isodamascone (1-(2,4,4-trimethyl-2-cyclohexen-1-yl)-2-buten-1-one, 10% in DPG | 5 |
| Isomuscone (cyclohexadecanone) | 40 |
| Jacinthaflor (2-methyl-4-phenyl-1,3-dioxolane) | 10 |
| Cis-jasmone, 10% in DPG | 20 |
| Linalool | 50 |
| Linalyl acetate | 30 |
| Methyl benzoate, 10% in DPG | 25 |
| para-Methyl cresol, 10% in DPG | 10 |
| Nerol | 20 |
| Phenylpropylaldehyde | 5 |
| 2-Phenylethyl alcohol | 82 |
| Tetrahydrogeraniol | 13 |
| 2,2-Dimethyl-3-cyclohexyl-1-propanol | 80 |
| **Total:** | **1000** |

**Table 10: Composition of perfume oil 4; PO4 (amounts in % by weight)**

| **Ingredients** | **Amount** |
|---|---|
| AMBRETTOLIDE (MACRO) | 10 |
| AMBROXIDE 10% in IPM | 10 |
| BENZYL ACETATE | 20 |
| BENZYL SALICYLATE | 15 |
| BERGAMOT OIL. bergapten-free | 60 |
| CALONE^{®} 1951 10% in DPG | 15 |
| COUMARIN | 5 |
| CYCLOGALBANATE^{®} 10% in DPG | 10 |
| ALPHA -DAMASCONE 1% in DPG | 20 |
| DIHYDROMYRCENOL | 10 |
| ETHYL LINALOOL | 75 |
| ETHYL LINALYLACETATE | 50 |
| ETHYL MALTOL 1% in DEP | 10 |
| ETHYLENE BRASSYLATE (MACRO) | 80 |
| FLOROSA | 40 |
| GERANYLACETATE | 10 |
| HEDIONE^{®} HC/30 | 35 |
| HEDIONE^{®} | 210 |
| HELIONAL^{®} | 15 |
| HELVETOLIDE^{®} (ALICYC) | 30 |
| HEXENYLSALICYLATE CIS-3 | 20 |
| ISO E SUPER^{®} | 40 |
| LEAFOVERT^{®} 10% in DEP | 10 |
| LILIAL^{®} | 80 |
| LYRAL^{®} | 20 |
| MANDARIN OIL | 10 |
| STYRALYL ACETATE | 5 |
| SYMROSE^{®} | 15 |
| VANILLIN 10% in DEP | 20 |
| DIPROPYLENE GLYCOL (DPG) | 50 |
| **Total:** | **1000** |

**Table 11: Composition of perfume oil 5; PO5 (amounts in % by weight)**

| **Ingredients** | **Amount** |
|---|---|
| AMAROCITE^{®} | 10 |
| AMBROCENIDE^{®} 10% in DPG | 5 |
| AMBROXIDE | 15 |
| AURELIONE^{®} (7/8-Cyclohexadecenone) (MACRO) | 70 |
| BERGAMOT OIL. bergapten-free | 90 |
| CALONE^{®} 1951 10% in DPG | 20 |
| CARAWAY OIL | 10 |
| CITRAL | 20 |
| COUMARIN | 10 |
| ALPHA-DAMASCONE 1% in DPG | 15 |
| DIHYDROMYRCENOL | 70 |
| ESTRAGON OIL | 10 |
| ETHYL LINALOOL | 100 |
| ETHYL LINALYLACETATE | 90 |
| EUGENOL | 10 |
| EVERNYL^{®} | 5 |
| FRUCTATE^{®} | 5 |
| GERANIUM OIL | 5 |
| HEDIONE^{®} HC/30 | 100 |
| HELIONAL^{®} | 10 |
| INDOLE 10% in DPG | 5 |
| ISO E SUPER^{®} | 100 |
| KEPHALIS^{®} | 5 |
| LAVENDER OIL | 40 |
| CITRUS OIL | 80 |
| LILIAL^{®} | 30 |
| MANDARIN OIL | 20 |
| MUSCENONE (MACRO) | 5 |
| SANDRANOL^{®} | 10 |
| VANILLIN 10% in DPG | 5 |
| DIPROPYLENE GLYCOL | 30 |
| **Total:** | **1000** |

The above perfume oils PO1, PO2, PO3, PO4, or PO5 were incorporated into the formulations presented below.

Cosmetic formulations (compositions) - amounts are indicated as % by weight for all formulations.

**Table 12: Deodorant Roll-on (not in accordance with the present invention)**

| **Ingredients** | **EU INCI** | **Amount** |
|---|---|---|
| Aqua/ Water | Aqua | ad 100 |
| Hydrolite^{®} CG | Caprylyl Glycol | 0.3 |
| 1,2-heptanediol | 1,2-heptanediol | 0.5 |
| SymSave^{®} H | Hydroxyacetophenone | 0.5 |
| EDTA BD | Disodium EDTA | 0.1 |
| Dracorin^{®} GOC | Glyceryl oleate citrate; Caprylic/Capric triglyceride | 4.0 |
| Dragoxat^{®} 89 | Ethylhexyl isononanoate | 5.0 |
| SymMollient^{®} S green | Cetearyl Nonanoate | 1.0 |
| Genuvisco^{®} CG 131 | Carrageenan | 0.5 |
| Potassium Sorbate | Potassium Sorbate | 0.5 |
| Farnesol | Farnesol | 0.3 |
| Perfume oil PO1, PO2, PO3, PO4, or PO5 | Perfume | 1.0 |
| SymGuard^{®} CD | Phenylpropanol, o-Cymen-5-ol, Decylene Glycol | 0.3 |
| Sodium Hydroxide, 50% solution | Aqua; Sodium hydroxide | 0.2 |

**Table13: Antiperspirant/deodorant roll-on (not in accordance with the present invention)**

| **Ingredients** | **INCI** | **Amount** |
|---|---|---|
| SymLite^{®} G8 | Glyceryl caprylate | 0.15 |
| Dragosantol^{®} 100 | Bisabolol | 0.1 |
| 1,2-heptanediol / 2,3-heptanediol (95:5) | 1,2-heptanediol, 2,3-heptanediol | 0.5 |
| Ethanol 96 % | Ethanol | 30.0 |
| Farnesol | Farnesol | 0.5 |
| Perfume oil PO1, PO2, PO3, PO4, or PO5 | Perfume | 1.5 |
| Frescolat^{®}ML cryst. | Menthyl Lactate | 0.2 |
| Irgasan^{®} DP 300 | Triclosan | 0.3 |
| Natrosol^{™} 250 HHR | Hydroxyethyl-cellulose | 0.3 |
| Solubilizer | PEG-40 Hydrogenated Castor Oil, Trideceth-9, Water (Aqua) | 2.0 |
| SymDeo^{®} B125 | 2-Methyl 5-Cyclohexylpentanol | 0.5 |
| Water (demineralized) | Water (Aqua) | ad 100 |
| Zirkonal L 450 | Aluminium Zirconium Pentachlorohydrate (40 % aqueous solution) | 37.0 |

**Table14: Deodorant formulation in the form of a roll-on gel (not in accordance with the present invention)**

| **Ingredients** | **INCI** | **Amount** |
|---|---|---|
| 1,3-butylene glycol | 1,3-butylene glycol | 2.0 |
| Cremophor ^{®} CO 40 Surfactant | PEG-40-hydrogenated castor oil | 2.0 |
| Hydroxyethylcellulose | Hydroxyethylcellulose | 0.5 |
| Perfume oil PO1, PO2, PO3, PO4, or PO5 | Perfume | 0.3 |
| 1,3-propanediol | | 0.5 |
| SymGuard^{®} CD | 3-Phenylpropanol, o-cymen-3-ol, Decylene glycol | 0.4 |
| Ethylhexyl glycerin | Ethylhexyl glycerin | 0.1 |
| Hydrolite-8 | 1,2-octanediol | 0.50 |
| 1,2-heptanediol / 2,3-heptanediol blend (95-5 w/w%) | 1,2-heptanediol, 2,3-heptanediol | 0.5 |
| SymLite^{®} G8 | Glyceryl Caprylate | 0.15 |
| Water | | ad 100 |

**Table 15: Clear deo anti-perspirant roll-on (not in accordance with the present invention)**

| **Ingredients** | **INCI** | Amount |
|---|---|---|
| Methocel^{™} E4M Premium | Hydroxypropyl Methylcellulose | 0.5 |
| Water | Water (Aqua) | ad 100 |
| Neo-PCL Water Soluble N | Trideceth-9, PEG-5 Ethylhexanoate, Water (Aqua) | 1.0 |
| Solubilizer | PEG-40 Hydrogenated Castor Oil, Trideceth-9, Propylene Glycol, Water (Aqua) | 3.0 |
| Deolite | Dimethyl Phenylpropanol, Pentylene Glycol | 0.5 |
| Locron^{®} LW | Aluminium Chlorohydrate | 25.0 |
| Aloe Vera Gel Concentrate 10/1 | Aloe Barbadensis Leaf Juice | 1.0 |
| 1,2-Propylene Glycol 99 P GC | Propylene Glycol | 4.0 |
| Ethanol 96 % | Alcohol Denat. | 30.0 |
| Perfume oil PO1, PO2, PO3, PO4, or PO5 | Perfume | 1.0 |
| 1,2-heptanediol, 2,3-heptanediol (w/w% 95:5) | 1,2-heptanediol, 2,3-heptanediol | 0.95 |
| 2,3-octanediol | 2,3-octanediol | 0.05 |

**Table 16: Hydratin Face Gel, water clear**

| **Ingredients** | **EU INCI** | **Amount** |
|---|---|---|
| Aqua/Water | Aqua | ad 100 |
| Gamma-Max | Sodium Polyglutamate | 0.1 |
| Glycerin 99.5% | Glycerin | 3.0 |
| Genu^{®} Gum | Gellan Gum | 1.0 |
| 1,2-heptanediol, 2,3-heptanediol 95:5 | 1,2-heptanediol, 2,3-heptanediol | 1.0 |
| SymOcide^{®} PS | Phenoxyethanol, Decylene Glycol, 1,2 Hexanediol | 0.6 |
| SymHair^{®} Force 1631 | Pentylene Glycol, Isochrysis Galbana Extract | 2.5 |
| SymHair^{®} Shape&Color | Cetearyl Nonanoate, Triticum Vulgare (Wheat) Germ Oil, Caprylic/Capric Triglyceride, Linoleic Acid, Triticum Vulgare (Wheat) Bran Extract, Triticum Vulgare (Wheat) Germ Extract, Camellia Oleifera Seed Oil | 0.5 |

**Tabelle 17: Intimate Sensi-Gel (not in accordance with the present invention)**

| **Ingredients** | **EU INCI** | **Amount** |
|---|---|---|
| Aqua/Water | Aqua | ad 100 |
| Sodium Benzoate | Sodium benzoate | 0.3 |
| Keltrol^{®} CG-T | Xanthan Gum | 0.2 |
| Cyamopsis Tetragonolobus Gum | Cyamopsis Tetragonolobus Gum | 0.1 |
| Glycerin | Glycerin | 4.0 |
| Tego^{®} Betain F50 | Cocamidopropyl betaine | 14.0 |
| Solubilizer | PEG-40 Hydrogenated castor oil, Trideceth-9, Propylene Glycol, Aqua | 3.0 |
| 1,2-heptanediol | 1,2-heptanediol | 0.5 |
| Hydrolite^{®} 6 | 1,2 Hexanediol | 1.0 |
| SymGuard^{®} CD | Phenylpropanol, o-cymen-5-ol, Decylene Glycol | 0.3 |
| SymRelief^{®} 100 | Bisabolol, Zingiber officinale root extract | 0.1 |
| Perfume oil PO1, PO2, PO3, PO4, or PO5 | Perfume | 0.2 |
| Frescolat^{®} X-cool | Menthyl ethylamido oxalate | 0.4 |
| Plantacare^{®} 2000 UP | Decyl Glucoside | 2.0 |
| Lactic acid 90% Nat. | Lactic Acid, Aqua | 0.3 |

**Table 18: Mild feminine wash**

| **Ingredients** | **EU INCI** | **Amount** |
|---|---|---|
| Aqua/Water | Aqua | ad 100 |
| SymSave^{®} H | Hydroxyacetophenone | 0.5 |
| Keltrol^{®} CG-T | Xanthan Gum | 0.5 |
| Glycerin | Glycerin | 2.0 |
| Propylene Glycol | Propylene Glycol | 3.0 |
| 1,2-heptanediol | 1,2-heptanediol | 0.5 |
| 2,3-Heptanediol | 2,3-Heptanediol | 0.03 |
| Tego^{®} Betain F50 | Cocamidopropyl betaine | 14.0 |
| SymGuard^{®} CD | Phenylpropanol, o-cymen-5-ol, Decylene Glycol | 0.3 |
| Plantacare 2000 UP | Decyl Glucoside | 2.0 |
| Extrapone^{®} Witch Hazel GW | Glycerin, Aqua, Hamamelis Virginiana Bark/-Leaf/Twiq Extract | 1.0 |
| Solubilizer | PEG-40 Hydrogenated castor oil, Trideceth-9, Propylene Glycol, Aqua | 1.5 |
| Perfume oil PO1, PO2, PO3, PO4, or PO5 | Perfume | 0.2 |
| SymCalmin^{®} | Pentylene Glycol, Butylene Blycol, Hydroxyphenyl propamidobenzoic acid | 1.0 |
| Lactic acid 90% Nat. | Lactic Acid, Aqua | 0.32 |

**Table 19: Intimate Rescue Gel (not in accordance with the present invention)**

| **Ingredients** | **EU INCI** | **Amount** |
|---|---|---|
| Aqua/Water | Aqua | ad 100 |
| SymSave^{®} H | Hydroxyacetophenone | 0.3 |
| Keltrol^{®} CG-T | Xanthan Gum | 0.3 |
| Genuvisco^{®} CG-131 | Carrageenan | 0.2 |
| Glycerin | Glycerin | 4.0 |
| Tego^{®} Betain F50 | Cocamidopropyl betaine | 14.0 |
| Sodium Hydroxide 10% solution | Aqua, Sodium hydroxide | 1.0 |
| Solubilizer | PEG-40 Hydrogenated castor oil, Trideceth-9, Propylene Glycol, Aqua | 3.0 |
| Hydrolite^{®} 6 | 1,2-Hexanediol | 1.0 |
| 1,2-heptanediol, 2,3-heptanediol (w/w% 95:5) | 1,2-heptanediol | 0.5 |
| 2,3 Octanediol | 2,3 Octanediol | 0.03 |
| SymGuard^{®} CD | Phenylpropanol, o-cymen-5-ol, Decylene Glycol | 0.3 |
| SymCalmin^{®} | Pentylene Glycol, Butylene Blycol, Hydroxyphenyl propamidobenzoic acid | 1.0 |
| Perfume oil PO1, PO2, PO3, PO4, or PO5 | Perfume | 0.2 |
| Frescolat^{®} MGA Plus | Menthone Glycerin Acetal, Menthol | 0.5 |
| Plantacare 2000 UP | Decyl Glucoside | 2.0 |

**Table 20: After Sun Refreshing Gel**

| **Ingredients** | **EU INCI** | **Amount** |
|---|---|---|
| Aqua/Water | Aqua | ad 100 |
| Hydrolite^{®} 5 green | Pentylene Glycol | 2.5 |
| SymSave^{®} H | Hydroxyacetophenone | 0.5 |
| Panthenol 75W | Panthenol | 0.5 |
| 1,3 Butylene Glycol | Butylene Glycol | 3.0 |
| Glycerin 99,5% | Glycerin | 2.0 |
| DragoCalm^{®} | Aqua, Glycerin, Avena sativa kernel extract | 1.5 |
| SymGlucan^{®} | Aqua, Glycerin, 1,2-Hexanediol, Caprylyl Glycol, Beta-Glucan | 5.0 |
| Dragosine^{®} | Carnosine | 0.1 |
| Nativacare^{™} 5600 | Zea (Mays) Corn Starch | 2.0 |
| Guar Gum | Guar Gum | 0.1 |
| Rheocare^{®} XGN | Xanthan Gum | 0.1 |
| Solubilizer | PEG-40 Hydrogenated castor oil, Trideceth-9, Propylene Glycol, Aqua | 1.5 |
| SymMollient^{®} W/S | Trideceth-9, PEG-5 Isononanoate, Aqua | 1.0 |
| SymBright^{®} 2036 | Sclareolide | 0.1 |
| SymSitive^{®} 1609 | Pentylene Glycol, 4-t-Butylcyclohexanol | 1.0 |
| SymUrban^{®} | Benzylidene Dimethoxydimethylidanone | 0.15 |
| 1,2-heptanediol, 2,3-heptanediol (w/w% 95:5) | 1,2-heptanediol, 2,3-heptanediol | 1.0 |
| Frescolat^{®} ML Cryst | Menthyl Lactate | 0.3 |
| Perfume oil PO1, PO2, PO3, PO4, or PO5 | Perfume | 0.05 |
| Sodium Hydroxide 10% solution | Aqua, Sodium hydroxide | 0.8 |
| Colour | | 0.4 |
| Extrapone^{®} Cucumber CL | Aqua, Propylene Glycol, cucumis sativus (Cucumber) Juice | 1.0 |
| SymFinity^{®} 1298 | Echinacea Purpurea Extract | 0.06 |

**Table 21: After Shave Fluid**

| **Ingredients** | **EU INCI** | **Amount** |
|---|---|---|
| Water demin | Aqua | ad 100 |
| Glycerin | Glycerin | 3.00 |
| 1,2-heptanediol, 2,3-heptanediol (95:5) | 1,2-heptanediol, 2,3-heptanediol | 0.5 |
| 2,3-octanediol | 2,3-octanediol | 0.01 |
| Dermosoft^{®} 1388 | Water, Glycerin, Sodium Levulinate, Sodium Anisate | 3.00 |
| Tego^{®} Care CG90 | Cetearyl Glucoside | 1.00 |
| Covathick 2009 | Microcrystalline Cellulose, Cellulose Gum | 0.50 |
| Cutina^{®} GMS V | Glyceryl Stearate | 1.00 |
| Coconut Oil refined, organic | Cocos Nucifera (Coconut) Oil | 10.00 |
| Dermofeel ^{®}BGC | Butylene Glycol Dicaprylate/Dicarate | 5.00 |
| Keltrol^{®} CG-SFT | Xanthan Gum | 0.30 |
| Genuvisco^{®} CG-131 | Carrageenan | 0.30 |
| Citric acid solution 10% | Water, citric acid | 1.70 |
| Ethanol | Alcohol | 5.00 |
| Perfume oil PO1, PO2, PO3, PO4, or PO5 | Perfume | 0.50 |
| Dragosantol^{®} 100 | Bisabolol | 0.2 |
| Frescolat^{®} X-cool | Menthyl Ethylamido oxalate | 1.0 |
| SymCalmin^{®} Green | Butylene Glycol, Pentylene Glycol, Hydroxyphenyl Propamidobenzoic acid | 1.0 |

**Table 22: Wild Berries Shower Polish**

| **Ingredients** | **EU INCI** | **Amount** |
|---|---|---|
| Aqua | Aqua | ad 100 |
| Glycerin 99.5% | Glycerin | 3.0 |
| Genuvisco^{®} CG-131 | Carrageenan | 0.7 |
| Keltrol^{®} CG-SFT | Xanthan Gum | 0.3 |
| Amisoft CS-22 | Sodium Cocoyl Glutamate, Disodium Cocoyl Glutamate | 20.0 |
| Eucarol AGE/EC | Disodium Coco-Glucoside Citrate | 7.0 |
| SymOcide PS | Phenoxyethanol, Decylene Glycol, 1,2 Hexanediol | 1.0 |
| 1,2-heptanediol, 2,3-heptanediol blend (w/w% 95:5) | 1,2-heptanediol, 2,3-heptanediol | 1.0 |
| 1,2-octanediol, 2,3-octanediol blend (w/w% 99:1) | 1,2-octanediol, 2,3-octanediol | 0,3 |
| Jojoba oil | Simmondsia Chinensis (Jojoba) Seed Oil | 8.0 |
| SymControl^{®} Scalp | Water (Aqua), Glycerin, Mannitol, Tetraselmis Suecica Extract | 2.0 |
| Actipone^{®} Goji Berry GW | Water (Aqua), Glycerin, Lycium Barbarum Fruit Extract | 2.0 |
| Vitacel^{®} CS250g | Cellulose | 2.0 |

**Table 23: All in one bar - Shampoo-body-beard (not in accordance with the present invention)**

| **Ingredients** | **EU INCI** | **Amount** |
|---|---|---|
| Tego^{®} Alkanol 1618 | Cetearyl Alcohol | 29.3 |
| Tego^{®} Feel Green | Cellulose | 1.0 |
| Sisterna A10E-C | Sucrose Tetrastearate Triacetate | 2.5 |
| HydraSynol^{™} DOI | Isosorbide Dicaprylate | 2.0 |
| Coconutoil | Cocos Nucifera (Coconut) Oil | 3.3 |
| Cacao Butter | Theobroma Cacao (Cocoa) Seed Butter | 2.0 |
| Dehymuls^{®} SMO | Sorbitan Oleate | 0.2 |
| Passion Fruit oil | Passiflora Edulis Seed Oil | 7.0 |
| Isoamyl Laurate | Isoamyl Laurate | 1.0 |
| SymLite^{®} G8 | Glyceryl Caprylate | 0.5 |
| 1,2-heptanediol | 1,2-heptanediol | 1.0 |
| Tocopherol | Tocopherol | 0.2 |
| Amisoft^{®} GS-11P | Sodium Stearoyl Glutamate, Sodium Cocoyl Glutamate | 20.0 |
| Hostapon^{®} SCI-85 | Sodium Cocoyl Isethionate | 30.0 |
| Perfume oil PO1, PO2, PO3, PO4, or PO5 | Perfume | 1.0 |

**Table 24: All Natural Shower Jelly (not in accordance with the present invention)**

| **Ingredients** | **EU INCI** | **Amount** |
|---|---|---|
| Water demin | Aqua | ad 100 |
| Glycerin 85% | Glycerin, Aqua | 25.0 |
| Dermosoft^{®} 1388 ECO | Aqua, Glycerin, Sodium Levulinate, Sodium Anisate | 3.0 |
| SymMollient^{®} S green | Cetearyl Nonanoate | 1.5 |
| Crinipan^{®} PMC green | Propanediol Caprylate | 0.5 |
| 1,2-heptanediol, 2,3-heptanediol blend (w/w% 95:5) | 1,2-heptanediol, 2,3-heptanediol | 0.5 |
| Citric Acid Solution 10% | Aqua, Citric acid | 9.0 |
| Genuvisco^{®} CG-131 | Carrageenan | 2.0 |
| Perfume oil PO1, PO2, PO3, PO4, or PO5 | Perfume | 0.5 |
| Plantacare^{®} 2000 UP | Decyl Glucoside | 20.0 |
| Amisoft^{®} CS-22 | Sodium Cocoyl Glutamate, Disodium Cocoyl Glutamate, Water | 20.0 |

**Table 25: Aloe Shower Cream**

| **Ingredients** | **EU INCI** | **Amount** |
|---|---|---|
| Water demin. | Water | ad 100 |
| Glycerin 85% | Glycerin, Water | 3.0 |
| Dermosoft ^{®} 1388 ECO | Glycerin, Water, Sodium Levulinate, Sodium Anisate | 3.5 |
| SymLite^{®} G8 | Glyceryl Caprylate | 0.5 |
| 1,2-heptanediol, 2,3-heptanediol (95:5) | 1,2-heptanediol, 2,3-heptanediol | 1.0 |
| Genuvisco^{®} CG-131 | Carrageenan | 0.8 |
| Keltrol^{®} CG-SFT | Xanthan Gum | 0.3 |
| Amisoft^{®} CS-22 | Sodium Cocoyl Glutamate, Disodium Cocoyl Glutamate, Water | 20.0 |
| Plantacare^{®} 2000 UP | Decyl Glucoside | 7.0 |
| Aloe Vera Gel Concentrate | Aloe Barbadensis Leaf Juice | 1.5 |
| SymOleo^{®} Vita 7 | Glycine Soja (Soybean) Oil, Gossypium Herbaceum (Cotton) Seed Oil, Mangifera Indica (Mango) Seed Butter, Olea Europaea (Olive) Fruit Oil, Persea Gratissima (Avocado) Oil, Prunus Amygdalus Dulcis (Sweet Almond) Oil, Theobroma Cacao (Cocoa) Seed Butter | 5.0 |
| Citric acid solution 10% | Aqua, citric acid | 6.7 |
| Perfume oil PO1, PO2, PO3, PO4, or PO5 | Perfume | 0.4 |
| Cosmopheres^{®} GYAM-M | Mannitol, Microcrystalline Cellulose, Aloe Barbadensis Leaf Juice Powder, Chromium Oxides greens, Iron Oxides | 2.0 |

**Table 26: Fresh Hair shampoo**

| **Ingredients** | **EU INCI** | **Amount** |
|---|---|---|
| Aqua | Aqua | ad 100 |
| EDTA B Powder | Disodium EDTA | 0.1 |
| Nativacare^{™} 5600 | Zea Mays (Corn Starch) | 2.0 |
| Rheocare^{®} XGN | Xanthan Gum | 0.5 |
| Texapon^{®} NSO UP | Sodium Laureth sulfate | 35.0 |
| Dehyton^{®} K | Cocamidopropyl betaine | 8.0 |
| Plantacare^{®} 2000 UP | Decyl Glucoside | 4.0 |
| Perfume oil PO1, PO2, PO3, PO4, or PO5 | Perfume | 1.0 |
| SymSave^{®} H | Hydroxyacetophenone | 0.5 |
| Hydrolite^{®} 6 | 1,2-Hexanediol | 1.0 |
| 1,2-heptanediol, 2,3-heptanediol (w/w% 95:5) | 1,2-heptanediol, 2,3-heptanediol | 1.0 |
| 2,3-nonanediol | 2,3-nonanediol | 0.01 |
| SymClariol^{®} | Decylene Glycol | 0.3 |
| Frescolat^{®} ML cryst | Menthyl Lactate | 1.0 |
| SymCalmin^{®} | Pentylene Glycol, Butylene Glycol, Hydroxyphenyl propamidobenzoic acid | 0.2 |
| Crinipan^{®} AD | Climbazole | 0.2 |
| Propylene Glycol | Propylene Glycol | 2.0 |
| SymHair^{®} Shield | Pentylene Glycol, Aqua, Glycerin, Triticum vulgare bran extract, 1,2-Hexanediol, Capryly glycol | 1.0 |
| Sodium Hydroxide 10% solution | Aqua, Sodium hydroxide | 2.2 |
| Actipone^{®} Alpha-Pulp | Aqua, Butylene Glycol, Malic acid, Actinidia chinensis fruit extract, citrus aurantium dulcis juice, citrus paradise juice, pyrus malus juice | 0.2 |

**Table 27: Revitalizing Conditioner (not in accordance with the present invention)**

| **Ingredients** | **EU INCI** | **Amount** |
|---|---|---|
| Aqua | Aqua | ad 100 |
| Covathick 2009 | Microcrystalline Cellulose (and) Cellulose Gum | 0.5 |
| EDETA BD | Disodium EDTA | 0.05 |
| Glycerin 99.5% | Glycerin | 1.00 |
| Lactic Acid | Lactic acid | 0.85 |
| Genamin^{®} KDMP | Behentrimonium chloride | 2.50 |
| SymSave^{®} H | Hydroxyacetophenone | 0.50 |
| Hydrolite^{®} CG | Caprylyl Glycol | 0.50 |
| 1,2-heptanediol | 1,2-heptanediol | 0.50 |
| Tego^{®} Amid S18 | Stearamidopropyl Dimethylamine | 3.00 |
| VARISOFT^{®} EQ65 | Distearoylethyl Dimonium chloride (and) ceterayl alcohol | 4.00 |
| SymMollient^{®} S green | Ceteraryl Nonanoate | 2.00 |
| Cetiol^{®} SB 45 | Butyrospermum parkii butter | 3.00 |
| Lanette^{®} 16 | Cetyl alcohol | 4.0 |
| Lanette^{®} 18 | Stearyl alcohol | 4.0 |
| Beeswax | Beeswax | 0.80 |
| SymOleo^{®} Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 3.00 |
| Eumulgin^{®} B2 | Ceteareth-20 | 1.00 |
| Perfume oil PO1, PO2, PO3, PO4, or PO5 | Perfume | 1.00 |
| SymHair^{®} Restore | Glycerin, Triticum vulgare protein, Aqua | 1.00 |
| Lactic acid | Lactic acid | 0.50 |

**Table 28: Hair Soap Shampoo Bar**

| **Ingredients** | **EU INCI** | **Amount** |
|---|---|---|
| Talco | Talc | ad 100 |
| Vivapur^{®} CS 032 XV | Microcrystaline Cellulose, Xanthan Gum | 0.3 |
| Texapon^{®} ZACD | Sodium Lauryl Sulfate | 10.00 |
| SymOleo^{®} Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 2.00 |
| Cetiol^{®} SB 45 | Butyrospermum parkii butter | 3.00 |
| Mackamide^{®} CMA | Cocamide Mea | 4.00 |
| SymSol^{®} PF-3 | Aqua, Pentylene Glycol, Sodium Lauryl sulfoacetate, sodium oleoyl sarcosinate, Sodium Chloride, Sodium Oleate | 5.00 |
| Food Color Beta Carotene E160A OilSoluble | Helianthus annuus (Sunflower) seed oil, Beta-Carotene (CI40800) | 0.45 |
| 1,2-heptanediol, 2,3-heptanediol blend (w/w% 95:5) | 1,2-heptanediol, 2,3-heptanediol | 1.0 |
| 1,2-octanediol, 2,3-octanediol blend (w/w% 95:5) | 1,2-octanediol, 2,3-octanediol | 0,3 |
| SymOcide^{®} PH | Phenoxyethanol, Hydroxyacetophenone, Caprylyl Glycol, Aqua | 1.00 |
| SymHair^{®} Restore | Glycerin, Triticum vulgare protein, Aqua | 1.00 |
| Perfume oil PO1, PO2, PO3, PO4, or PO5 | Perfume | 1.50 |

**Table 29: Conditioner Spray Breath fresh air (not in accordance with the present invention)**

| **Ingredients** | **EU INCI** | **Amount** |
|---|---|---|
| Aqua/Water | Aqua | ad 100 |
| Rheocare^{®} XGN | Xanthan Gum | 0.05 |
| Edeta^{®} BD | Disodium EDTA | 0.10 |
| Glycerin 99.5% | Glycerin | 1.00 |
| SymSave^{®} H | Hydroxyacetophenone | 0.50 |
| Ucare^{™} Polymer JR-400 | Polyquaternium-10 | 0.30 |
| VARISOFT^{®} 300 | Cetrimonium Chloride | 3.50 |
| SymHair^{®} Shield | Pentylene Glycol, Aqua, Glycerin, Triticum vulgare bran extract, 1,2-Hexanediol, Caprylyl Glycol | 1.00 |
| Cremophor^{®} CO 40 Surfactant | PEG-40 Hydrogenated castor oil | 1.00 |
| Perfume oil PO1, PO2, PO3, PO4, or PO5 | Perfume | 0.70 |
| 1,2-heptanediol | 1,2-heptanediol | 0.50 |
| SymHair^{®} Force 1631 | Pentylene Glycol, Isochrysis Galbana extract | 1.00 |
| Citric Acid 50% Sol. | Aqua, Citric acid | 0.10 |

**Table 30: Hair Refresher Mist (not in accordance with the present invention)**

| **Ingredients** | **EU INCI** | **Amount** |
|---|---|---|
| SymDeo^{®} Plus | Lauryl alcohol, Phenoxyethanol, 2-Benzyleptanol, Decylene Glycol | 0.5 |
| Perfume oil PO1, PO2, PO3, PO4, or PO5 | Perfume | 0.1 |
| Frescolat^{®} ML nat | Mentyl Lactate | 0.3 |
| Crinipan^{®} PMC green | Propanediol Caprylate | 0.3 |
| Solubilizer | PEG-40 Hydrogenated Castor oil, Trideceth-9, Propylene glycol, Aqua | 5.0 |
| SymCalmin^{®} | Pentylene Glycol, Butylene Glycol, Hydroxyphenyl Propamidobenzoic acid | 0.2 |
| SymControl^{®} Scalp | Water (Aqua), Glycerin, Mannitol, Tetraselmis Suecica Extract | 2.0 |
| Genuvisco^{®} CG-131 | Carrageenan | 0.2 |
| 1,2-heptanediol, 2,3-heptanediol (w/w% 95:5) | 1,2-heptanediol, 2,3-heptanediol | 0.05 |
| 2,3 Octanediol | 2,3 Octanediol | 0.01 |
| Propylene Glycol | Propylene Glycol | 3.0 |
| Butylene Glycol | Butylene Glycol | 3.0 |
| Aqua | Aqua | ad 100 |
| Edeta^{®} BD | Disodium EDTA | 0.1 |
| Sodium Benzoate | Sodium benzoate | 0.4 |
| Colour | | 0.5 |
| Actipone^{®} Rosemary GW | Glycerin, Aqua, Rosmarinus officinalis leaf extract | 0.5 |

**Table 31: Virgin Mojito Pre-Shampoo**

| **Ingredients** | **EU INCI** | **Amount** |
|---|---|---|
| Aqua/Water | Aqua | ad 100 |
| Nativacare^{™} 5600 | Zea (Mays) Corn Starch | 1.0 |
| Keltrol^{®} CG- SFT | Xanthan Gum | 0.5 |
| Glycerin | Glycerin | 2.0 |
| SymSave^{®} H | Hydroxyacetophenone | 0.5 |
| Hydrolite^{®} 5 green | Pentylene Glycol | 3.0 |
| Solubilizer | PEG-40 Hydrogenated castor oil, Trideceth-9, Propylene Glycol, Aqua | 3.0 |
| Oxynex^{®} ST Liquid | Diethylhexyl Syringylidenemalonate, Caprylic/capric triglyeride | 0.1 |
| Frescolat^{®} Plus | Menthol, Menthyl Lactate | 1.0 |
| Perfume oil PO1, PO2, PO3, PO4, or PO5 | Perfume | 0.5 |
| 2,3-heptanediol | 2,3-heptanediol | 0.5 |
| 1,2-heptanediol | 1,2-heptanediol | 0.5 |
| SymReboot^{™} L19 | Maltodextrin, Lactobacillus ferment | 0.5 |
| Extrapone^{®} Lime | Propylene Glycol, Aqua, Citrus aurantifolia juice | 1.0 |
| Color I: FD&C Yellow no.5 - 0.01% solution | Aqua, CI19140, Sodium Chloride, Sodium Sulfate | 0.8 |
| Color II: FD&C Blue no.1 - 0.01% solution | Aqua, CI42090, Sodium Chloride, Sodium Sulfate | 0.4 |
| Sodium Hydroxide 10% solution | Aqua, Sodium Hydroxide | 0.2 |

**Table 32: Sensi-SCALP (Green Solid Shampoo) (not in accordance with the present invention)**

| **Ingredients** | **EU INCI** | **Amount** |
|---|---|---|
| AMISOFT^{®} MS-11 | Sodium Myristoyl Glutamate | 30.0 |
| ELFAN^{®} AT 84 | Sodium cocoyl Isethionate | 15.0 |
| Sensocel^{®} 10 | Cellulose | 3.0 |
| ImerCare^{®} 02K-S | Kaolin | 18.8 |
| Hydrolite^{®} 5 green | Pentylene Glycol | 1.5 |
| 1,2-heptanediol | 1,2-heptanediol | 0.5 |
| Dragosantol^{®} 100 | Bisabolol | 0.1 |
| Potassium Sorbate | Potassium Sorbate | 0.3 |
| Aqua/Water | Aqua | ad 100 |
| SymDecanox^{™} HA | Caprylic/Capric Triglyceride, Hydroxymethoxyphenyl decanone | 2.0 |
| Cetiol^{®} SB45 | Butyrospermum parkii butter | 13.0 |
| Extrapone^{®} Rooibus GW | Aqua, Glycerin, Aspalathus Linearis leaf extract | 1.0 |
| SymReboot^{™} L19 | Maltodextrin, Lactobacillus Ferment | 0.5 |
| SymOleo^{®} Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 1.0 |
| Perfume oil PO1, PO2, PO3, PO4, or PO5 | Perfume | 1.2 |

**Table 33: Hair conditioner, leave on**

| **Ingredients** | **INCI** | **Amount** |
|---|---|---|
| 1,2-heptanediol, 2,3-heptanediol blend (w/w% 95:5) | 1,2-heptanediol, 2,3-heptanediol | 0.5 |
| 2,3-octanediol | 2,3-octanediol | 0.05 |
| Rheocare^{®} XGN | Xanthan Gum | 0.1 |
| KelcoGel^{®} CG-HA | Gellan Gum | 0.1 |
| Dehyquart^{®} A CA | Cetrimonium Chloride | 0.2 |
| Dehyquart^{®} SP | Quaternium-52 | 2.0 |
| Dracorin^{®} CE | Glyceryl Stearate Citrate | 1.0 |
| Drago-Calm^{®} | Water, Glycerin, Avena Sativa (Oat) Kernel Extract | 2.0 |
| Farnesol | Farnesol | |
| Perfume oil PO1, PO2, PO3, PO4, or PO5 | Perfume | 0.5 |
| Lara Care^{®} A-200 | Galactoarabinan | 0.1 |
| Ucare^{™} Polymer JR 400 | Polyquaternium-10 | 0.1 |
| Propylene Glycol | Propylene Glycol | 0.8 |
| SymMollient^{®} WS | Trideceth-9, PEG-5 Isononanoate, Water | 1.0 |
| SymSol^{®}PF3 | Water, Pentylene Glycol, Sodium Lauryl Sulfoacetate, Sodium Oleoyl Sarcosinate, Sodium Chloride, Disodium Sulfoacetate, Sodium Oleate, Sodium Sulfate | 1.5 |
| SymTriol^{®} | Caprylyl Glycol, 1,2-Hexanediol, Methylbenzyl Alcohol | 1.0 |
| Water (demineralized) | Water (Aqua) | ad 100 |

**Table 34: Anti-itch hair conditioner, leave on (not in accordance with the present invention)**

| **Ingredients** | **INCI** | **Amount** |
|---|---|---|
| SymLite^{®} G8 | Glyceryl caprylate | 0.15 |
| 1,2-heptanediol, 2,3-heptanediol blend (w/w% 99:1) | 1,2-heptanediol, 2,3-heptanediol | 0.8 |
| Dragosantol^{®} 100 | Bisabolol | 0.1 |
| Dehyquart^{®} A CA | Cetrimonium Chloride | 0.5 |
| Dehyquart^{®} SP | Quaternium-52 | 4.0 |
| Dracorin^{®} CE | Glyceryl Stearate Citrate | 1.0 |
| Drago-Oat-Active | Water (Aqua), Butylene Glycol, Avena Sativa (Oat) Kernel Extract | 2.0 |
| Perfume oil PO1, PO2, PO3, PO4, or PO5 | Perfume | 0.1 |
| Nativacare^{™} 5600 | Zea (Mays) Corn Starch | 1.5 |
| Lara Care^{®} A-200 | Galactoarabinan | 1.5 |
| Neutral Oil | Caprylic/Capric Triglyceride | 1.0 |
| PCL Liquid^{®} 100 | Cetearyl Ethylhexoate | 0.3 |
| Ucare^{™} Polymer JR 400 | Polyquaternium-10 | 0.1 |
| Propylene Glycol | Propylene Glycol | 0.8 |
| SymGlucan^{®} | Aqua, Glycerin, 1,2-Hexanediol, Caprylyl Glycol, Beta-Glucan | 5 |
| SymMollient^{®} W/S | Trideceth-9, PEG-5 Isononanoate, Water (Aqua) | 2.0 |
| SymOcide^{®} PH | Phenoxyethanol, Hydroxyacetophenone, Caprylyl Glycol, Aqua | 1.2 |
| SymSol^{®}PF3 | Water, Pentylene Glycol, Sodium Lauryl Sulfoacetate, Sodium Oleoyl Sarcosinate, Sodium Chloride, Disodium Sulfoacetate, Sodium Oleate, Sodium Sulfate | 1.5 |
| Water, demineralized | Water (Aqua) | ad 100 |

### Emulsions

**Table 35: Anti-ageing Face Cream, soft touch**

| **Ingredients** | **EU INCI** | **Amount** |
|---|---|---|
| Aqua/ Water | Aqua | ad 100 |
| Glycerin 99.5% | Glycerin | 2.0 |
| 1,2-heptanediol, 2,3-heptanediol blend (w/w% 99:1) | 1,2-heptanediol, 2,3-heptanediol | 0.6 |
| Dragosine^{®} | Carnosine | 0.1 |
| Kelcogel^{®} CG-HA | Gellan Gum | 0.1 |
| Keltrol^{®} CG-SFT | Xanthan Gum | 0.4 |
| Dracorin^{®} CE | Glyceryl Stearate Citrate | 2.0 |
| Lanette^{®} O | Cetearyl Alcohol | 5.0 |
| SymMollient^{®} PDCC | Propanediol Dicaprylate/Caprate | 3.0 |
| SymMollient^{®} S green | Cetearyl Nonanoate | 2.0 |
| Plantoil Triglyceride | Caprylic/Capric Triglyceride | 8.0 |
| Dragoxat ^{®} 89 | Ethylhexyl Isononanoate | 2.0 |
| SymLite^{®} G8 | Glyceryl Caprylate | 0.3 |
| SymFinity ^{®} 1298 | Echinacea Purpurea Extract | 0.1 |
| LIFTONIN XPERT Eco | Water, Mannan, Pentylene Glycol, Citric Acid | 2.0 |
| Perfume oil PO1, PO2, PO3, PO4, or PO5 | Perfume | 0.3 |

**Table 36: Detox Face Primer**

| **Ingredients** | **EU INCI** | **Amount** |
|---|---|---|
| Aqua/ Water | Aqua | ad 100 |
| 1,2-heptanediol, 2,3-heptanediol blend (w/w% 95:5) | 1,2-heptanediol, 2,3-heptanediol | 1.0 |
| Glycerin 99.5% | Glycerin | 1.0 |
| 1,2-octanediol / 2,3-octanediol blend (w/w% 98:2) | 1,2-octanediol, 2,3-octanediol | 0,3 |
| Sodium Benzoate | Sodium Benzoate | 0.3 |
| Dragosine^{®} | Carnosine | 0.1 |
| UniqSens^{®} SFE-System | Pectin, Xanthan Gum, Carrageenan | 1.25 |
| Keltrol^{®} CG-SFT | Xanthan Gum | 0.25 |
| Dermofeel^{®} Sensolv | Isoamyl Laurate | 2.5 |
| Perfume oil PO1, PO2, PO3, PO4, or PO5 | Perfume | 0.3 |
| SymOcide^{®} PS | Phenoxyethanol, Decylene Glycol, 1,2 Hexanediol | 0.6 |
| Actipone^{®} Alpha Cranberry CA | Glycerin, Vaccinium Macrocarpon (Cranberry) Fruit Extract, Pentylene Glycol, Glyceryl Caprylate | 1.5 |
| Citric acid 10% sol | Citric acid, water | 0.15 |

**Table 37: Eye Cream for Mature Skin**

| **Ingredients** | **EU INCI** | **Amount** |
|---|---|---|
| Water | Aqua | ad 100 |
| Dermosoft^{®} 1288 | Glycerin, Water, Sodium Levulinate, Sodium Anisate | 3.5 |
| Glycerin | Glycerin | 3.0 |
| SymLite^{®} G8 | Glyceryl Caprylate | 0.2 |
| Sisterna SP30-C | Sucrose Distearate | 3.0 |
| Lanette^{®} O | Cetearyl Alcohol | 2.0 |
| Sisterna SP70-C | Sucrose Stearate | 1.0 |
| Keltrol^{®} CG-SFT | Xanthan Gum | 0.3 |
| 1,2-heptanediol, 2,3-heptanediol blend (w/w% 99:1) | 1,2-heptanediol, 2,3-heptanediol | 1.5 |
| Amisoft^{®} HS-11P(F) | Sodium Stearoyl Glutamate | 0.2 |
| Genuvisco^{®} CG-131 | Carrageenan | 0.1 |
| Cacao Butter | Cacao Butter | 2.0 |
| Avocado Oil | Persea Gratissima (Avocado) Oil | 2.0 |
| Plantoil Triglyceride | Caprylic/Capric Triglyceride | 17.0 |
| Perfume oil PO1, PO2, PO3, PO4, or PO5 | Perfume | 1.0 |
| SymOcide^{®} PH | Phenoxyethanol, Hydroxyacetophenone, Caprylyl Glycol, Water(Aqua) | 1.0 |
| Citric Acid, 10% sol | Aqua, Citric acid | 3.5 |

**Table 38: Eye contour serum**

| **Ingredients** | **EU INCI** | **Amount** |
|---|---|---|
| Dracorin^{®} GOC | Glyceryl Oleate Citrate, Caprylic/Capric Triglyceride | 1.50 |
| Dracorin^{®} CE | Glyceryl Stearate Citrate | 3.00 |
| Dragoxat^{®} 89 | Ethylhexyl Isononanoate | 3.00 |
| PCL-Liquid^{®}100 | Ceterayl Ethylhexanonate | 2.00 |
| SymMollient^{®} S green | Ceterayl Nonanoate | 2.00 |
| SymRelief^{®} 100 | Bisabolol, Zingiber officinale root extract | 0.10 |
| SymDetox^{™} 1711 | 3,3,5 Trimethylcyclohexyl succinate dimethylamide | 0.50 |
| Tocopheryl Acetate | Tocopheryl acetate | 0.25 |
| Aqua/Water | Aqua | ad 100 |
| Keltrol^{®} CG-F | Xanthan Gum | 0.20 |
| Nativacare^{™} 5600 | Zea(Mays) Corn Starch | 1.50 |
| Amigum | Sclerotium Gum | 0.20 |
| SymLift^{®} | Aqua, Trehalose, Glycerin, Pentylene Glycol, Beta-Glucan, Hordeum vulgare seed extract, sodium hyluronate, 1,2-Hexanediol, Caprylyl Glycol | 5.00 |
| SymSave^{®} H | Hydroxyacetophenone | 0.50 |
| EDTA NA2 | Disodium EDTA | 0.10 |
| Hydrolite^{®} 6 | 1,2-Hexanediol | 0.5 |
| 1,2-heptanediol, 2,3-heptanediol (w/w% 97:3) | 1,2-heptanediol, 2,3-heptanediol | 0,3 |
| Glycerin | Glycerin | 4.00 |
| Caffeine | Caffeine | 0.10 |
| Retinyl Palmitate | Retinyl Palmitate | 0.10 |
| Perfume oil PO1, PO2, PO3, PO4, or PO5 | Perfume | 0.50 |

**Table 39: Intensive Care, Detox and Repair Hand Cream 2-in-1**

| **Ingredients** | **EU INCI** | **Amount** |
|---|---|---|
| Emulsiophos^{®} | Potassium cetyl phosphate, hydrogenated palm glycerides | 1.50 |
| Lanette^{®} O | Cetearyl alcohol | 1.50 |
| Beeswax | Beeswax | 1.00 |
| PCL-Liquid^{®}100 | Cetearyl Ethylhexanoate | 3.00 |
| Softisan^{®}100 | Hydrogenated coco-glycerides | 5.00 |
| PCL-Solid | Stearyl Heptanoate, Stearyl Caprylate | 5.00 |
| Caprylic/Capric Triglyceride | Caprylic/ Capric Triglyceride | 1.50 |
| Tegosoft^{®} MM | Myristyl myristate | 2.50 |
| SymDetox^{®}1711 | 3,3,5-Trimethylcyclohexyl succinate dimethylamide | 0.50 |
| SymRepair^{®} 100 | Hexyldecanol, Bisabolol, Cethylhydroxyproline palmitamide, stearic acid, Brassica campestris (Rapeseed) sterols | 1.00 |
| SymDecanox^{™} HA | Caprylic/Capric Triglyceride, Hydroxymethoxyphenyl decanone | 1.00 |
| Rheocare XGN | Xanthan Gum | 0.20 |
| Nativacare^{™} 5600 | Zea(Mays) Corn Starch | 2.50 |
| Amigum | Sclerotium Gum | 0.20 |
| Aqua/Water | Aqua | ad 100 |
| Edeta^{®} BD | Disodium Edta | 0.10 |
| SymSave^{®} H | Hydroxyacetophenone | 0.50 |
| Hydroviton^{®} PLUS 2290 | Aqua, Pentylene Glycol, Glycerin, Fructose, Urea, Citric acid, Sodium hydroxide, Maltose, Sodium PCA, Sodium chloride, Sodium Lactate, Trehalose, Allantoin, Sodium Hyaluronate, Glucose | 2.00 |
| SymEssence^{®} Camomile CC | Chamomilla recutita (Matricaria) Flower/Leaf/Stem water | 1.00 |
| Colour | | 0.80 |
| Tinogard^{®} Q | Tris(tetramethylhydroxypiperidino)citr ate | 0.05 |
| Perfume oil PO1, PO2, PO3, PO4, or PO5 | Perfume | 0.40 |
| Sensocel^{®}10 | Cellulose | 2.00 |
| Dipropylene glycol | Dipropylene Glycol | 0.80 |
| Retinyl Palmitate | Retinyl Palmitate | 0.10 |
| 1,2-heptanediol, 2,3-heptanediol (w/w% 99:1) | 1,2-heptanediol, 2,3-heptanediol | 0.75 |
| 2,3-Octanediol | 2,3-Octanediol | 0.05 |
| SymOcide^{®}C | o-cymen-5-ol | 0.10 |

**Table 40: Refreshing and Soothing After Sun Cream**

| **Ingredients** | **EU INCI** | **Amount** |
|---|---|---|
| Dracorin^{®} CE | Glyceryl Stearate Citrate | 2.5 |
| Cutina^{®} GMS V | Glyceryl Stearate | 2.5 |
| Coconut Oil | Cocos Nucifera Oil | 3.0 |
| SymMollient^{®} PDCC | Propanediol Dicaprylate/ Caprate | 8.0 |
| Plantoil Triglyceride | Caprylic/Capric Triglyceride | 4.0 |
| 1,2-heptanediol, 2.3-heptanediol (w/w% 99:1) | 1,2-heptanediol, 2,3-heptanediol | 1.0 |
| Tocopherol | Tocopherol | 0.05 |
| Demin. Water | Aqua | ad 100 |
| Glycerin | Glycerin | 2.0 |
| SymOcide^{®} PS | Phenoxyethanol, Decylene Glycol, 1,2 Hexanediol | 1.0 |
| Nativacare^{®} 8600 | Oryza Sativa (Rice) Starch | 1.0 |
| Keltrol ^{®}CG-SFT | Xanthan Gum | 0.4 |
| Sirhamnose | Silanetriol (and) Rhamnose | 3.0 |
| D-Panthenol 75 L | Panthenol | 1.0 |
| Perfume oil PO1, PO2, PO3, PO4, or PO5 | Perfume | 0.3 |

**Table 41: Emulsion with low emulsifier content (not in accordance with the present invention)**

| **Ingredients** | **EU INCI** | **Amount** |
|---|---|---|
| Water, demin. | Aqua | ad 100 |
| Hydrolite^{®} 5 green | Pentylene Glycol | 3.0 |
| 1,2-heptanediol | 1,2-heptanediol | 2.0 |
| SymClariol | Decylene Glycol | 0.1 |
| SymSave^{®} H | Hydroxyacetophenone | 0.5 |
| Glycerin | Glycerin | 1.0 |
| Nativacare^{™} 5600 | Zea Mays (Corn) Starch | 1.5 |
| SymMollient^{®} S green | Cetaryl Nonanoate | 3.0 |
| Lanette^{®} O | Cetearyl Alcohol | 2.5 |
| Plant oil Triglyceride | Caprylic/Capric Triglyceride | 4.0 |
| SymMollient ^{®}PDCC | Propanediol Dicaprylate / Caprate | 3.0 |
| SymLite^{®} G8 | Glyceryl Caprylate | 0.5 |
| Dracorin^{®} CE | Glyceryl Stearate Citrate | 0.5 |
| Rheocare^{®} XGN | Xanthan Gum | 0.1 |
| Amigum | Sclerotium Gum | 0.25 |
| Citric Acid 10% solution | Aqua, Citric Acid | 0.1 |
| Perfume oil PO1, PO2, PO3, PO4, or PO5 | Perfume | 0.3 |

**Table 42: Tinted Sunscreen cream against hyperpigmentation exp. SPF 50***

| **Ingredients** | **INCI** | **Amount** |
|---|---|---|
| Emulsiphos^{®} | Potassium cetyl phosphate | 2.0 |
| | Hydrogenated palm glycerides | |
| Neo Heliopan^{®} 357 | Butyl methoxydibenzoylmethane | 4.2 |
| Neo Heliopan^{®} OS | Ethylhexyl salicylate | 5.0 |
| Neo Heliopan^{®} BMT | Bis-ethylhexyloxyphenol methoxyphenyl triazine | 3.5 |
| Uvasorb^{®} HEB | Diethylhexyl butamido triazone | 7.0 |
| SymMollient^{®} S green | Cetearyl nonanoate | 1.0 |
| lsoadipate | Diisopropyl adipate | 4.0 |
| SymBright^{™} 2036 | Sclareolide | 0.2 |
| SymSitive^{®} 1609 | Pentylene glycol, 4-t-butylcyclohexanol | 1.0 |
| Caprylic/Capric Triglyceride | Caprylic/capric triglyceride | 8.0 |
| SymDecanox^{™} HA | Caprylic/capric triglyceride Hydroxymethoxyphenyl decanone | 1.0 |
| Cetiol^{®} AB | C12-15 alkyl benzoate | 13.5 |
| EDTA^{®} BD | Disodium edta | 0.1 |
| Keltrol^{®} CG-BT | Xanthan gum | 0.3 |
| Genuvisco^{®} Carrageenan CG-131 | Chondrus crispus powder | 0.2 |
| Food Color Brown E172+E171 Powder | Titanium dioxides (ci 77891), Iron oxides (ci 77492), Iron oxides (ci 77491), Iron oxides (ci. 77499) | 2.0 |
| Eusolex^{®} T-AVO | Titanium dioxide, Silica | 2.0 |
| Aqua/Water | Aqua | 31.4 |
| Neo Heliopan^{®} Hydro | Phenylbenzimidazole sulfonic acid | 2.0 |
| Biotive^{®} L-Arginine | Arginine | 1.0 |
| Sodium Hydroxide 10% | Aqua, Sodium hydroxide | 0.7 |
| solution | | |
| Dragosine^{®} | Carnosine | 0.2 |
| Glycerin 99.5 % | Glycerin | 3.0 |
| | Aqua | |
| SymSave^{®} H | Hydroxyacetophenone | 0.5 |
| Hydrolite^{®} 5 green | Pentylene glycol | 1.5 |
| 1,2-heptanediol | 1,2-heptanediol | 0.2 |
| 2,3-heptanediol | 2,3-heptanediol | 0.3 |
| Phenoxyethanol | Phenoxyethanol | 0.5 |
| Lanette^{®} E | Sodium cetearyl sulfate | 0.8 |
| Perfume oil PO1; PO2; PO3; PO4; or PO5 | Perfume | 0.5 |
| SymOat PLUS (400124) | Aqua, Glycerin, Maltodextrin, 1,2-hexanediol, Beta-glucan, Caprylyl glycol | 2.0 |
| Sensocel^{®} 10 | Cellulose | 1.0 |

**Table 43: Urban Sun Spray, exp. SPF 50+ (not in accordance with the present invention)**

| **Ingredients** | **INCI** | **Amount** |
|---|---|---|
| Aqua /Water | Aqua | ad 100 |
| Vivapur^{®} CS Tex sun | Microcrystalline Cellulose, Cellulose Gum | 1.0 |
| Vivapur^{®} CS 032 XV | Microcrystalline Cellulose, Xanthan Gum | 1.0 |
| SymOcide^{®} PH | Phenoxyethanol, Hydroxyacetophenone, Caprylyl Glycol, Aqua | 1.4 |
| Emulsiphos^{®} | Potassium cetyl phosphate, Hydrogenatedpalm glycerides | 2.5 |
| Dragoxat^{®}89 | Ethylhexyl Isononanoate | 2.0 |
| SymMollient ^{®} PDCC | Propanediol Dicaprylate/Caprate | 3.5 |
| SymSol^{®} PF-3 | Aqua, Pentylene Glycol, Sodium Lauryl Sulfoacetate, Sodium Oleoyl Sarcosinate, Sodium Chlrodie, Sodium Oleate | 2.0 |
| Prisorine ^{™} 3505 | Isostearic acid | 1.5 |
| Neo Heliopan^{®} BMT | Bis-Ethylhexyloxyphenol Methoxypenyl Triazine | 2.5 |
| Neo Heliopan^{®} 357 | Butyl Methoxydibenzoylmethane | 4.5 |
| Neo Heliopan^{®} 303 | Octocrylene | 10.0 |
| Neo Heliopan^{®} HMS | Homosalate | 10.0 |
| Neo Heliopan^{®} OS | Ethylhexyl Salicylate | 5.0 |
| Dragosine^{®} | Carnosine | 0.2 |
| SymUrban^{®} | Benzylidene Dimethoxydimetylindanone | 0.2 |
| 1,2-heptanediol | 1,2-heptanediol | 0.5 |
| 2,3-octeanediol | 2,3-octanediol | 0,1 |
| SymVital^{®} AR 3040 | Zingiber officinale (Ginger) Root extract | 0.1 |
| SymDecanox^{™} HA | Caprylic/Capric Triglyceride, Hydroxymethoxyphenyl decanone | 1.0 |
| Floraesters K-100^{®} Jojoba | Hydrolyzed jojoba esters, Jojoba esters, Aqua | 0.5 |
| EDTA BD | Disodium EDTA | 0.1 |
| Keltrol^{®} CG-BT | Xanthan Gum | 0.3 |
| Perfume oil PO1; PO2; PO3; PO4; or PO5 | Perfume | 0.5 |
| Farmal^{®} MS6135 | Calcium Starch Octenylsuccinate | 2.0 |
| Tapioca Pure | Tapioca starch | 1.5 |

**Table 44: Sunscreen Spray (O/W), exp. SPF 30* (all in one pot/cold manufacturing process)**

| **Ingredients** | **INCI** | **Amount** |
|---|---|---|
| Dracorin^{®} GOC | Glyceryl Oleate Citrate Caprylic/ Capric Triglyceride | 2.0 |
| Dragosine^{®} | Carnosine | 0.2 |
| Neo Heliopan^{®} Flat | Homosalate, Octocrylene, Bis-Ethylhexyloxyphenol Methoxyphenyl Triazine, Butyl Methoxyd ibenzoylmethane Ethylhexyl Salicylate | 25.0 |
| SymMollient^{®} PDCC | Propanediol Dicaprylate/Caprate | 7.0 |
| Aristoflex^{®} Silk | Sodium Polyacryloyldiemthyl taurate | 0.6 |
| 2,3-Octanediol | 2,3-Octanediol | 0.2 |
| 1,2-heptanediol, 2,3-heptanediol (w/w% 95:5) | 1,2-heptanediol, 2,3-heptanediol | 0.3 |
| Aqua /Water | Aqua | ad 100 |
| Citric Acid 10% Sol. | Aqua, Citric acid | 0.25 |
| SymOcide^{®} PH | Phenoxyethanol, Hydroxyacetophenone, Caprylyl Glycol, Aqua | 1.45 |
| SymMollient^{®} PDCC (102119) | Propanediol Dicaprylate/ Caprate | 3.0 |
| Glycerin 99.5 % | Glycerin | 3.0 |
| Dragoxat^{®} 89 ( | Ethylhexyl Isononanoate | 2.0 |
| PCL-Liquid^{®} 100 | Cetearyl Ethylhexanoate | 2.0 |
| Edta^{®} BD | Disodium EDTA | 0.1 |
| Farmal^{®} MS 6135 | Calcium Starch Octenylsuccinate | 2.0 |
| Keltrol^{®} CG-BT | Xanthan Gum | 0.4 |
| Perfume oil PO1; PO2; PO3; PO4; or PO5 | Perfume | 0.3 |

**Table 45: Sunscreen Spray, *SPF 50+, "high water resistancy***

| **Ingredients** | **INCI** | **Amount** |
|---|---|---|
| Emulsiphos^{®} | Potassium Cetyl Phosphate Hydrogenated Palm Glycerides | 2.0 |
| SymMollient^{®} PDCC | Propanediol Dicaprylate/ Caprate | 5.0 |
| SymMollient^{®} S green | Cetearyl Nonanoate | 2.5 |
| Neo Heliopan^{®} 357 | Butyl Methoxydibenzoylmethane | 5.0 |
| Neo Heliopan^{®} HMS | Homosalate | 8.0 |
| Neo Heliopan^{®} 303 | Octocrylene | 8.0 |
| Neo Heliopan^{®} OS | Ethylhexyl Salicylate | 4.0 |
| Neo Heliopan^{®} BMT | Bis-Ethylhexyloxyphenol Methoxypenyl Triazine | 4.0 |
| Tocopherol Alpha DL | Tocopherol | 0.2 |
| Edeta^{®} BD | EDTA | 0.1 |
| SymEffect^{®} Sun | Cera Alba. Sodium Stearoyl Lactylate | 3.0 |
| Keltrol^{®} CG-SFT | Xanthan Gum | 0.2 |
| Water/ Aqua | Aqua | ad 100 |
| Neo Heliopan^{®} Hydro | Phenylbenzimidazole Sulfonic Acid | 1.5 |
| Biotive^{®} L-Arginine | Arginine | 1.0 |
| Dragosine^{®} | Carnosine | 0.1 |
| SymSave^{®} H | Hydroxyacetophenone | 0.5 |
| Hydrolite^{®} 5 green | Pentylene glycol | 1.8 |
| SymLite^{®} G8 | Glyceryl caprylate | 0.6 |
| Cranberry Oil CC | Vaccinium Macrocappon seed oil | 1.0 |
| Tapioca Pure | Tapioca starch | 2.0 |
| VITACEL^{®} CS 20 FC | Cellulose | 2.0 |
| 1,2-heptanediol 2,3-heptanediol (w/w% 95:5) | 1,2-heptanediol, 2,3-heptanediol | 0.5 |
| Perfume oil PO1; PO2; PO3; | Perfume | 0.3 |
| PO4; or PO5 | | |

**Table 46: Full protection drops, exp. SPF 50**

| **Ingredients** | **INCI** | **Amount** |
|---|---|---|
| Dracorin^{®} GOC | Glyceryl oleate citrate, Caprylic/capric triglyceride | 2.5 |
| Neo Heliopan^{®} 303 | Octocrylene | 8.0 |
| Neo Heliopan^{®} 357 | Butyl methoxydibenzoylmethane | 4.5 |
| Neo Heliopan^{®} HMS | Homosalate | 10.0 |
| Neo Heliopan^{®} OS | Ethylhexyl salicylate | 5.0 |
| Neo Heliopan^{®} BMT | Bis-ethylhexyloxyphenol methoxyphenyl triazine | 5.0 |
| SymMollient^{®} PDCC | Propanediol dicaprylate/caprate | 5.0 |
| Velvesil^{®} DM | Dimethicone, Cetearyl dimethicone crosspolymer | 3.0 |
| Dow Corning^{®} 9041 Silicone Elastomer Blend | Dimethicone, Dimethicone crosspolymer | 2.0 |
| EDTA^{®} BD | Disodium edta | 0.1 |
| Keltrol^{®} CG-SFT | Xanthan gum | 0.2 |
| Pemulen^{™} TR-2 Polymeric Emulsifier | Acrylates/c10-30 alkyl acrylate crosspolymer | 0.1 |
| Aqua/Water | Aqua | ad 100 |
| Glycerin 99.5 % | Glycerin, Aqua | 3.0 |
| Dragosine^{®} | Carnosine | 0.2 |
| SymSave^{®} H | Hydroxyacetophenone | 0.5 |
| Hydrolite^{®} 5 green | Pentylene glycol | 0.8 |
| Hydrolite^{®} CG | Caprylyl glycol | 0.3 |
| 1,2-heptanediol/ 2,3-heptanediol blend (95:5 w/w %) | 1,2-heptanediol, 2,3-heptanediol | 0.2 |
| SymVital^{®} AR (974839) | Zingiber officinale (ginger) root extract | 0.2 |
| Tapioca Pure | Tapioca starch | 2.0 |
| Perfume oil PO1; PO2; PO3; PO4; or PO5 | Perfume | 0.5 |

**Table 47: Anti-Sweat Sunscreen Lotion**

| **Ingredients** | **INCI** | **Amount** |
|---|---|---|
| Aqua/Water | Aqua | ad 100 |
| 2,3-Undecanediol | 2,3-Undecanediol | 0.05 |
| 1,2-heptanediol, 2,3-heptanediol (w/w% 95:5) | 1,2-heptanediol, 2,3-heptanediol | 0.3 |
| Citric Acid 30% Sol. | Aqua, Citric acid | 0.6 |
| Aristoflex^{®} Velvet | Polyacrylate crosspolymer 11 | 0.6 |
| Glycerin 99.5 % | Glycerin, Aqua | 2.0 |
| SymSol^{®} PF-3 | Aqua, Pentylene glycol, Sodium lauryl sulfoacetate, Sodium oleoyl sarcosinate, Sodium chloride, Sodium oleate | 1.0 |
| SymSave^{®} H | Hydroxyacetophenone | 0.3 |
| Neo Heliopan^{®} 357 | Butyl methoxydibenzoylmethane | 3.0 |
| Neo Heliopan^{®} 303 | Octocrylene | 10.0 |
| Neo Heliopan^{®} OS | Ethylhexyl salicylate | 5.0 |
| Neo Heliopan^{®} HMS | Homosalate | 10.0 |
| Neo Heliopan^{®} E1000 | Isoamyl p-methoxycinnamate | 2.0 |
| Neo Heliopan^{®} BMT | Bis-ethylhexyloxyphenol methoxyphenyl triazine | 3.0 |
| Floraesters K-100^{®} Jojoba | Hydrolyzed jojoba esters Jojoba esters Aqua | 2.0 |
| Dragoxat^{®} 89 | Ethylhexyl isononanoate | 6.0 |
| SymMollient^{®} S green | Cetearyl nonanoate | 1.0 |
| EDTA^{®} BD | Disodium edta | 0.1 |
| SymDecanox^{™} HA | Caprylic/capric triglyceride Hydroxymethoxyphenyl decanone | 1.0 |
| Frescolat^{®} Plus | Menthol Menthyl lactate | 0.3 |
| Perfume oil PO1; PO2; PO3; PO4; or PO5 | Perfume | 0.6 |
| SymDeo^{®} Plus | Lauryl alcohol, Phenoxyethanol, 2-benzylheptanol, Decylene glycol | 1.0 |
| Hydroviton^{®} PLUS 2290 | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 4.0 |
| Sensocel^{®} 10 | Cellulose | 2.0 |

**Table 48: HH-Cream Haute Homme, exp. SPF 15, UVA/UVB (not in accordance with the present invention)**

| **Ingredients** | **INCI** | **Amount** |
|---|---|---|
| Aqua/Water | Aqua | ad 100 |
| 2,3-Heptanediol | 2,3-heptanediol | 0.5 |
| SymSol^{®} PF-3 | Aqua, Pentylene glycol, Sodium lauryl sulfoacetate, Sodium oleoyl sarcosinate, Sodium chloride, Sodium oleate | 2.0 |
| Neo Heliopan^{®} Hydro | Phenylbenzimidazole sulfonic acid | 2.5 |
| Biotive^{®} L-Arginine | Arginine | 1.0 |
| Sodium Hydroxide 10% solution | Aqua, Sodium hydroxide | 1.3 |
| Aqua Keep 10SH-NFC | Sodium acrylates crosspolymer-2 | 2.0 |
| SymSave^{®} H | Hydroxyacetophenone | 0.5 |
| Dragosine^{®} | Carnosine | 0.1 |
| Hydrolite^{®} 5 | Pentylene glycol | 2.5 |
| Neo Heliopan^{®} OS | Ethylhexyl salicylate | 3.0 |
| Neo Heliopan^{®} 303 | Octocrylene | 3.0 |
| Neo Heliopan^{®} HMS | Homosalate | 3.0 |
| SymMollient^{®} S | Cetearyl nonanoate | 1.0 |
| Isoadipate | Diisopropyl adipate | 2.5 |
| SymRelief^{®} 100 | Bisabolol Zingiber officinale root extract | 0.2 |
| SymDecanox^{™} HA | Caprylic/capric triglyceride Hydroxymethoxyphenyl decanone | 1.5 |
| SymDetox^{®} | 3,3,5-trimethylcyclohexyl succinate dimethylamide | 0.5 |
| SymUrban^{®} | Benzylidene dimethoxydimethylindanone | 0.5 |
| Edeta^{®} BD | Disodium edta | 0.1 |
| Frescolat^{®} X-Cool | Menthyl ethylamido oxalate | 0.5 |
| SymBright^{™} 2036 | Sclareolide | 0.1 |
| SymWhite^{®} PLUS | Caprylic/capric triglyceride, Pentylene glycol Phenylethyl resorcinol, Bisabolol, Butyl methoxydibenzoylmethane | 1.3 |
| Keltrol^{®} CG-BT | Xanthan gum | 0.2 |
| FOOD COLOR BROWN E172+E171 POWDER (656623) | Titanium dioxides (ci 77891), Iron oxides (ci 77492), Iron oxides (ci 77491), Iron oxides (ci. 77499) | 2.0 |
| Perfume oil PO1; PO2; PO3; PO4; or PO5 | Perfume | 0.5 |
| SymGlucan^{®} | Aqua, Glycerin, 1,2-hexanediol, Caprylyl glycol, Beta-glucan | 5.0 |

**Table 49: BB Cream Dark Tone, exp. SPF 20 (not in accordance with the present invention)**

| **Ingredients** | **INCI** | **Amount** |
|---|---|---|
| Emulsiphos^{®} | Potassium cetyl phosphate Hydrogenated palm glycerides | 2.5 |
| Eumulgin^{®} SG | Sodium stearoyl glutamate | 0.5 |
| PCL-Liquid^{®} 100 | Cetearyl ethylhexanoate | 2.0 |
| Lanette^{®} O | Cetearyl alcohol | 1.0 |
| Dragoxat^{®} 89 | Ethylhexyl isononanoate | 1.0 |
| Cutina^{®} PES | Pentaerythrityl distearate | 1.0 |
| SymMollient^{®} S | Cetearyl nonanoate | 1.0 |
| Xiameter^{®} PMX-0245 Cyclopentasiloxane | Cyclopentasiloxane | 2.0 |
| Xiameter^{®} PMX-200 Silicone Fluid 50 cs | Dimethicone | 1.0 |
| Neo Heliopan^{®} 357 | Butyl methoxydibenzoylmethane | 3.0 |
| Neo Heliopan^{®} 303 | Octocrylene | 7.0 |
| Neo Heliopan^{®} HMS | Homosalate | 7.0 |
| Neo Heliopan^{®} OS | Ethylhexyl salicylate | 5.0 |
| SymDecanox^{™} HA | Caprylic/capric triglyceride Hydroxymethoxyphenyl decanone | 2.0 |
| Talcum | Talc | 2.0 |
| Food Color Titanium Dioxide Powder E171 | Ci 77891 | 0.8 |
| Keltrol^{®} CG-SFT | Xanthan gum | 0.4 |
| Carbopol^{®} Ultrez 20 Polymer | Acrylates/c10-30 alkyl acrylate crosspolymer | 0.3 |
| EDTA NA2 | Disodium EDTA | 0.2 |
| Aqua/Water | Aqua | ad 100 |
| Neo Heliopan^{®} Hydro | Phenylbenzimidazole sulfonic acid | 1.0 |
| Biotive^{®} L-Arginine | Arginine | 1.0 |
| Sodium Hydroxide 10% solution | Aqua Sodium hydroxide | 0.7 |
| | Ci 77891 | 1.5 |
| COSMETIC COLOR | Ci 77492 | |
| BROWN POWDER | Ci 77491 | |
| | Ci 77499 | |
| Food Color Iron Oxide Yellow E172 | Ci 77492 | 0.3 |
| Glycerin | Glycerin | 3.0 |
| Hydrolite^{®} 5 green | Pentylene glycol | 1.5 |
| Hydrolite^{®} CG | Caprylyl glycol | 0.2 |
| SymSave^{®} H | Hydroxyacetophenone | 0.5 |
| 2,3-heptanediol | 2,3-heptanediol | 1.0 |
| Hydroviton^{®} PLUS 2290 | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 2.0 |
| Perfume oil PO1; PO2; PO3; PO4; or PO5 | Perfume | 0.5 |

**Table 50: Daily Well Aging, SPF 30, UVA/UVB balanced, w/o Octocrylene**

| **Ingredients** | **INCI** | **Amount** |
|---|---|---|
| Lanette^{®} O | Cetearyl alcohol | 1.5 |
| Emulsiphos^{®} | Potassium cetyl phosphate Hydrogenated palm glycerides | 2.5 |
| SymUrban^{®} | Benzylidene dimethoxydimethylindanone | 0.3 |
| Neo Heliopan^{®} 357 | Butyl methoxydibenzoylmethane | 4.0 |
| Neo Heliopan^{®} OS | Ethylhexyl salicylate | 5.0 |
| Uvasorb^{®} HEB | Diethylhexyl butamido triazone | 2.0 |
| SymMollient^{®} S green | Cetearyl nonanoate | 1.0 |
| Cetiol^{®} Sensoft | Propylheptyl caprylate | 4.0 |
| Tegosoft^{®} TN | C12-15 alkyl benzoate | 11.0 |
| SymDecanox^{™} HA | Caprylic/capric triglyceride Hydroxymethoxyphenyl decanone | 1.0 |
| Isoadipate | Diisopropyl adipate | 7.5 |
| EDTA^{®} BD | Disodium edta | 0.1 |
| Carbopol^{®} ETD 2050 Polymer | Carbomer | 0.3 |
| Keltrol^{®} CG-T | Xanthan gum | 0.2 |
| Aqua/Water | Aqua | ad 100 |
| 1,2-heptanediol, 2,3-heptanediol (w/w% 95:5) | 1,2-heptanediol, 2,3-heptanediol | 0.7 |
| 2,3-Octanediol | 2,3-Octanediol | 0.1 |
| Glycerin 99.5 % | Glycerin, Aqua | 1.0 |
| Lanette^{®} E | Sodium cetearyl sulfate | 0.8 |
| Neo Heliopan^{®} Hydro | Phenylbenzimidazole sulfonic acid | 3.0 |
| Biotive^{®} L-Arginine | Arginine | 1.5 |
| Propylene Glycol | Propylene glycol | 3.0 |
| Sodium Hydroxide 10% solution | Aqua, Sodium hydroxide | 2.0 |
| SymSave^{®} H | Hydroxyacetophenone | 0.6 |
| Phenoxyethanol | Phenoxyethanol | 0.5 |
| Dragosine^{®} | Carnosine | 0.2 |
| SymVital^{®} AR | Zingiber officinale (ginger) root extract | 0.2 |
| Frescolat^{®} ML Cryst | Menthyl lactate | 0.5 |
| Perfume oil PO1; PO2; PO3; PO4; or PO5 | Perfume | 1.0 |
| Tapioca Pure | Tapioca starch | 2.0 |

**Table 51: Anti-Wrinkle Day Emulsion, exp. SPF 15**

| **Ingredients** | **INCI** | **Amount** |
|---|---|---|
| Emulsiphos^{®} | Potassium cetyl phosphate, Hydrogenated palm glycerides | 2.5 |
| Neo Heliopan^{®} 303 | Octocrylene | 2.0 |
| Neo Heliopan^{®} OS | Ethylhexyl salicylate | 5.0 |
| Isoadipate | Diisopropyl adipate | 2.0 |
| SymMollient^{®} S green | Cetearyl nonanoate | 2.0 |
| Caprylic/Capric Triglyceride | Caprylic/capric triglyceride | 3.0 |
| Dragoxat^{®} 89 | Ethylhexyl isononanoate | 1.0 |
| PCL-Liquid^{®} 100 | Cetearyl ethylhexanoate | 3.0 |
| SymUrban^{®} | Benzylidene dimethoxydimethylindanone | 0.3 |
| SymDecanox^{™} HA | Caprylic/capric triglyceride Hydroxymethoxyphenyl decanone | 2.0 |
| Edeta^{®} BD | Disodium edta | 0.1 |
| Keltrol^{®} CG-SFT | Xanthan gum | 0.2 |
| Cosmedia^{®} SP | Sodium polyacrylate | 0.4 |
| Aqua/Water | Aqua | ad 100 |
| 2,3-heptanediol | 2,3-heptanediol | 0.3 |
| 1,2-heptanediol | 1,2-heptanediol | 0.2 |
| Glycerin | Glycerin | 3.0 |
| Neo Heliopan^{®} Hydro | Phenylbenzimidazole sulfonic acid | 2.0 |
| Biotive^{®} L-Arginine | Arginine | 1.0 |
| Sodium Hydroxide 10% solution | Aqua, Sodium hydroxide | 1.2 |
| Dragosine^{®} | Carnosine | 0.2 |
| SymSave^{®} H | Hydroxyacetophenone | 0.5 |
| SymDiol^{®} 68 ( | 1,2-hexanediol, Caprylyl glycol | 0.3 |
| Phenoxyethanol | Phenoxyethanol | 0.2 |
| Hydroviton^{®} PLUS 2290 | Aqua, Pentylene glycol, Glycerin, Fructose, Urea, Citric acid, Sodium | 3.0 |
| | hydroxide, Maltose, Sodium pca Sodium chloride, Sodium lactate, Trehalose, Allantoin, Sodium hyaluronate, Glucose | |
| SymLift^{®} | Aqua, Trehalose, Glycerin, Pentylene glycol, Beta-glucan, Hordeum vulgare seed extract, Sodium hyaluronate, 1,2-hexanediol, Caprylyl glycol | 5.0 |
| Macadamia Oil (refined) | Macadamia integrifolia seed oil | 0.2 |
| SymVital^{®} AR | Zingiber officinale (ginger) root extract | 0.2 |
| Actipone^{®} Laminaria Saccharina GW | Glycerin, Aqua, Laminaria saccharina extract | 1.0 |
| Ethanol 96 % | Alcohol denat. | 3.0 |
| Sensocel^{®} 10 | Cellulose | 1.0 |
| Sipernat^{®} 11 PC | Hydrated silica | 1.0 |
| Perfume oil PO1; PO2; PO3; PO4; or PO5 | Perfume | 0.4 |

**Table 52: Sensitive Day Care Cream, exp. SPF 15 (not in accordance with the present invention)**

| **Ingredients** | **INCI** | **Amount** |
|---|---|---|
| Lanette^{®} O | Cetearyl alcohol | 4.0 |
| Emulsiphos^{®} | Potassium cetyl phosphate, Hydrogenated palm glycerides | 2.0 |
| Cetiol^{®} AB | C12-15 alkyl benzoate | 1.0 |
| Cetiol^{®} OE | Dicaprylyl ether | 1.0 |
| SymMollient^{®} S | Cetearyl nonanoate | 1.0 |
| Neo Heliopan^{®} 303 | Octocrylene | 3.0 |
| Grape Seed Oil (Refined) | Vitis vinifera seed oil | 1.0 |
| Corapan^{®} TQ | Diethylhexyl 2,6-naphthalate | 3.0 |
| Eumulgin^{®} SG | Sodium stearoyl glutamate | 1.0 |
| SymCare^{®} O | Hexyldecanol, Pentylene glycol, 4-t-butylcyclohexanol, Bisabolol, Cetylhydroxyproline palmitamide, Hydroxyphenyl propamidobenzoic acid, Stearic acid, Brassica campestris (rapeseed) sterols, Zingiber officinale (ginger) root extract | 3.0 |
| Softisan^{®} 100 | Hydrogenated coco-glycerides | 1.0 |
| Eutanol^{®} G | Octyldodecanol | 2.0 |
| Caprylic/Capric Triglyceride | Caprylic/capric triglyceride | 5.0 |
| Carbopol^{®} Ultrez 10 Polymer | Carbomer | 0.3 |
| Aqua/Water | Aqua | ad 100 |
| 1,2-heptanediol | 1,2-heptanediol | 0.3 |
| Neo Heliopan^{®} Hydro | Phenylbenzimidazole sulfonic acid | 3.0 |
| Biotive^{®} L-Arginine | Arginine | 1.0 |
| Sodium Hydroxide 10% solution | Aqua, Sodium hydroxide | 2.0 |
| Edeta^{®} BD | Disodium EDTA | 0.1 |
| Glycerin | Glycerin | 5.0 |
| SymSave^{®} H | Hydroxyacetophenone | 0.5 |
| Phenoxyethanol | Phenoxyethanol | 0.5 |
| Hydroviton^{®} PLUS 2290 | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 3.0 |
| D-Panthenol 75 W | Panthenol, Aqua | 2.0 |
| Tapioca Pure | Tapioca starch | 4.0 |
| Actipone^{®} Licorice | Aqua, Butylene glycol, Glycyrrhiza glabra root extract | 2.0 |
| Sodium Hydroxide 10% solution | Aqua, Sodium hydroxide | 1.5 |

**Table 53: Perfect Hand, exp. SPF 15, UVA/UVB Balanced**

| **Ingredients** | **INCI** | **Amount** |
|---|---|---|
| Emulsiphos^{®} | Potassium cetyl phosphate Hydrogenated palm glycerides | 1.5 |
| Lanette^{®} O | Cetearyl alcohol | 1.0 |
| Tegosoft^{®} MM | Myristyl myristate | 0.5 |
| Neo Heliopan^{®} OS | Ethylhexyl salicylate | 5.0 |
| Edeta^{®} BD | Disodium EDTA | 0.1 |
| Dragoxat^{®} 89 | Ethylhexyl isononanoate | 2.0 |
| Isoadipate | Diisopropyl adipate | 6.0 |
| PCL-Liquid^{®} 100 | Cetearyl ethylhexanoate | 3.0 |
| Corapan^{®} TQ | Diethylhexyl 2,6-naphthalate | 2.0 |
| Cetiol^{®} SB 45 | Butyrospermum parkii butter | 1.5 |
| SymBright^{™} 2036 | Sclareolide | 0.2 |
| SymDecanox^{™} HA | Caprylic/capric triglyceride, Hydroxymethoxyphenyl decanone | 1.0 |
| SymCare^{®} O | Hexyldecanol, Pentylene glycol, 4-t-butylcyclohexanol, Bisabolol, Cetylhydroxyproline palmitamide, | 2.0 |
| | Hydroxyphenyl propamidobenzoic acid, Stearic acid, Brassica campestris (rapeseed) sterols, Zingiber officinale (ginger) root extract | |
| Sym Detox^{®} | 3,3,5-trimethylcyclohexyl succinate dimethylamide | 0.5 |
| Genuvisco^{®} Carrageenan CG-131 | Chondrus crispus powder | 0.4 |
| Keltrol^{®} CG-SFT | Xanthan gum | 0.2 |
| Aqua/Water | Aqua | ad 100 |
| 1,2-heptanediol, 2,3-heptanediol (95:5 w/w%) | 1,2-heptanediol, 2,3-heptanediol | 0.3 |
| 2,3 Undecanediol | 2,3 Undecanediol | 0.01 |
| Neo Heliopan^{®} Hydro | Phenylbenzimidazole sulfonic acid | 2.5 |
| Biotive^{®} L-Arginine | Arginine | 1.0 |
| Sodium Hydroxide 10% solution | Aqua | 1.3 |
| | Sodium hydroxide | |
| Glycerin | Glycerin | 2.0 |
| SymSave^{®} H | Hydroxyacetophenone | 0.5 |
| SymDiol^{®} 68 | 1,2-hexanediol, Caprylyl glycol | 0.5 |
| RonaFlair^{®} Flawless | Silica | 1.5 |
| | Ci 77892 | |
| | Ci 77491 | |
| Extrapone^{®} Moroccan Rose GW | Aqua, Glycerin, Peg-40 hydrogenated castor oil, Rosa damascena flower extract | 1.0 |
| Tego^{®} Feel C10 | Cellulose | 1.5 |
| Perfume oil PO1; PO2; PO3; PO4; or PO5 | Perfume | 0.5 |

**Table 54: Natural Tanning Sun Milk, SPF 30**

| **Ingredients** | **INCI** | **Amount** |
|---|---|---|
| Aqua/Water | Aqua | ad 100 |
| Vivapur^{®} CS Tex Sun | Microcrystalline cellulose, Cellulose gum | 1.0 |
| Vivapur^{®} CS 032 XV | Microcrystalline cellulose, Xanthan Gum | 1.0 |
| SymSave^{®} H | Hydroxyacetophenone | 0.5 |
| Hydrolite^{®} 5 green | Pentylene Glycol | 1.5 |
| Hydrolite^{®} CG | Capryly Glycol | 0.25 |
| Dragosine^{®} | Carnosine | 0.2 |
| Neo Helipan^{®} BMT | Bis-Ethylhexyloxyphenol | 3.5 |
| | Methoxyphenyl Triazine | |
| Neo Helipan^{®} 357 | Butyl Methoxydibenzoylmethane | 4.5 |
| Neo Heliopan^{®} 303 | Octocrylene | 7.5 |
| Neo Heliopan^{®} HMS | Homosalate | 8.0 |
| Neo Heliopan^{®} OS | Ethylhexyl Salicylate | 4. |
| 1,2-octanediol, 2,3-octanediol (w/w% 95:5) | 1,2-octeanediol, 2,3-octeanediol | 0.1 |
| 1,2-heptanediol, 2,3-heptanediol (w/w% 90:10) | 1,2-heptanediol, 2,3-heptanediol | 0.6 |
| Emulsiphos^{®} | Potassium Cetyl Phosphate, Hydrogenated palm glycerides | 2.5 |
| SymSol^{®} PF-3 | Aqua, Pentylene Glycol, Sodium Lauryl Sulfoacetate, Sodium Oleoyl Sarcosinte, Sodium Chloride, Sodium Oleate | 2.0 |
| SymMollient^{®} S green | Cetearyl Nonanoate | 1.5 |
| Dragoxat^{®} 89 | Ethylhexyl Nonanoate | 2.0 |
| SymMollient^{®} PDCC | Propanediol Dicaprylate/Caprate | 2.0 |
| Mango Butter | Mangifera indica seed butter | 3.0 |
| Prisorine^{®} 3505 | Isostearic acid | 1.5 |
| SymDecanox^{™} HA | Caprylic/ Capric Triglyceride, Hydroxymethoxyphenyl decanone | 1.0 |
| Floraesters K-100^{®} Jojoba | Hydrolyzed Jojoba esters, Jojoba esters, Aqua | 0.5 |
| Edeta^{®} BD | Disodium EDTA | 0.1 |
| Keltrol^{®} CG-BT | Xanthan Gum | 0.3 |
| Phytoconcentrole^{®} Carrot | Helianthus annuus seed oil, Beta-carotene, Daucus carota sativa root extract | 1.0 |
| SymBronze^{®} 1659 | Caprylic/Capric Triglyceride, lochrysis Galbana Extract | 2.0 |
| Tapioca Pure | Tapioca Starch | 2.0 |
| Perfume oil PO1; PO2; PO3; PO4; or PO5 | Perfume | 0.5 |

The aroma composition (A1) described below was incorporated into the oral care formulations presented below.

**Table 55: Aroma composition (A1); Peppermint flavour (amounts in ‰ by weight)**

| **Ingredients** | **Amount** |
|---|---|
| Isobutyraldehyde | 0.5 |
| 3-Octanol | 0.5 |
| Dimethyl sulphide | 0.5 |
| Trans-2-Hexanal | 1.0 |
| Cis-3-Hexenol | 1.0 |
| 4-Terpineol, natural | 1.0 |
| Isopulegol | 1.0 |
| Piperitone, natural, from eucalyptus | 2.0 |
| Linalool | 3.0 |
| 8-Ocimenyl acetate 10% in triacetin | 5.0 |
| Isoamyl alcohol | 10.0 |
| Isovaleraldehyde | 10.0 |
| Alpha-Pinene,natural | 25.0 |
| Beta-Pinene, natural | 25.0 |
| Neomenthol. Racemic | 40.0 |
| Eucalyptiol (1.8-cineol).natural | 50.0 |
| L-Menthyl acetate of the formula D | 70.0 |
| L-Menthone | 220.0 |
| D-Isomenthone | 50.0 |
| L-Menthol | 483.5 |
| Nonenolide | 1.0 |
| **Total:** | **1000** |

**Table 56: Protective Mouth Rinse (not in accordance with the present invention)**

| **Ingredients** | **EU INCI** | **Amount** |
|---|---|---|
| Cremophor CO 40 Surfactant | PEG-40 Hydrogenated Castor oil | 3.0 |
| 1,2-Heptanediol | 1,2-heptanediol | 1.0 |
| Aroma 1 (A1) | Aroma 1 (A1) | 0.5 |
| SymGuard^{®} CD | Phenylpropanol, o-cymen-5-ol, Decylene Glycol | 0.3 |
| Glycerin | Glycerin | 5.0 |
| Propylene Glycol | Propylene Glycol | 10.0 |
| SymOcide^{®} PH | Phenoxyethanol, Hydroxyacetophenone, Caprylyl Glycol, Aqua | 0.5 |
| Sorbitol Liq EP 70% | Sorbitol | 10.0 |
| Aqua! Water | Aqua | ad 100 |
| Sodium Saccharin | Sodium Sachharin | 0.1 |
| Colour I | | 1.2 |
| Colour II | | 0.2 |

**Table 57: Refresh your breath - mouth spray (not in accordance with the present invention)**

| **Ingredients** | **EU INCI** | **Amount** |
|---|---|---|
| Aqua | Aqua | ad 100 |
| Sorbitol Liq. EP 70% | Sorbitol | 1.0 |
| Sodium Saccharin | Sodium Saccharin | 0.1 |
| Cremophor^{®} CO455 | PEG_40 Hydrogenated castor oil, Propylene Glycol | 5.0 |
| Aroma 1 (A1) | Aroma 1 (A1) | 0.8 |
| Frescolat^{®} ML Cryst | Mentyl lactate | 2.0 |
| 1,2-heptanediol. 2,3-heptanediol (w/w% 95:5) | 1,2-heptanediol, 2,3-heptanediol | 1.0 |
| Propylene Glycol | Propylene Glycol | 10.0 |
| SymOcide^{®} C | o-Cymen-5-ol | 0.1 |
| SymSave^{®} H | Hydroxyacetophenone | 0.25 |
| SymDiol^{®} 68 | 1,2-Hexanediol, Caprylyl Glycol | 0.5 |
| Extrapone^{®} Peppermint | Aqua, Propylene Glycol, Mentha piperita leaf extract | 2.0 |
| Colour | | 0.194 |

**Table 58: Mouthwash (Ready to use) without alcohol (not in accordance with the present invention)**

| **Ingredients** | **EU INCI** | **Amount** |
|---|---|---|
| Cremophor^{®} CO40 | PEG_40 Hydrogenated castor oil | 2.5 |
| SymGuard^{®} CD | Phenylpropanol, o-cymen-5-ol, Decylene Glycol | 0.5 |
| Propylene Glycol | Propylene Glycol | 5.0 |
| Aroma 1 (A1) | Aroma 1 (A1) | 0.2 |
| Aqua | Aqua | ad 100 |
| Sorbitol Liq. EP 70% | Sorbitol | 10.0 |
| 1,2-heptanediol, 2,3-heptanediol (w/w% 95:5) | 1,2-heptanediol, 2,3-heptanediol | 0.5 |
| 2,3 Octanediol | 2,3 Octanediol | 0.025 |
| Sodium Saccharin | Sodium Saccharin | 0.07 |
| Sodium Fluorid | Sodium Fluorid | 0.18 |
| SymSave^{®} H | Hydroxyacetophenone | 0.3 |

**Table 59: Mouthwash concentrate (not in accordance with the present invention)**

| **Ingredients** | **EU INCI** | **Amount** |
|---|---|---|
| Ethanol | Ethanol | ad 100 |
| Aroma 1 (A1) | Aroma 1 (A1) | 0.8 |
| 1,2-heptanediol | 1,2-heptanediol | 0.75 |
| 2,3-heptanediol | 2,3-heptanediol | 0.05 |
| Menthol | Menthol | 0.1 |
| Cremophor CO 40 Surfactant | PEG-40 Hydrogenated Castor oil | 3.0 |
| Disodium Azacycloheptane Diphosphonate | Disodium Azacycloheptane Diphosphonate | 0.5 |
| Cetylpyridinium chloride | Cetylpyridinium chloride | 0.1 |
| Zinc-Chloride | Zinc-Chloride | 0.1 |
| Disodium Phosphate | Disodium Phosphate | 0.15 |
| Trisodium Phosphate | Trisodium Phosphate | 0.05 |
| Sodium Fluoride | Sodium Fluoride | 0.18 |
| Aqua | Aqua | 45 |

**Table 60: Air freshener (A/F spray water based) (not in accordance with the present invention)**

| **No** | **Ingredients** | **Amount** |
|---|---|---|
| 1 | Fragrance | 4 |
| 2 | Ethanol | 8 |
| 3 | PEG-40 Hydrogenated Castor Oil | 5 |
| 4 | 1,2-heptanediol | 1 |
| 5 | Aqua | add to 100 |

### Colorless liquid, pH neutral

**Table 61: All purpose cleaner (not in accordance with the present invention)**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 1,2-heptanediol | 1 |
| 6 | Aqua | add to 100 |

### Colorless liquid, pH 7

**Table 62: All purpose cleaner (not in accordance with the present invention)**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 2,3-octanediol | 0.1 |
| 6 | 1,2-heptanediol | 0.9 |
| 7 | Aqua | add to 100 |

### Colorless liquid, pH 7

**Table 63: APC alkaline (not in accordance with the present invention)**

| **No** | **Ingredients** | **Amount** |
|---|---|---|
| 1 | Coco-Glycoside | 1 |
| 2 | Benzalkonium Chloride | 0.2 |
| 3 | Sodiumbicarbonat | 0.1 |
| 4 | Tri-sodiumcitrate-dihydrate | 0.1 |
| 5 | Methylglycinediacetic acid | 0.1 |
| 6 | 1,2-Benzisothiazol-3(2H)-on | 0.1 |
| 7 | Fragrance | 0.1 |
| 8 | 1,2-heptanediol | 0.5 |
| 9 | Aqua | add to 100 |

### Colorless liquid, pH 9

**Table 64: Cleaner, liquid, citric acid (not in accordance with the present invention)**

| **No** | **Ingredients** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol | 0.5 |
| 5 | Fragrance | 0.3 |
| 6 | Aqua | add 100 |

### Liquid of low viscosity, pH 2

**Table 65: Cleaner, liquid, citric acid**

| **No** | **Ingredient** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol/2,3-heptanediol 90 : 10 | 0.5 |
| 5 | Fragrance | 0.3 |
| 6 | Aqua | add 100 |

### Liquid of low viscosity, pH 2

**Table 66: Detergent liquid light duty (not in accordance with the present invention)**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-octanediol | 0.1 |
| 9 | 1,2-heptanediol | 0.9 |
| 10 | Fragrance | 0.5 |
| 11 | Aqua | add to 100 |

### Liquid, pH 7.3

**Table 67: Detergent liquid light duty (not in accordance with the present invention)**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-heptanediol | 0.1 |
| 9 | 1,2-heptanediol | 0.9 |
| 10 | Fragrance | 0.5 |
| 11 | Aqua | add to 100 |

### Liquid, pH 7.3

**Table 68: Dishwash liquid manual (not in accordance with the present invention)**

| **No** | **Ingredients** | **Amount** |
|---|---|---|
| 1 | Sodium Laureth Sulfate | add 100 |
| 2 | Ethanol | 2.9 |
| 3 | lauramine oxide | 7.7 |
| 4 | propylene glycol | 1.9 |
| 5 | Phenoxyethanol | 0.1 |
| 6 | Benzisothiazolinone, Methylisothiazolinone, Laurylamine Dipropylenediamine | 0.1 |
| 7 | 1,2-heptanediol | 0.5 |
| 8 | sodium chloride | 3.85 |
| 9 | Fragrance | 0.3 |

### Colorless liquid, pH 8.5

**Table 69: Fabric softener (not in accordance with the present invention)**

| **No** | **Ingredients** | **Amount** |
|---|---|---|
| 1 | Di-(Talg Carboxyethyl) Hydroxyethyl methylammonium-methosulfat | 5.5 |
| 2 | Simethicone | 0.3 |
| 3 | 2-octyl-2H-isothiazol-3-one | 0.1 |
| 4 | 1,2-heptanediol | 0.5 |
| 5 | Fragrance | 0.2 |
| 6 | Aqua | add 100 |

### Liquid, pH 3

**Table 70: Fabric softener concentrate, encapsulated (not in accordance with the present invention)**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 1,2-heptanediol | 0.5 |
| 8 | Fragrance | 0.6 |
| 9 | Aqua | add 100 |

### White liquid, pH 2.5

**Table 71: Fabric softener concentrate, encapsulated (not in accordance with the present invention)**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 2,3-heptanediol | 0.05 |
| 8 | 1,2-heptanediol | 0.5 |
| 9 | Fragrance | 0.6 |
| 10 | Aqua | add 100 |

### White liquid, pH 2.5

**Table 72: Hand soap, liquid (not in accordance with the present invention)**

| **No** | **Ingredients** | **Amount** |
|---|---|---|
| 1 | Sodium Laureth Sulfate | 18 |
| 2 | Cocoamidopropylbetaine | 6 |
| 3 | Cocamide DEA | 3 |
| 4 | citric acid | 0.5 |
| 5 | sodium chloride | 1.9 |
| 6 | Glycerol | 2 |
| 7 | Fragrance | 0.2 |
| 8 | 1,2-heptanediol | 0.25 |
| 9 | Aqua | add 100 |

### Slightly colored liquid, pH 6

**Table 73: Rim block gel (not in accordance with the present invention)**

| **No** | **Ingredients** | **Amount** |
|---|---|---|
| 1 | Sodium Laureth Sulfate | 11 |
| 2 | Trideceth-9 | 2 |
| 3 | Xanthan gum | 2 |
| 4 | Hydroxyethylcellulose | 0.5 |
| 5 | Citric Acid | 0.4 |
| 6 | Ethanol | 6 |
| 7 | Fragrance | 5 |
| 8 | 1,2-heptanediol | 0.5 |
| 9 | Aqua | add 100 |

### Colorless paste/gel, pH = 4.5

**Table 74: Scent Lotion with capsules (not in accordance with the present invention)**

| **No** | **Ingredients** | **Amount** |
|---|---|---|
| 1 | microcrystalline cellulose/cellulose gum | 1 |
| 2 | PEG-40 hydrogenated Castor oil/ Trideceth-9 | 0.5 |
| 3 | encapsulated perfume oil | 1 |
| 4 | 2-octyl-2H-isothiazol-3-one | 0.1 |
| 5 | Fragrance | 5 |
| 6 | 1,2-heptanediol | 1 |
| 7 | Aqua | add 100 |

### White emulsion, pH neutral

**Table 75: Cleaner, liquid, lactic acid (not in accordance with the present invention)**

| **No** | **Ingredients** | **Amount** |
|---|---|---|
| 1 | Sodium Laureth Sulfate | 1.8 |
| 2 | Trideceth-9 | 2.1 |
| 3 | Xanthan gum | 0.35 |
| 4 | Lactic Acid | 2,8 |
| 5 | 1,2-heptanediol | 1 |
| 6 | Fragrance | 0.3 |
| 7 | Aqua | add 100 |

### Colorless liquid, pH 2,0

**Table 76: Cleaner, liquid, citric acid (not in accordance with the present invention)**

| **No** | **Ingredients** | **Amount** |
|---|---|---|
| 1 | Sulfonic acid, C 13-17-sec-Alkyl-, Sodium salt | 1 |
| 2 | Xanthan gum | 0-3 |
| 3 | citric acid | 5 |
| 4 | 1,2-heptanediol | 0.25 |
| 5 | Fragrance | 0.3 |
| 6 | Aqua | add 100 |

Liquid, pH 2

## Claims

1. A cosmetic or pharmaceutical composition or homecare product, comprising or consisting of:
(a) 0.3 to 3.0 % by weight of a mixture comprising at least one first linear alkanediol having a carbon chain of 5 to 14 carbon atoms and one or more second linear alkanediols having a carbon chain of 5 to 14 carbon atoms which is different from the first linear alkanediol, based on the total weight of the composition or homecare product;
(b) 0.05 to 6.0 % by weight of at least one natural polymer, based on the total weight of the composition or homecare product; and
(c) optionally at least one active substance for a cosmetic or pharmaceutical composition or a homecare product and/or additive,
wherein the mixture comprising at least one first linear alkanediol and one or more second linear alkanediol is selected from the group consisting of:
a mixture comprising 1,2-hexanediol and 2,3-hexanediol;
a mixture comprising 1,2-heptanediol and 2,3-heptanediol;
a mixture comprising 1,2-octanediol and 2,3-octanediol; and
a mixture comprising 1,2-heptanediol and 1,2-nonanediol; and
wherein the at least one natural polymer is selected from the group consisting of guar gum, xanthan gum, carboxymethylcellulose, and mixtures of two or more of the aforesaid natural polymers.

2. The cosmetic or pharmaceutical composition or homecare product according to claim 1, wherein the first linear alkanediol or the second alkanediol is an alkanediol having a maximum water solubility of less than or equal to 10% by weight, in particular a maximum water solubility of less than or equal to 5 % by weight and more than or equal to 1.4 % by weight, preferably an alkanediol selected from the group consisting of 1,2-heptanediol, 2,3-heptanediol, and mixtures thereof.

3. The cosmetic or pharmaceutical composition or homecare product according to claim 1 or claim 2,
- wherein the mixture comprising at least one first linear alkanediol and one or more second linear alkanediol comprises the first linear alkanediol and the second alkanediol in a ratio in a range of 50 : 50 to 99.9 : 0.1, preferably in a ratio in a range of 75 : 25 to 99 : 1, more preferably in a ratio in a range of 80 : 20 to 98 : 2, even more preferably in a ratio in a range of 90 : 10 to 95 : 5.

4. The cosmetic or pharmaceutical composition or homecare product according to any one of claims 1 to 3, wherein the at least one active substance for a cosmetic or pharmaceutical composition or homecare product and/or additive is selected from the group consisting of agents against ageing of the skin, antioxidants, chelating agents, emulsifiers, surfactants, preservatives, synthetic polymers, skin-cooling agents, rheology additives, oils, fragrances or perfume oils, polyols and mixtures of two or more of the aforementioned substances.

5. The cosmetic or pharmaceutical composition or homecare product according to any one of claims 1 to 4, further comprising at least one antimicrobial agent selected from the group consisting of Caprylhydroxamic Acid, o-cymen-5-ol, Isopropylparaben, Capryloyl Glycine, Phenylpropanol, Tropolone, PCA Ethyl Cocoyl Arginate, 2-Methyl 5-Cyclohexylpentanol, Phenoxyethanol, Disodium EDTA, Methylparaben and its salts, Sodium Benzoate, Benzyl Alcohol, Potassium Sorbate, Benzyl Salicylate, Propylparaben, Methylchloroisothiazolinone, Methylisothiazolinone, Ethylhexylglycerin, Butylparaben, Ethylparaben, Sodium Propylparaben, DMDM Hydantoin, Hydroxyethoxyphenyl Butanone, Hydroxyethoxyphenyl Butanol, Itaconic Acid and salts thereof, Octopirox, Propanediol Caprylate, Climbazole, 4-Hydroxyacetophenone, Dehydroacetic Acid, lodopropynyl Butylcarbamate, Salicylic Acid, Chlorphenesin, Isobutylparaben, Sodium Ethylparaben, Diazolidinyl Urea, Farnesol, Bisabolol, Glyceryl Caprylate, Sodium Phytate or Phytic acid, Sodium Levulinate or Levulinic Acid and esters therof, Chlorhexidine, Glyceryl Laurate, Anisic Acid and salts thereof, Chlorhexidine Digluconate, TEA-Salicylate, Phenethyl Alcohol, Glyceryl Caprate, Sorbitan Caprylate, Tetrasodium Glutamate Diacetate, Trisodium Ethylenediamine Disuccinate, Sodium Lactate, Undecylenoyl glycine, Thymol, 1,3-Propanediol, 4-hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, 3,4,4'-trichlorocarbanilide (TTC), antibacterial fragrances, thymol, thyme oil, eugenol, oil of cloves, menthol, mint oil, glycerol monocaprate, glycerol monocaprylate, glycerol monolaurate (GML), diglycerol monocaprate (DMC), salicylic acid N-alkylamides, such as, for example, n-octylsalicylamide or n-decylsalicylamide, Dehydroacetic Acid, 4-Methylbenzyl Alcohol, Frambinon, Xylityl caprylate, Benzoic acid 3-hyroxypropylester, Anisic acid 3-hydroxypropylester, 3-Hydroxypropyl 2-hydroxybenzoate and mixtures of two or more the aforesaid antimicrobial agents.

6. The cosmetic or pharmaceutical composition or homecare product according to any one of claims 1 to 5, comprising the mixture comprising at least one first linear alkanediol and one or more second linear alkanediols in an amount of 0.5 to 1.0 % by weight, based on the total weight of the composition or homecare product;
and/or comprising the 2,3-alkandediol of the mixture comprising at least one first linear alkanediol and one or more second linear alkanediol in an amount of 0.5 to 1.0 % by weight, based on the total weight of the composition or homecare product; preferably in an amount of 0.001 to 0.5 % by weight, more preferably in an amount of 0.005 to 0.1 % by weight, and most preferably in an amount of 0.01 to 0.075 % by weight, based on the total weight of the composition or homecare product.

7. The cosmetic or pharmaceutical composition according to any one of claims 1 to 6
(i) in the form of a dispersion comprising the oil component in an amount of ≥ 1 % by weight, in particular in an amount of ≥ 3 % by weight, preferably in the form of an emulsion, in particular an oil-in-water (O/W) emulsion, a water-in-oil (W/O) emulsion, a multiple emulsion, a hydrodispersion gel, a balm, a multiple emulsion of the water-in-oil type (W/O/W) or of the oil-in-water type (O/W/O), a PIT emulsion, a Pickering emulsion, a micro-emulsion, a liquid, a lotion, a suspension, a milk, an ointment, a paste, a gel, a cream-based formulation, an oil-based formulation or a liposome-based formulation; or
(ii) in the form of a liquid surfactant formulation; or
(iii) in the form of an aqueous or aqueous/alcoholic, in particular aqueous/ethanolic, or aqueous/glycolic based solution, comprising the alcohol or glycol in an amount of 0.1 % to ≤ 50 % by weight, based on the total weight of the solution;
in particular, wherein the cosmetic or pharmaceutical composition is a cold formulation preparation.

8. The cosmetic or pharmaceutical composition or homecare product according to any one of claims 1 to 7 as a cosmetic for personal care, in particular for skin, hair, scalp and nail care, or as a pharmaceutical or for animal care, or wherein the homecare product is selected from the group consisting of liquid detergents, all-purpose cleaners, laundry and cleaning agents, fabric softeners, laundry scent and laundry scent boosters.

9. Use of a mixture comprising at least one first linear alkanediol and one or more second linear alkanediol as defined in any one of claims 1 to 3 for reducing the microbial load of a natural polymer, in particular in a cosmetic or pharmaceutical composition or homecare product, and/or as an antimicrobial agent replacer in a cosmetic or pharmaceutical composition or homecare product comprising at least one natural polymer,
wherein the at least one natural polymer is selected from the group consisting of guar gum, xanthan gum, carboxymethylcellulose, and mixtures of two or more of the aforesaid natural polymers.

10. Method for reducing the microbial load of a cosmetic or pharmaceutical composition or homecare product comprising a natural polymer, comprising the steps of:
- providing a cosmetic or pharmaceutical composition or homecare product comprising a natural polymer; and
- incorporating an effective amount of a mixture comprising at least one first linear alkanediol and one or more second linear alkanediol as defined in any one of claims 1 to 3,
wherein the at least one natural polymer is selected from the group consisting of guar gum, xanthan gum, carboxymethylcellulose, and mixtures of two or more of the aforesaid natural polymers.

## Patentansprüche

1. Kosmetische oder pharmazeutische Zusammensetzung oder Homecare-Produkt, das aufweist oder besteht aus:
(a) 0,3 bis 3,0 Gew.-% einer Mischung, die mindestens ein erstes lineares Alkandiol mit einer Kohlenstoffkette von 5 bis 14 Kohlenstoffatomen und ein oder mehrere zweite lineare Alkandiole mit einer Kohlenstoffkette von 5 bis 14 Kohlenstoffatomen aufweist, die sich von dem ersten linearen Alkandiol unterscheiden, basierend auf dem Gesamtgewicht der Zusammensetzung oder des Homecare-Produkts;
(b) 0,05 bis 6,0 Gew.-% mindestens eines natürlichen Polymers, basierend auf dem Gesamtgewicht der Zusammensetzung oder des Homecare-Produkts; und
(c) optional mindestens einen Wirkstoff für eine kosmetische oder pharmazeutische Zusammensetzung oder ein Homecare-Produkt und/oder einen Zusatzstoff,
wobei die Mischung, die mindestens ein erstes lineares Alkandiol und ein oder mehrere zweite lineare Alkandiole aufweist, aus der Gruppe ausgewählt ist, die besteht aus:
einer Mischung, die 1,2-Hexandiol und 2,3-Hexandiol aufweist;
einer Mischung, die 1,2-Heptandiol und 2,3-Heptandiol aufweist;
einer Mischung, die 1,2-Octandiol und 2,3-Octandiol aufweist; und
einer Mischung, die 1,2-Heptandiol und 1,2-Nonandiol aufweist; und
wobei das mindestens eine natürliche Polymer aus der Gruppe ausgewählt ist, die aus Guarkernmehl, Xanthan, Carboxymethylcellulose und Mischungen aus zwei oder mehr der vorgenannten natürlichen Polymere besteht.

2. Kosmetische oder pharmazeutische Zusammensetzung oder Homecare-Produkt nach Anspruch 1, wobei das erste lineare Alkandiol oder das zweite Alkandiol ein Alkandiol mit einer maximalen Wasserlöslichkeit von weniger als oder gleich 10 Gew.-%, insbesondere einer maximalen Wasserlöslichkeit von weniger als oder gleich 5 Gew.-% und mehr als oder gleich 1,4 Gew.-%, vorzugsweise ein Alkandiol ist, das aus der Gruppe ausgewählt ist, die aus 1,2-Heptandiol, 2,3-Heptandiol und Mischungen davon besteht.

3. Kosmetische oder pharmazeutische Zusammensetzung oder Homecare-Produkt nach Anspruch 1 oder Anspruch 2,
- wobei die Mischung, die mindestens ein erstes lineares Alkandiol und ein oder mehrere zweite lineare Alkandiole aufweist, das erste lineare Alkandiol und das zweite Alkandiol in einem Verhältnis im Bereich von 50 : 50 bis 99,9 : 0,1, vorzugsweise in einem Verhältnis im Bereich von 75 : 25 bis 99 : 1, noch bevorzugter in einem Verhältnis im Bereich von 80 : 20 bis 98 : 2, am meisten bevorzugt in einem Verhältnis im Bereich von 90 : 10 bis 95 : 5 aufweist.

4. Kosmetische oder pharmazeutische Zusammensetzung oder Homecare-Produkt nach einem der Ansprüche 1 bis 3, wobei der mindestens eine Wirkstoff für eine kosmetische oder pharmazeutische Zusammensetzung oder ein Homecare-Produkt und/oder Zusatzstoff ausgewählt ist aus der Gruppe, die aus Mitteln gegen Hautalterung, Antioxidantien, Chelatbildnern, Emulgatoren, Tensiden, Konservierungsmitteln, synthetischen Polymeren, hautkühlenden Mitteln, Rheologieadditiven, Ölen, Duftstoffen oder Parfümölen, Polyolen und Mischungen aus zwei oder mehr der vorgenannten Substanzen ausgewählt ist.

5. Kosmetische oder pharmazeutische Zusammensetzung oder Homecare-Produkt nach einem der Ansprüche 1 bis 4, das ferner mindestens einen antimikrobiellen Wirkstoff enthält, der aus der Gruppe ausgewählt ist, die besteht aus: Caprylhydroxamsäure, o-Cymol-5-ol, Isopropylparaben, Capryloylglycin, Phenylpropanol, Tropolon, PCA-Ethylcocoylarginat, 2-Methyl-5-cyclohexylpentanol, Phenoxyethanol, Dinatrium-EDTA, Methylparaben und dessen Salze, Natriumbenzoat, Benzylalkohol, Kaliumsorbat, Benzylsalicylat, Propylparaben, Methylchloroisothiazolinon, Methylisothiazolinon, Ethylhexylglycerin, Butylparaben, Ethylparaben, Natriumpropylparaben, DMDM-Hydantoin, Hydroxyethoxyphenylbutanon, Hydroxyethoxyphenylbutanol, Itaconsäure und deren Salze, Octopirox, Propandiolcaprylat, Climbazol, 4-Hydroxyacetophenon, Dehydroessigsäure, lodopropynylbutylcarbamat, Salicylsäure, Chlorphenesin, Isobutylparaben, Natriumethylparaben, Diazolidinylharnstoff, Farnesol, Bisabolol, Glycerylcaprylat, Natriumphytat oder Phytinsäure, Natriumlevulinat oder Levulinsäure und deren Ester, Chlorhexidin, Glyceryllaurat, Anissäure und deren Salze, Chlorhexidindigluconat, TEA-Salicylat, Phenethylalkohol, Glycerylcaprat, Sorbitancaprylat, Tetranatriumglutamatdiacetat, Trinatriumethylendiamindisuccinat, Natriumlactat, Undecylenoylglycin, Thymol, 1,3-Propandiol, 4-Hydroxybenzoesäure und deren Salze und Ester, N-(4-Chlorphenyl)-N'-(3,4-dichlorphenyl)harnstoff, 2,4,4'-Trichlor-2'-hydroxydiphenylether (Triclosan), 4-Chlor-3,5-dimethylphenol, 2,2'-Methylenbis(6-brom-4-chlorphenol), 3-Methyl-4-(1-methylethyl)phenol, 2-Benzyl-4-chlorphenol, 3-(4-Chlorphenoxy)-1,2-propandiol, 3-Iodo-2-propynylbutylcarbamat, 3,4,4'-Trichlorcarbanilid (TTC), antibakterielle Duftstoffe, Thymol, Thymianöl, Eugenol, Nelkenöl, Menthol, Minzöl, Glycerinmonocaprat, Glycerinmonocaprylat, Glycerinmonolaurat (GML), Diglycerinmonocaprat (DMC), Salicylsäure-N-alkylamide, wie beispielsweise n-Octylsalicylamid oder n-Decylsalicylamid, Dehydroessigsäure, 4-Methylbenzylalkohol, Frambinon, Xylitylcaprylat, 3-Hydroxypropylester der Benzoesäure, 3-Hydroxypropylester der Anissäure, 3-Hydroxypropyl-2-hydroxybenzoat und Mischungen aus zwei oder mehr der vorgenannten antimikrobiellen Wirkstoffe.

6. Kosmetische oder pharmazeutische Zusammensetzung oder Homecare-Produkt nach einem der Ansprüche 1 bis 5, die die Mischung, die mindestens ein erstes lineares Alkandiol und ein oder mehrere zweite lineare Alkandiole in einer Menge von 0,5 bis 1,0 Gew.-%, basierend auf dem Gesamtgewicht der Zusammensetzung oder des Homecare-Produkts, aufweist;
und/oder das 2,3-Alkandiol der Mischung aufweist, die mindestens ein erstes lineares Alkandiol und ein oder mehrere zweite lineare Alkandiole in einer Menge von 0,5 bis 1,0 Gew.-%, basierend auf dem Gesamtgewicht der Zusammensetzung oder des Homecare-Produkts, aufweist; vorzugsweise in einer Menge von 0,001 bis 0,5 Gew.-%, noch bevorzugter in einer Menge von 0,005 bis 0,1 Gew.-% und am meisten bevorzugt in einer Menge von 0,01 bis 0,075 Gew.-%, basierend auf dem Gesamtgewicht der Zusammensetzung oder des Homecare-Produkts.

7. Kosmetische oder pharmazeutische Zusammensetzung nach einem der Ansprüche 1 bis 6
(i) in Form einer Dispersion, die die Ölkomponente in einer Menge von > 1 Gew.-%, insbesondere in einer Menge von > 3 Gew.-%, aufweist, vorzugsweise in Form einer Emulsion, insbesondere einer Öl-in-Wasser-Emulsion (O/W), einer Wasser-in-Öl-Emulsion (W/O), einer Mehrfachemulsion, eines Hydrodispersionsgels, eines Balsams, einer Mehrfachemulsion vom Wasser-in-Öl-Typ (W/O/W) oder vom Öl-in-Wasser-Typ (O/W/O), einer PIT-Emulsion, einer Pickering-Emulsion, einer Mikroemulsion, einer Flüssigkeit, einer Lotion, einer Suspension, einer Milch, einer Salbe, einer Paste, eines Gels, einer cremebasierten Formulierung, einer ölbasierten Formulierung oder einer liposomenbasierten Formulierung; oder
(ii) in Form einer flüssigen Tensidformulierung; oder
(iii) in Form wässrig-alkoholischen, insbesondere wässrig-ethanolischen, oder wässrig-glykolischen Lösung, die den Alkohol oder das Glykol in einer Menge von 0,1 Gew.-% bis < 50 Gew.-%, basierend auf dem Gesamtgewicht der Lösung, aufweist;
wobei die kosmetische oder pharmazeutische Zusammensetzung insbesondere ein Kaltformulierungspräparat ist.

8. Kosmetische oder pharmazeutische Zusammensetzung oder Homecare-Produkt nach einem der Ansprüche 1 bis 7 als Kosmetikum für die Körperpflege, insbesondere für die Haut-, Haar-, Kopfhaut- und Nagelpflege, oder als Arzneimittel oder für die Tierpflege, oder wobei das Homecare-Produkt aus der Gruppe ausgewählt ist, die aus flüssigen Waschmitteln, Allzweckreinigern, Wasch- und Reinigungsmitteln, Weichspülern, Waschduftstoffen und Waschduftverstärkern besteht.

9. Verwendung einer Mischung, die mindestens ein erstes lineares Alkandiol und ein oder mehrere zweite lineare Alkandiole aufweist, nach einem der Ansprüche 1 bis 3, zur Verringerung der mikrobiellen Belastung eines natürlichen Polymers, insbesondere in einer kosmetischen oder pharmazeutischen Zusammensetzung oder einem Homecare-Produkt, und/oder als Ersatz für ein antimikrobielles Mittel in einer kosmetischen oder pharmazeutischen Zusammensetzung oder einem Homecare-Produkt, das mindestens ein natürliches Polymer aufweist,
wobei das mindestens eine natürliche Polymer aus der Gruppe ausgewählt ist, die aus Guarkernmehl, Xanthan, Carboxymethylcellulose und Mischungen aus zwei oder mehr der vorgenannten natürlichen Polymere besteht.

10. Verfahren zur Verringerung der mikrobiellen Belastung einer kosmetischen oder pharmazeutischen Zusammensetzung oder eines Homecare-Produkts, das ein natürliches Polymer aufweist, das die Schritte aufweist:
- Bereitstellen einer kosmetischen oder pharmazeutischen Zusammensetzung oder eines Homecare-Produkts, das ein natürliches Polymer aufweist; und
- Einbringen einer wirksamen Menge einer Mischung, die mindestens ein erstes lineares Alkandiol und ein oder mehrere zweite lineare Alkandiole aufweist, nach einem der Ansprüche 1 bis 3,
wobei das mindestens eine natürliche Polymer aus der Gruppe ausgewählt ist, die aus Guarkernmehl, Xanthangummi, Carboxymethylcellulose und Mischungen aus zwei oder mehr der vorgenannten natürlichen Polymere besteht.

## Revendications

1. Composition cosmétique ou pharmaceutique ou produit d'entretien, comprenant, ou consistant en :
(a) 0,3 à 3,0 % en poids d'un mélange comprenant au moins un premier alcanediol linéaire ayant une chaîne carbonée de 5 à 14 atomes de carbone et un ou plusieurs deuxièmes alcanediols linéaires ayant une chaîne carbonée de 5 à 14 atomes de carbone, différents du premier alcanediol linéaire, sur la base du poids total de la composition ou du produit d'entretien ;
(b) 0,05 à 6,0 % en poids d'au moins un polymère naturel, sur la base du poids total de la composition ou du produit d'entretien ; et
(c) facultativement, au moins une substance active pour une composition cosmétique ou pharmaceutique ou un produit d'entretien et/ou un additif,
où le mélange comprenant au moins un premier alcanediol linéaire et un ou plusieurs deuxièmes alcanediols linéaires est sélectionné dans le groupe comprenant :
un mélange comprenant du 1,2-hexanediol et du 2,3-hexanediol ;
un mélange comprenant du 1,2-heptanediol et du 2,3-heptanediol ;
un mélange comprenant du 1,2-octanediol et du 2,3-octanediol ; et
un mélange comprenant du 1,2-heptanediol et du 1,2-nonanediol ; et
où ledit au moins un polymère naturel est sélectionné dans le groupe comprenant de la gomme de guar, de la gomme xanthane, de la carboxyméthylcellulose et des mélanges de deux ou de plusieurs des polymères naturels susmentionnés.

2. Composition cosmétique ou pharmaceutique ou produit d'entretien selon la revendication 1, où le premier alcanediol linéaire ou le deuxième alcanediol est un alcanediol ayant une solubilité maximale dans l'eau inférieure ou égale à 10 % en poids, en particulier une solubilité maximale dans l'eau inférieure ou égale à 5 % en poids et supérieure ou égale à 1,4 % en poids, de préférence un alcanediol sélectionné dans le groupe comprenant du 1,2-heptanediol, du 2,3-heptanediol et des mélanges de ceux-ci.

3. Composition cosmétique ou pharmaceutique ou produit d'entretien selon la revendication 1 ou la revendication 2,
- où le mélange comprenant au moins un premier alcanediol linéaire et un ou plusieurs deuxièmes alcanediols linéaires comprend le premier alcanediol linéaire et le deuxième alcanediol dans un rapport compris entre 50 : 50 à 99,9 : 0,1, avantageusement dans un rapport compris entre 75 : 25 à 99 : 1, de préférence dans un rapport compris entre 80 : 20 et 98 : 2, et tout particulièrement dans un rapport compris entre 90 : 10 et 95 : 5.

4. Composition cosmétique ou pharmaceutique ou produit d'entretien selon l'une des revendications 1 à 3, où ladite au moins une substance active pour une composition cosmétique ou pharmaceutique ou un produit d'entretien et/ou un additif est sélectionnée dans le groupe comprenant des agents anti-vieillissement de la peau, des antioxydants, agents chélateurs, émulsifiants, tensioactifs, conservateurs, polymères synthétiques, agents rafraîchissants pour la peau, additifs rhéologiques, huiles, parfums ou huiles parfumées, des polyols et des mélanges de deux ou de plusieurs des substances susmentionnées.

5. Composition cosmétique ou pharmaceutique ou produit d'entretien selon l'une des revendications 1 à 4, comprenant en outre au moins un agent antimicrobien sélectionné dans le groupe comprenant l'acide caprylhydroxamique, l'o-cymen-5-ol, l'isopropylparabène, la capryloyl glycine, le phénylpropanol, la tropolone, l'arginate d'éthylcocoyle de PCA, le 2-méthyl-5-cyclohexylpentanol, le phénoxyéthanol, l'EDTA disodique, le méthylparabène et ses sels, le benzoate de sodium, l'alcool benzylique, le sorbate de potassium, le salicylate de benzyle, le propylparabène, la méthylchloroisothiazolinone, la méthylisothiazolinone, l'éthylhexylglycérine, le butylparabène, l'éthylparabène, propylparabène de sodium, la DMDM hydantoïne, l'hydroxyéthoxyphénylbutanone, l'hydroxyéthoxyphénylbutanol, l'acide itaconique et ses sels, l'octopirox, le caprylate de propanediol, la climbazole, le 4-hydroxyacétophénone, l'acide déhydroacétique, le butylcarbamate d'iodopropynyle, l'acide salicylique, la chlorphénésine, l'isobutylparabène, l'éthylparabène de sodium, la diazolidinyl urée, le farnésol, le bisabolol, le caprylate de glycéryle, le phytate de sodium ou l'acide phytique, le lévulinate de sodium ou l'acide lévulinique et ses esters, la chlorhexidine, le laurate de glycéryle, l'acide anisique et ses sels, le digluconate de chlorhexidine, le salicylate de TEA, l'alcool phényléthylique, le caprate de glycéryle, le caprylate de sorbitane, le diacétate de glutamate tétrasodique, le disuccinate d'éthylènediamine trisodique, le lactate de sodium, l'undécylénoylglycine, le thymol, le 1,3-propanediol, l'acide 4-hydroxybenzoïque et ses sels et esters, la N-(4-chlorophényl)-N'-(3,4-dichlorophényl)urée, le 2,4,4'-trichloro-2'-hydroxy-diphényl éther (triclosan), le 4-chloro-3,5-diméthylphénol, 2,2'-méthylènebis(6-bromo-4-chlorophénol), le 3-méthyl-4-(1-méthyléthyl)phénol, le 2-benzyl-4-chlorophénol, le 3-(4-chlorophénoxy)-1,2-propanediol, le 3-iodo-2-propynyl butylcarbamate, le 3,4,4'-trichlorocarbanilide (TTC), les parfums antibactériens, le thymol, l'huile de thym, l'eugénol, l'huile de clou de girofle, le menthol, l'huile de menthe, le monocaprate de glycérol, le monocaprylate de glycérol, le monolaurate de glycérol (GML), le monocaprate de diglycérol (DMC), les N-alkylamides de l'acide salicylique, tels que, par exemple, le n-octylsalicylamide ou le n-décylsalicylamide, l'acide déhydroacétique, l'alcool 4-méthylbenzylique, la frambinone, le caprylate de xylityle, l'ester 3-hydroxypropylique de l'acide benzoïque, l'ester 3-hydroxypropylique de l'acide anisique, le 2-hydroxybenzoate de 3-hydroxypropyle et des mélanges de deux ou de plusieurs des agents antimicrobiens susmentionnés.

6. Composition cosmétique ou pharmaceutique ou produit d'entretien selon l'une des revendications 1 à 5, comprenant le mélange comprenant au moins un premier alcanediol linéaire et un ou plusieurs deuxièmes alcanediols linéaires en une teneur de 0,5 à 1,0 % en poids, sur la base du poids total de la composition ou du produit d'entretien ;
et/ou comprenant le 2,3-alcanediol du mélange comprenant au moins un premier alcanediol linéaire et un ou plusieurs deuxièmes alcanediols linéaires en une teneur de 0,5 à 1,0 % en poids, sur la base du poids total de la composition ou du produit d'entretien à domicile ; avantageusement en une teneur de 0,001 à 0,5 % en poids, de préférence en une teneur de 0,005 à 0,1 % en poids, et tout particulièrement en une teneur de 0,01 à 0,075 % en poids, sur la base du poids total de la composition ou du produit d'entretien.

7. Composition cosmétique ou pharmaceutique selon l'une des revendications 1 à 6
(i) sous la forme d'une dispersion comprenant le composant huileux en une teneur > 1 % en poids, en particulier en une teneur > 3 % en poids, de préférence sous la forme d'une émulsion, en particulier d'une émulsion huile-dans-eau (H/E), d'une émulsion eau-dans-huile (E/H), d'une émulsion multiple, d'un gel hydrodispersé, d'un baume, une émulsion multiple de type eau-dans-huile (E/H/E) ou de type huile-dans-eau (H/E/H), d'une émulsion PIT, d'une émulsion de Pickering, d'une micro-émulsion, d'un liquide, d'une lotion, d'une suspension, d'un lait, d'une pommade, d'une pâte, d'un gel, d'une formulation à base de crème, d'une formulation à base d'huile ou d'une formulation à base de liposome ; ou
(ii) sous la forme d'une formulation tensioactive liquide ; ou
(iii) sous la forme d'une solution aqueuse ou aqueuse/alcoolique, en particulier aqueuse/éthanolique, ou aqueuse/glycolique, comprenant l'alcool ou le glycol en une teneur de 0,1 % à 50 % en poids, sur la base du poids total de la solution ;
en particulier, où la composition cosmétique ou pharmaceutique est une préparation à formulation à froid.

8. Composition cosmétique ou pharmaceutique ou produit d'entretien selon l'une des revendications 1 à 7, en tant que produit cosmétique pour des soins corporels, en particulier pour les soins de la peau, des cheveux, du cuir chevelu et des ongles, ou en tant que produit pharmaceutique ou pour des soins animaux, ou où le produit d'entretien est sélectionné dans le groupe comprenant des détergents liquides, des produits de nettoyage polyvalents, des lessives et des agents de nettoyage, des adoucissants, des parfums pour le linge et des renforçateurs de parfum pour le linge.

9. Utilisation d'un mélange comprenant au moins un premier alcanediol linéaire et un ou plusieurs deuxièmes alcanediols linéaires selon l'une des revendications 1 à 3 pour réduire la charge microbienne d'un polymère naturel, en particulier dans une composition cosmétique ou pharmaceutique ou un produit d'entretien, et/ou en tant que substitut d'agent antimicrobien dans une composition cosmétique ou pharmaceutique ou un produit d'entretien comprenant au moins un polymère naturel,
où ledit au moins un polymère naturel est sélectionné dans le groupe comprenant de la gomme de guar, de la gomme xanthane, de la carboxyméthylcellulose et des mélanges de deux ou de plusieurs des polymères naturels susmentionnés.

10. Procédé de réduction de la charge microbienne d'une composition cosmétique ou pharmaceutique ou d'un produit d'entretien comprenant un polymère naturel, comprenant les étapes suivantes :
- préparation d'une composition cosmétique ou pharmaceutique ou d'un produit d'entretien comprenant un polymère naturel ; et
- incorporation d'une quantité efficace d'un mélange comprenant au moins un premier alcanediol linéaire et un ou plusieurs deuxièmes alcanediols linéaires selon l'une des revendications 1 à 3,
où ledit au moins un polymère naturel est sélectionné dans le groupe comprenant de la gomme de guar, de la gomme xanthane, de la carboxyméthylcellulose et des mélanges de deux ou de plusieurs des polymères naturels susmentionnés.
